(19) Europäisches Patentamt
European Patent Office
Office européen des brevets

(11) EP 2 141 206 B1

(12) **EUROPEAN PATENT SPECIFICATION**

(45) Date of publication and mention of the grant of the patent:
**03.12.2014 Bulletin 2014/49**

(51) Int Cl.:
***C08F 2/50*** *(2006.01)* ***C09D 4/00*** *(2006.01)*
***G02B 5/22*** *(2006.01)*

(21) Application number: **09008446.8**

(22) Date of filing: **29.06.2009**

(54) **Novel compound, polymerizable composition, color filter and production method thereof, solid-state imaging device, and planographic printing plate precursor**

Neuartige Verbindung, polymerisierbare Zusammensetzung, Farbfilter und Herstellungsverfahren dafür, Festkörper-Bildgebungsvorrichtung und planografischer Druckplattenvorläufer

Nouveau composé, composition polymérisable, filtre couleur et son procédé de production, dispositif d'imagerie à l'état solide, et précurseur à plaque d'impression planographique

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.06.2008 JP 2008171000**
**24.09.2008 JP 2008244416**

(43) Date of publication of application:
**06.01.2010 Bulletin 2010/01**

(73) Proprietor: **FUJIFILM Corporation**
**Minato-ku**
**Tokyo (JP)**

(72) Inventors:
- **Makino, Masaomi**
**Hibarana-gun**
**Shizuoka-ken (JP)**
- **Tsuchimura, Tomotaka**
**Hibarana-gun**
**Shizuoka-ken (JP)**

(74) Representative: **Hoffmann Eitle**
**Patent- und Rechtsanwälte PartmbB**
**Arabellastraße 30**
**81925 München (DE)**

(56) References cited:
**EP-A1- 2 105 443** **JP-A- 2007 231 000**
**JP-A- 2007 322 744**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 141 206 B1

## Description

BACKGROUND OF THE INVENTION

Field of the Invention

The present invention relates to a novel compound, a polymerizable composition, a color filter and a production method thereof, a solid-state imaging device, and a planographic printing plate precursor.

Photopolymerizable compositions are known which include, for example, an ethylenically unsaturated bond-containing polymerizable compound and a photopolymerization initiator. Such photopolymerizable compositions polymerize and cure when they are irradiated with light, and are therefore used for, for example, photocurable inks, photosensitive printing plates, color filters, and a variety of photoresists.

Another type of photopolymerizable compositions are also known in which, for example, an acid is generated upon application of light, and the acid serves as a catalyst. Specifically, such compositions may be used in materials for image formation, anti-counterfeiting or detection of energy-ray dose, in which a color reaction of a pigment precursor in the presence of the generated acid serving as a catalyst is used, or may be used for positive resists for use in manufacture of semiconductors, thin film transistors (TFTs), color filters, micromachine components, and the like, in which a decomposition reaction in the presence of the generated acid serving as a catalyst is used.

In recent years, photopolymerizable compositions sensitive to shorter wavelength (365 nm or 405 nm) light sources have been demanded in various applications, and demands for such compounds (e.g. photopolymerizable initiators) capable of exhibiting high sensitivity to such short wavelength light sources have been increasing. However, highly sensitive photopolymerization initiators are generally poor in stability. Therefore, there is a demand for photopolymerization initiators having both of improved sensitivity and stability over time (storage stability).

Under the circumstances, it is proposed to use oxime ester derivatives as photopolymerization initiators for photopolymerizable compositions, for example in U.S. Patent Nos. 4,255,513 and 4,590,145 and Japanese Patent Application Laid-Open (JP-A) Nos. 2000-80068, 2001-233842, 2006-342166, and 2007-231000. However, these known oxime ester compounds have low absorbance at a wavelength of 365 nm or 405 nm and are not satisfactory in terms of sensitivity.

At present, there has been a demand for photopolymerizable compositions having not only high stability over time but also high sensitivity to light having a short wavelength such as 365 nm or 405 nm.

For example, JP-A No. 2005-202252 discloses a colored radiation-sensitive composition for color filters which contains an oxime compound. However, the stability over time and short-wavelength sensitivity of such a composition is still insufficient.

There has also been a demand for colored radiation-sensitive compositions for color filters, which have improved reproducibility of hue after pattern formation, and suppression of change in coloring property over time has been strongly demanded.

On the other hand, there has been a demand for color filters for image sensors, which have high color concentrations and small thicknesses, in order to enhance the light-gathering ability of solid-state imaging devices such as charge coupled devices (CCDs) and in order to improve the image quality by improving color-separation properties. When a large amount of a coloring material is added to achieve a high color concentration, the sensitivity may be insufficient for faithful reproduction of the shape of a fine image pattern of 2.5 μm or less, whereby pattern defects can frequently occur over the product. In order to avoid such defects, photo-irradiation has to be performed with higher energy, which requires a long exposure time and leads to a significant reduction in manufacturing yield.

Under the circumstances, there has been a demand for colored radiation-sensitive compositions for color filters, which includes a large amount of a coloring material (colorant) and also has a high sensitivity, in order to achieve excellent pattern forming property.

A coloured photosensitive resin composition has also been disclosed that has fine chromaticity and is suitable for use in colour filters (JP-A-2007-322744). The resin composition comprises a binder, a polymerisable compound and an oxime ester photopolymerisation initiator.

A further oxime photopolymerisation initiator has been disclosed in JP-A-2007-23100. This oxime photopolymerisation initiator is stable on storage and on addition to a polymerisable composition, but efficiently initiates polymerisation in the polymerisable composition upon irradiation with light.

EP-A-2 105 443, which constitutes prior art under Article 54(3) EPC, also discloses oxime photopolymerisation initiators, and their use in photopolymerisable composition for colour filters. The photopolymerisable composition is highly sensitive to light having a wavelength in the range 365-405 nm, has excellent temporal stability and provides cured films that resist deterioration due to prolonged exposure to heat.

BRIEF DESCRIPTION OF THE DRAWINGS

FIG. 1 is a diagram illustrating an absorption curve of Specified Compound 1 obtained in Synthesis Example 1 of the invention; and

FIG. 2 is a diagram illustrating an absorption curve of Specified Compound 2 obtained in Synthesis Example 1 of the invention.

SUMMARY OF THE INVENTION

According to a first aspect of the invention, a polymerizable composition is obtained which has a high sensitivity to light sources having wavelengths of around 365 nm, excellent storage stability, and can form a cured film which can prevent deterioration in film properties thereof due to heat aging, and an oxime compound which can be used suitably for the polymerizable composition can be obtained.

According to a second aspect of the invention, a polymerizable composition is obtained which has excellent storage stability, is cured at a high sensitivity, has good pattern forming ability, can form a color pattern having excellent adhesiveness to a support, exhibits an excellent pattern shape even after post-heating following development, and which is to be used for formation of a colored region of a color filter.

According to a third aspect of the invention, a color filter, obtained by using the polymerizable composition to be used for formation of a colored region of a color filter, is provided, which exhibits a satisfactory pattern shape, and has a color pattern having excellent adhesiveness to a support, and a method for producing the color filter with high productivity, and a solid-state imaging device having the color filter, are also provided.

According to a fourth aspect of the invention, a planographic printing plate precursor is obtained which includes the polymerizable composition.

According to a still another aspect of the invention, a novel oxime compound which is suitably used for the polymerizable composition is provided.

The inventors of the present invention have found a novel oxime compound having a specified structure, and that a polymerizable composition including the oxime compound has excellent light absorption to wavelengths of around 365 nm and excellent storage stability. The present invention is directed to the followings. The term "$\lambda$max" as used in the invention refers to the maximum absorption wavelength.

According to a first aspect, the present invention provides a polymerizable composition comprising:

(A) an oxime polymerization initiator which is represented by the following Formula (1A) or (1B), and which has, in ethyl acetate, a molar absorption coefficient of 20,000 or more at a $\lambda$max within a wavelength range of 300 nm or more and less than 400 nm and a molar absorption coefficient of 400 or less at ($\lambda$max + 20 nm); and

(B) a polymerizable compound:

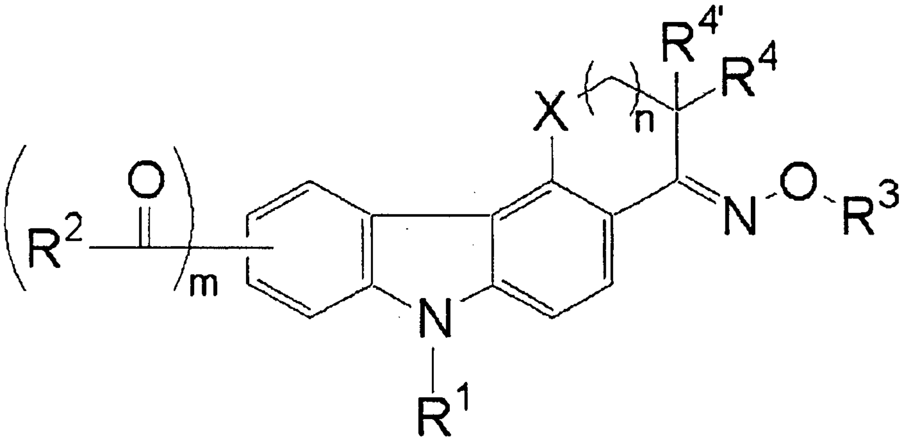

(1A)

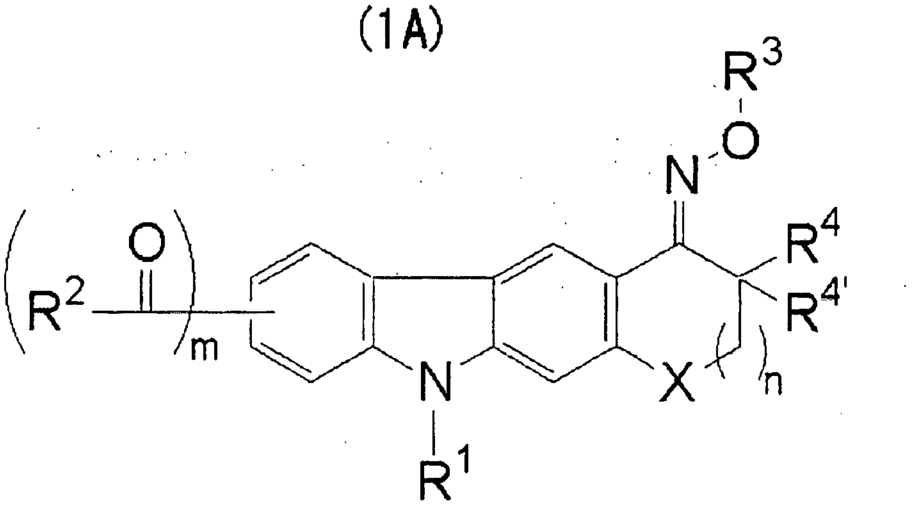

(1B)

wherein $R^1$ and $R^2$ each independently represents a substituted or unsubstituted alkyl group, a substituted or unsubstituted aryl group or a substituted or unsubstituted heterocyclic group; and $R^3$ represents a substituted or unsubstituted acyl group or a substituted or unsubstituted sulfonyl group; $R^4$ and $R^{4'}$ each independently represents a hydrogen atom, a substituted or unsubstituted alkyl group or a substituted or unsubstituted allyl group; n represents an integer of from 0 to 2, m represents 0 or 1; and X represents a carbon atom, an oxygen atom, a sulfur atom or a nitrogen atom.

Preferably $R^2$ in Formula (1A) or (1B) is an aryl group represented by the following Formula (2):

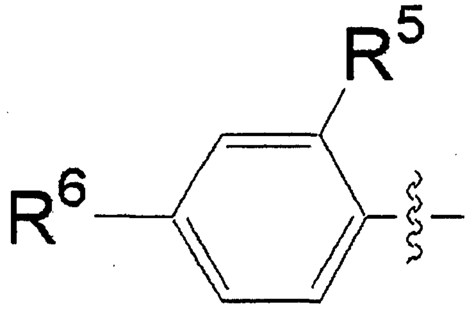

(2)

wherein $R^5$ and $R^6$ each independently represents a hydrogen atom, a halogen atom, an alkyl group, an aryl group, a hydroxy group, a thiol group, an amino group, a morpholino group, an alkyloxycarbonyl group, an acyloxy group, an

alkoxy group, an alkylthio group or an alkylseleno group, and these groups may each further have a substituent.

Preferably the polymerizable composition further comprises (C) a colorant.

Preferably the colorant (C) is a pigment, and the polymerizable composition further comprises (D) a pigment dispersant.

Preferably the colorant (C) is a black colorant.

According to a second aspect, the present invention provides the use of a polymerizable composition according to the above first aspect for forming a colored pattern of a color filter.

According to a third aspect, the present invention provides a color filter comprising:

a support; and
a colored pattern formed on the support by using the polymerizable composition according to the above second aspect.

According to a fourth aspect, the present invention provides a method for producing a color filter comprising:

applying the polymerizable composition according to the above second aspect onto a support to form a polymerizable composition layer;
subjecting the polymerizable composition layer to pattern exposure; and
developing the exposed polymerizable composition layer to form a colored pattern.

According to a fifth aspect, the present invention provides a solid-state imaging device comprising the color filter according to the above third aspect.

According to a sixth aspect, the present invention provides a planographic printing plate precursor comprising:

a support; and
a photosensitive layer including the polymerizable composition according to the above first aspect.

According to a seventh aspect, the present invention provides a compound represented by the following Formula (1A) or (1B):

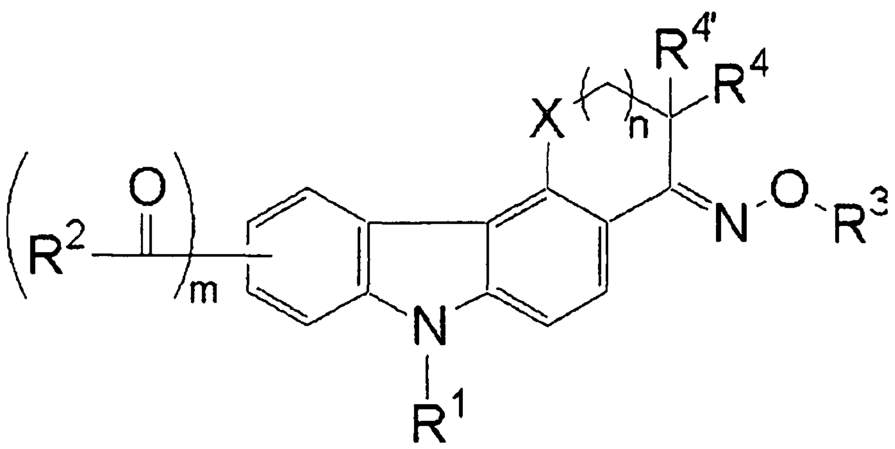

(1A)

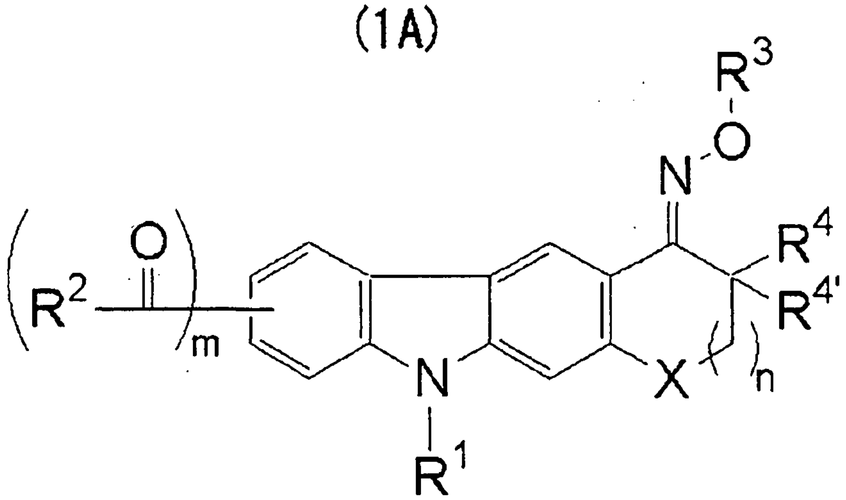

(1B)

wherein $R^1$ and $R^2$ each independently represents a substituted or unsubstituted alkyl group, a substituted or unsubstituted aryl group or a substituted or unsubstituted heterocyclic group, and $R^3$ represents a substituted or unsubstituted acyl group or a substituted or unsubstituted sulfonyl group; $R^4$ and $R^{4'}$ each independently represents a hydrogen atom, a substituted or unsubstituted alkyl group or a substituted or unsubstituted allyl group; n represents an integer of from 0 to 2, m represents 0 or 1; and X represents a carbon atom, an oxygen atom, a sulfur atom or a nitrogen atom.

According to an aspect of the invention, a polymerizable composition can be obtained which has a high sensitivity to light sources having wavelengths of around 365 nm, excellent storage stability, and can form a cured film which can prevent deterioration in film properties thereof due to heat aging, and an oxime compound which can be used suitably for the polymerizable composition can be obtained.

According to another aspect of the invention, a polymerizable composition can be obtained which has excellent storage stability, is cured at a high sensitivity, has good pattern forming ability, can form a color pattern having excellent adhesiveness to a support, exhibits an excellent pattern shape even after post-heating following development, and which is to be used for formation of a colored region of a color filter.

According to still another aspect of the invention, a color filter, obtained by using the polymerizable composition to be used for formation of a colored region of a color filter, is provided, which exhibits a satisfactory pattern shape, and has a color pattern having excellent adhesiveness to a support, and a method for producing the color filter with high productivity, and a solid-state imaging device having the color filter, are also provided.

According to still another aspect of the invention, a planographic printing plate precursor is obtained which includes the polymerizable composition.

DETAILED DESCRIPTION OF THE INVENTION

A polymerizable composition according to an exemplary embodiment of the invention includes (A) an oxime polymerization initiator which has, in ethyl acetate, a molar absorption coefficient of 20,000 or more at a λmax within the wavelength range of 300 nm or more and less than 400 nm and a molar absorption coefficient of 400 or less at (λmax + 20 nm) (the oxime polymerization initiator may hereinbelow be referred to as "specified oxime compound") and (B) a polymerizable compound.

The specified oxime compound of the exemplary embodiment functions as a photopolymerization initiator which is decomposed by light to initiate and promote the polymerization of a polymerizable compound. Particularly, since the specified oxime compound has a high sensitivity to light sources having wavelengths of around 365 nm, the specified oxime compound exerts an excellent effect when it is used as a photopolymerization initiator in a polymerizable composition.

*Specified Oxime Compound*

The specified oxime compound used in the polymerizable composition of the invention is an oxime polymerization initiator which is represented by the following Formula (1A) or (1B), and which has, in ethyl acetate, a molar absorption coefficient of 20,000 or more at a λmax within a wavelength range of 300 nm or more and less than 400 nm and a molar absorption coefficient of 400 or less at λmax + 20 nm):

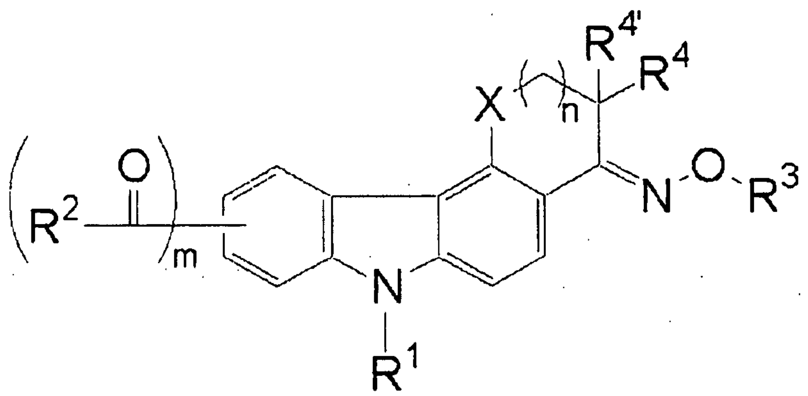

(1A)

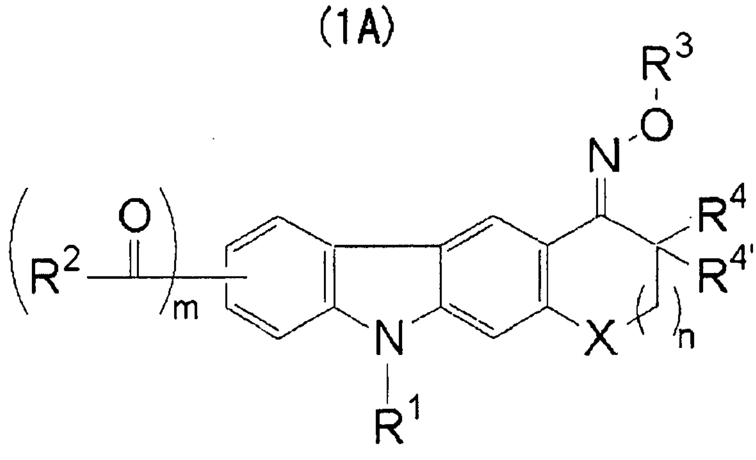

(1B)

wherein $R^1$ and $R^2$ each independently represents a substituted or unsubstituted alkyl group, a substituted or unsubstituted aryl group or a substituted or unsubstituted heterocyclic group; and $R^3$ represents a substituted or unsubstituted acyl group or a substituted or unsubstituted sulfonyl group; $R^4$ and $R^{4'}$ each independently represents a hydrogen atom, a substituted or unsubstituted alkyl group or a substituted or unsubstituted allyl group; n represents an integer of from 0 to 2, m represents 0 or 1; and X represents a carbon atom, an oxygen atom, a sulfur atom or a nitrogen atom.

Thus, the specified oxime compound demonstrates an extremely high sensitivity at λmax, and the sensitivity

decreases remarkably at λmax + 20 nm). That is, the specified oxime compound is highly sensitive only at a specified wavelength. Therefore, since the absorbance of the specified oxime compound decreases remarkably in the range of visible light at wavelengths of 400 nm or more, the specified oxime compound has no influence on the chromaticity of a color filter.

The acyl group which may have a substituent preferably is an acyl group having 2 to 20 carbon atoms, more preferably is an acyl group having 2 to 12 carbon atoms, and even more preferably is an acyl group having 2 to 7 carbon atoms.

Specific examples of the acyl group include an acetyl group, a propanoyl group, a butanoyl group, a trifluoromethylcarbonyl group, a pentanoyl group, a benzoyl group, a toluyl group, a 1-naphthoyl group, a 2-naphthoyl group, a 4-methylsulfanylbenzoyl group, a 4-phenylsulfanylbenzoyl group, a 4-dimethylaminobenzoyl group, a 4-diethylaminobenzoyl group, a 2-chlorobenzoyl group, a 2-methylbenzoyl group, a 2-methoxybenzoyl group, a 2-butoxybenzoyl group, a 3-chlorobenzoyl group, a 3-trifluoromethylbenzoyl group, a 3-cyanobenzoyl group, a 3-nitrobenzoyl group, a 4-fluorobenzoyl group, a 4-cyanobenzoyl group, and a 4-methoxybenzoyl group.

Among the specific examples, an acetyl group, a propanoyl group, a benzoyl group or a toluyl group is preferable, and an acetyl group or a benzoyl group is more preferable.

From the viewpoint of achieving higher sensitivity, $R^3$ in Formula (1A) and (1B) preferably is an acyl group, and specific examples thereof include an acetyl group, a propionyl group, a benzoyl group and a toluyl group.

The alkyl group which may have a substituent preferably is an alkyl group having 1 to 30 carbon atoms, more preferably is an alkyl group having 1 to 20 carbon atoms, and even more preferably is an alkyl group having 1 to 10 carbon atoms.

Specific examples thereof include a methyl group, an ethyl group, a propyl group, a butyl group, a hexyl group, an octyl group, a decyl group, a dodecyl group, an octadecyl group, an isopropyl group, an isobutyl group, a sec-butyl group, a tert-butyl group, a 1-ethylpentyl group, a cyclopentyl group, a cyclohexyl group, a trifluoromethyl group, a 2-ethylhexyl group, a phenacyl group, a 1-naphthoylmethyl group, a 2-naphthoylmethyl group, a 4-methylsulfanylphenacyl group, a 4-phenylsulfanylphenacyl group, a 4-dimethylaminophenacyl group, a 4-cyanophenacyl group, a 4-methylphenacyl group, a 2-methylphenacyl group, a 3-fluorophenacyl group, a 3-trifluoromethylphenacyl group, a 3-nitrophenacyl group, an isopentyl group, an ethoxyethyl group, a methoxyethoxyethyl group, a phenoxyethyl group, a methoxyethyl group, a cyclohexylmethyl group, a cyclopentylmethyl group, a tetrahydrofuranylmethyl group, a methoxypropyloxy group, a methoxypropyloxypropyl group, and a tert-butylmethyl group.

Among the specific examples, the alkyl group is preferably a methyl group, an ethyl group, a propyl group, a 2-ethylhexyl group, an isopentyl group, an ethoxyethyl group, a methoxyethoxyethyl group, a phenoxyethyl group, a methoxyethyl group, a cyclohexylmethyl group, a cyclopentylmethyl group, a tetrahydrofuranylmethyl group, a methoxypropyloxy group, a methoxypropyloxypropyl group or a tert-butylmethyl group, more preferably an ethyl group, a 2-ethylhexyl group, an isopentyl group, an ethoxyethyl group, a methoxyethoxyethyl group, a phenoxyethyl group, a methoxyethyl group, a cyclohexylmethyl group or a tert-butyl methyl, and even more preferably an ethyl group, a 2-ethylhexyl group, an isopentyl group, an ethoxyethyl group or a cyclohexylmethyl group.

The allyl group as $R^4$ and/or $R^{4'}$ in Formula (1A) and Formula (1B) is more preferably a group represented by the following Formula (3).

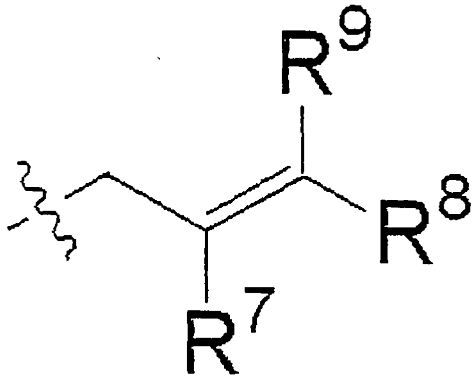

(3)

In Formula (3), $R^7$, $R^8$ and $R^9$ each independently represent a hydrogen atom, an alkyl group which may have a substituent, or an aryl group which may have a substituent. $R^7$ and $R^8$ may be bonded to each other to form a ring. $R^8$ and $R^9$ may be bonded to each other to form a ring.

It is most preferable that all of $R^7$, $R^8$ and $R^9$ are hydrogen atoms.

The alkyl group in Formula (3) preferably is an alkyl group having 1 to 10 carbon atoms, more preferably is an

alkyl group having 1 to 7 carbon atoms, and even more preferably is an alkyl group having 1 to 3 carbon atoms. Specific examples thereof include a methyl group, an ethyl group, a propyl group, a butyl group, a pentyl group, a hexyl group, an isopropyl group, an isobutyl group, a sec-butyl group, an isopentyl group, an ethoxyethyl group, a phenylmethyl group, a phenylethyl group and a phenoxyethyl group. Among these, a methyl group, an ethyl group and an ethoxyethyl group are preferable, and a methyl group is more preferable.

The aryl group in Formula (3) preferably is an aryl group having 6 to 20 carbon atoms, more preferably is an aryl group having 6 to 12 carbon atoms, and even more preferably is an aryl group having 6 to 8 carbon atoms. Specific examples thereof include a phenyl group, a tolyl group, a naphthyl group, a 4-methoxyphenyl group, a 4-fluorophenyl group, a 4-chlorophenyl group, a 4-bromophenyl group, a 4-nitrophenyl group, a 4-phenylphenyl group, and a 3,5-diphenylphenyl group. Among these, a phenyl group, a tolyl group and a naphthyl group are preferable, and a phenyl group is more preferable.

In Formula (1A) and Formula (1B), n preferably is 0 or 1, and more preferably is 0.

In Formula (1A) and Formula (1B), X represents a carbon atom, an oxygen atom, a sulfur atom or a nitrogen atom. From the viewpoint of achieving higher sensitivity, X is preferably an oxygen atom, a nitrogen atom or a sulfur atom, and more preferably is an oxygen atom.

Furthermore, the heterocycle which may have a substituent, the alkyl group which may have a substituent, the aryl group which may have a substituent, and the acyl group which may have a substituent may each be substituted by another substituent.

Example of the another substituent include: halogen groups such as a fluorine atom, a chlorine atom, a bromine atom and an iodine atom; alkoxy groups such as a methoxy group, an ethoxy group and a tert-butoxy group; aryloxy groups such as a phenoxy group and a p-tolyloxy group; alkoxycarbonyl groups such as a methoxycarbonyl group, a butoxycarbonyl group and a phenoxycarbonyl group; acyloxy groups such as an acetoxy group, a propionyloxy group and a benzoyloxy group; acyl groups such as an acetyl group, a benzoyl group, an isobutyryl group, an acryloyl group, a methacryloyl group and a methoxalyl group; alkylsulfanyl groups such as a methylsulfanyl group and a tert-butylsulfanyl group; arylsulfanyl groups such as a phenylsulfanyl group and a p-tolylsulfanyl group; alkylamino groups such as a methylamino group and a cyclohexylamino group; dialkylamino groups such as a dimethylamino group, a diethylamino group, a morpholino group and a piperidino group; arylamino groups such as a phenylamino group and a p-tolylamino group; alkyl groups such as a methyl group, an ethyl group, a tert-butyl group and a dodecyl group; aryl groups such as a phenyl group, a p-tolyl group, a xylyl group, a cumenyl group, a naphthyl group, an anthryl group and a phenanthryl group; a hydroxy group, a carboxy group, a formyl group, a mercapto group, a sulfo group, a mesyl group, a p-toluenesulfonyl group, an amino group, a nitro group, a cyano group, a trifluoromethyl group, a trichloromethyl group, a trimethylsilyl group, a phosphinico group, a phosphono group, a trimethylammoniumyl group, a dimethylsulfoniumyl group, and group triphenylphenacyl phosphoniumyl group.

As a result of investigations, the inventors of the present invention have found that the above compounds represented by Formulae (1A) and (1B), respectively, are novel compounds. The novel oxime compound having a structure represented by Formula (1A) or (1B) is extremely useful as a photopolymerization initiator, as described in detail hereinbelow.

The atomic group including $R^2$ and the bonding oxygen atom in Formulae (1A) and (1B) may be bonded to the benzene ring to form a cyclic structure.

Each of the alkyl group, the aryl group and the heterocyclic group represented by $R^1$ and $R^2$ in Formulae (1A) and (1B) may further have a substituent.

The aryl group which may have a substituent preferably is an aromatic ring group having 6 to 30 carbon atoms, more preferably is an aromatic ring group having 6 to 20 carbon atoms, and even more preferably is an aromatic ring group having 6 to 10 carbon atoms.

Specific examples include a phenyl group, a biphenyl group, a 1-naphthyl group, a 2-naphthyl group, a 9-anthryl group, a 9-phenanthryl group, a 1-pyrenyl group, a 5-naphthacenyl group, a 1-indenyl group, a 2-azulenyl group, a 9-fluorenyl group, a terphenyl group, a quarterphenyl group, an o-, m- or p-tolyl group, a xylyl group, an o-, m- or p-cumenyl group, a mesityl group, a pentalenyl group, a binaphthalenyl group, a ternaphthalenyl group, a quarternaphthalenyl group, a heptalenyl group, a biphenylenyl group, an indacenyl group, a fluoranthenyl group, an acenaphthylenyl group, an aceanthrylenyl group, a phenalenyl group, a fluorenyl group, an anthryl group, a bianthracenyl group, a teranthracenyl group, a quarteranthracenyl group, an anthraquinolyl group, a phenanthryl group, a triphenylenyl group, a pyrenyl group, a crycenyl group, a naphthacenyl group, a pleiadenyl group, a picenyl group, a perylenyl group, a pentaphenyl group, a pentacenyl group, a tetraphenylenyl group, a hexaphenyl group, a hexacenyl group, a rubicenyl group, a coronenyl group, a trinaphthylenyl group, a heptaphenyl group, a heptacenyl group, a pyranthrenyl group, and an ovalenyl group.

Among the specific examples, a phenyl group and an o-, m-, or p-tolyl group are preferable, and an o-tolyl group is more preferable.

Examples of the heterocycle group include an aromatic or aliphatic heterocycle having a nitrogen atom, an oxygen atom, a sulfur atom or a phosphorus atom.

Specific examples thereof include a thienyl group, a benzo[b]thienyl group, a naphtho[2,3-b]thienyl group, a thianthrenyl group, a furyl group, a pyranyl group, an isobenzofuranyl group, a chromenyl group, a xanthenyl group, a phenoxathiinyl group, a 2H-pyrrolyl group, a pyrrolyl group, an imidazolyl group, a pyrazolyl group, a pyridyl group, a pyrazinyl group, a pyrimidinyl group, a pyridazinyl group, an indolizinyl group, an isoindolyl group, a 3H-indolyl group, an indolyl group, a 1H-indazolyl group, a purinyl group, a 4H-quinolizinyl group, an isoquinolyl group, a quinolyl group, a phthalazinyl group, a naphthyridinyl group, a quinoxalinyl group, a quinazolinyl group, a cinnolinyl group, a pteridinyl group, a 4aH-carbazolyl group, a carbazolyl group, a β-carbolinyl group, a phenanthridinyl group, an acridinyl group, a perimidinyl group, a phenanthrolinyl group, a phenazinyl group, a phenarsazinyl group, an isothiazolyl group, a phenothiazinyl group, an isoxazolyl group, a furazanyl group, a phenoxazinyl group, an isochromanyl group, a chromanyl group, a pyrrolidinyl group, a pyrrolinyl group, an imidazolidinyl group, an imidazolinyl group, a pyrazolidinyl group, a pyrazolinyl group, a piperidyl group, a piperazinyl group, an indolinyl group, an isoindolinyl group, a quinuclidinyl group, a morpholinyl group, and a thioxanthonyl group. Among these, a thienyl group, a pyridyl group, a furyl group, a pyranyl group, an imidazolyl group, a piperidyl group, a morpholinyl group, and a thioxanthonyl are preferable, a thienyl group, a pyridyl group, a furyl group, a pyranyl group, and imidazolyl group are more preferable, and a thienyl group, a pyridyl group, and a furyl group are even more preferable.

$R^2$ shown in Formulae (1A) and (1B) preferably is an aryl group represented by the following Formula (2).

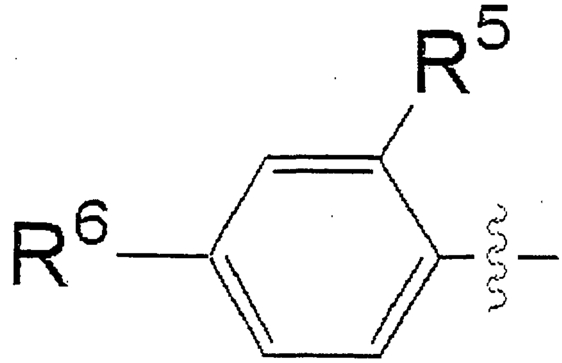

(2)

In Formula (2), $R^5$ and $R^6$ each independently represent a hydrogen atom, a halogen atom, an alkyl group, an aryl group, a hydroxy group, a thiol group, an amino group, a morpholino group, an alkyloxycarbonyl group (including an alkoxycarbonyl group and an aryloxycarbonyl group), a carbazolyl group, an acyloxy group, or an alkoxy group. Each of the substituents may further have an additional substituent, if the additional substituent can be introduced thereto.

Examples of the halogen atom include fluorine, chlorine, bromine and iodine. Among these, fluorine and bromine are preferable, and bromine is more preferable.

The alkyl group which may have a substituent and the aryl group which may have a substituent have the same meanings as the alkyl group and the aryl group represented by $R^1$ and $R^4$ in Formulae (1A) and (1B), respectively, and their preferable scopes are also the same.

The alkoxycarbonyl group which may have a substituent is preferably an alkoxycarbonyl group having 2 to 20 carbon atoms. Examples thereof include a methoxycarbonyl group, an ethoxycarbonyl group, a propoxycarbonyl group, a butoxycarbonyl group, a hexyloxycarbonyl group, an octyloxycarbonyl group, a decyloxycarbonyl group, an octadecyloxycarbonyl group, and a trifluoromethyloxycarbonyl group. Among these, a methoxycarbonyl group, an ethoxycarbonyl group and a propoxycarbonyl group are preferable, and a methoxycarbonyl group and an ethoxycarbonyl group are more preferable.

Examples of the aryloxycarbonyl group which may have a substituent include a phenoxycarbonyl group, a 1-naphthyloxycarbonyl group, a 2-naphthyloxycarbonyl group, a 4-methylsulfanylphenyloxycarbonyl group, a 4-phenylsulfanylphenyloxycarbonyl group, a 4-dimethylaminophenyloxycarbonyl group, a 4-diethylaminophenyloxycarbonyl group, a 2-chlorophenyloxycarbonyl group, a 2-methylphenyloxycarbonyl group, a 2-methoxyphenyloxycarbonyl group, a 2-butoxyphenyloxycarbonyl group, a 3-chlorophenyloxycarbonyl group, a 3-trifluoromethylphenyloxycarbonyl group, a 3-cyanophenyloxycarbonyl group, a 3-nitrophenyloxycarbonyl group, a 4-fluorophenyloxycarbonyl group, a 4-cyanophenyloxycarbonyl group, and a 4-methoxyphenyloxycarbonyl group. Among these, a phenoxycarbonyl group, a 1-naphthyloxycarbonyl group and a 2-naphthyloxycarbonyl group are preferable, and a phenoxycarbonyl group is more preferable.

Examples of the acyloxycarbonyl group which may have a substituent include a methylcarbonyloxy group, an ethylcarbonyloxy group, a propylcarbonyloxy group, a methoxymethylcarbonyloxy group, and a methoxyethylcarbonyloxy group. Among these, a methylcarbonyloxy group, an ethylcarbonyloxy group, and a propylcarbonyloxy group are preferable, and a methylcarbonyloxy group and an ethylcarbonyloxy group are more preferable.

Preferable examples of the alkoxy group which may have a substituent include a methoxy group, an ethoxy group, and a propyloxy group.

The alkoxy group, the alkylthio group, and the alkylseleno group are preferably those represented by the following formulae.

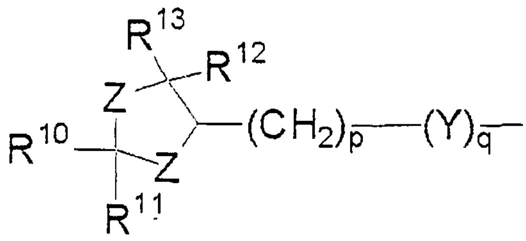

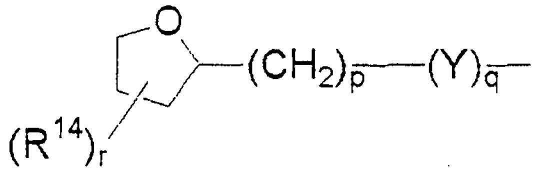

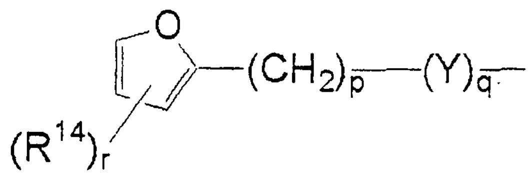

In the formulae, each Y represents an oxygen atom, a sulfur atom, or a selenium atom; each p represents an integer of from 0 to 5; each q represents 0 or 1; $R^{10}$, $R^{11}$, $R^{12}$, and $R^{13}$ independently represent a hydrogen atom, a halogen atom, or an alkyl group; each Z represents independently an oxygen atom, a sulfur atom, or a selenium atom; each $R^{14}$ represents an alkyl group or a halogen atom; and each r represents an integer of from 0 to 4.

In a preferable combination of the specified oxime compounds represented by the formulae (1A) and (1B), $R^1$ is a methyl group, an ethyl group, a propyl group, a 2-ethylhexyl group, a phenyl group, an isopentyl group, an ethoxyethyl group, a methoxyethoxyethyl group, or a phenoxyethyl group; $R^2$ is a phenyl group, an o-, m-, or p-tolyl group, a thienyl group, a pyridyl group, or a furyl group; $R^3$ is an acetyl group or a benzoyl group; $R^4$ and $R^{4'}$ each independently are a hydrogen atom or an allyl group; X is an oxygen atom; m is 1; and n is 0 or 1.

In a more preferable combination, $R^1$ is an ethyl group, an isopentyl group, an ethoxyethyl group, a methoxyethoxyethyl group, or a phenoxyethyl group; $R^2$ is a phenyl group, an o-tolyl group, a thienyl group, a pyridyl group, or a furyl group; $R^3$ is an acetyl group; $R^4$ and $R^{4'}$ each independently are a hydrogen atom; X is an oxygen atom; m is 1; and n is 0.

In an even more preferable combination, $R^1$ is an ethyl group, an isopentyl group, or an ethoxyethyl group; $R^2$ is an o-tolyl group; $R^3$ is an acetyl group; $R^4$ and $R^{4'}$ each independently are a hydrogen atom; X is an oxygen atom; m is 1; and n is 0.

Examples of the specified oxime compound of the invention are shown below, but the invention is not limited thereto.

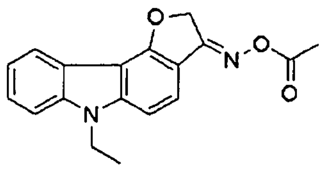

**(A-1)**

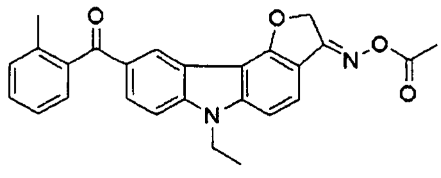

**(A-2)**

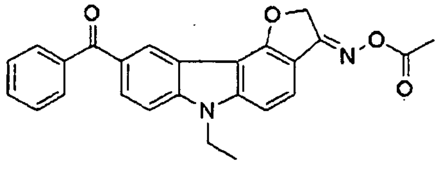

**(A-3)**

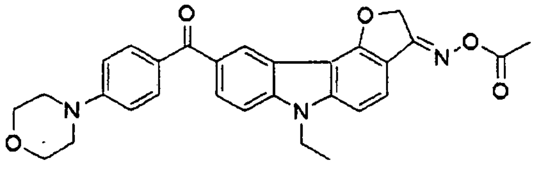

**(A-4)**

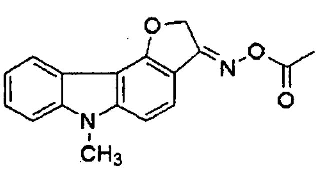
(A-5)

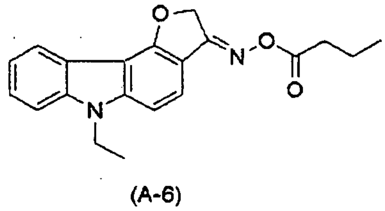
(A-6)

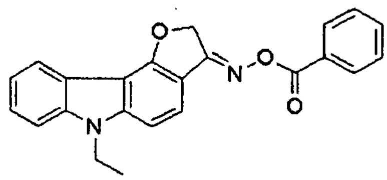
(A-7)

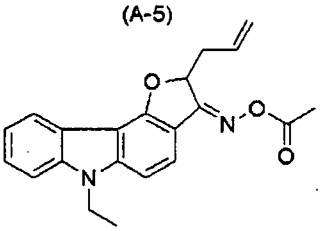
(A-8)

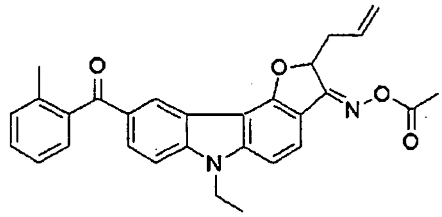
(A-9)

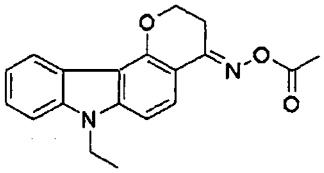
(A-10)

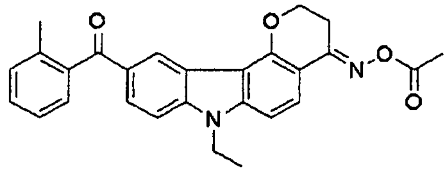
(A-11)

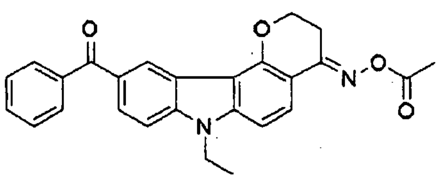
(A-12)

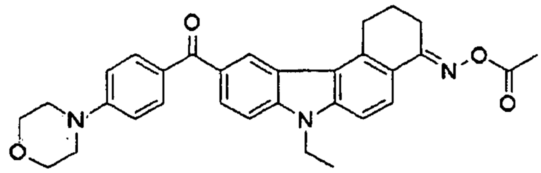
(A-13)

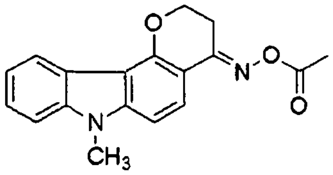
(A-14)

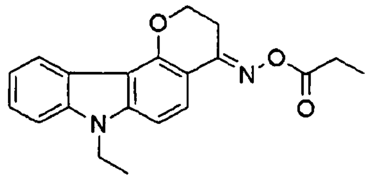
(A-15)

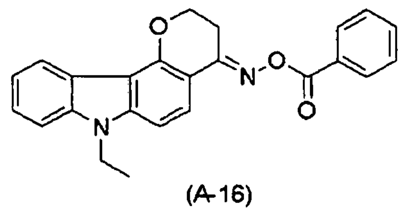
(A-16)

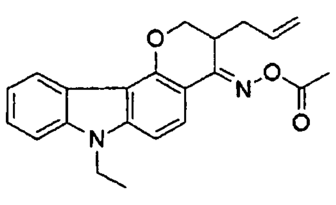
(A-17)

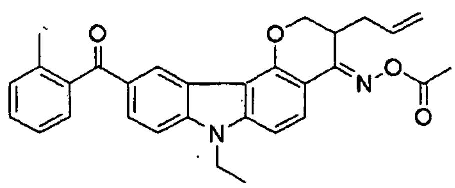
(A-18)

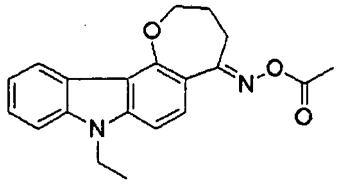

(A-19)

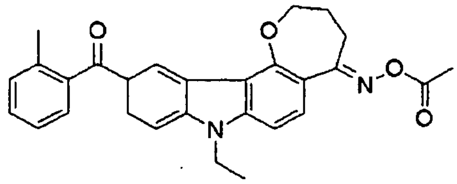

(A-20)

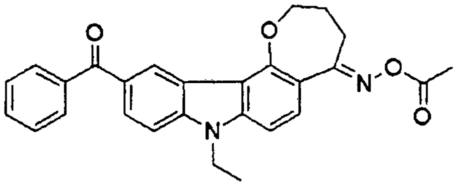

(A-21)

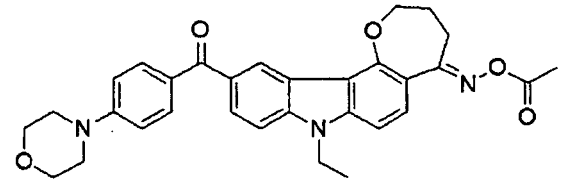

(A-22)

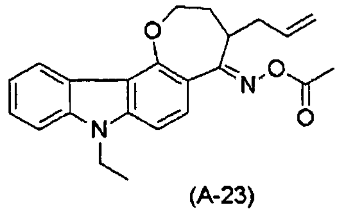

(A-23)

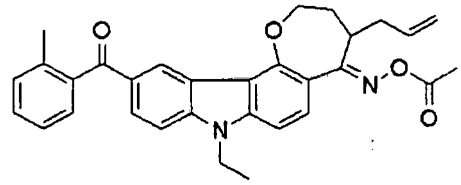

(A-24)

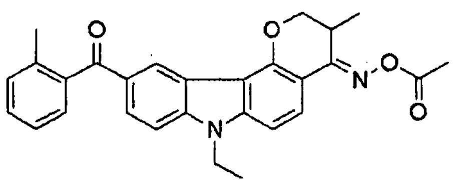

(A-25)

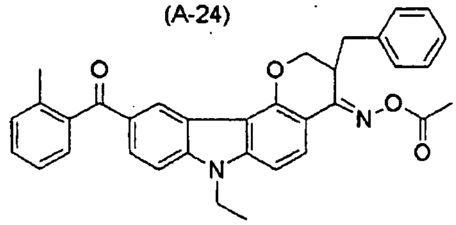

(A-26)

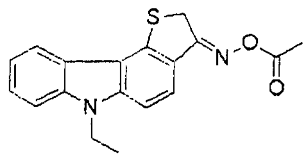

(A-34)

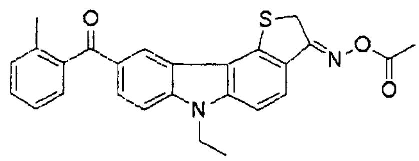

(A-35)

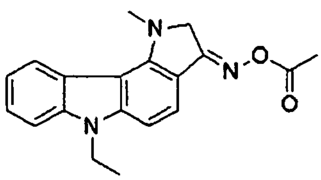

(A-36)

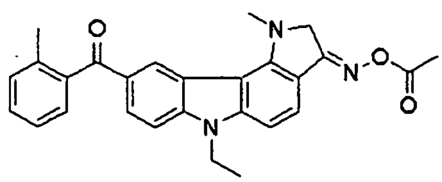

(A-37)

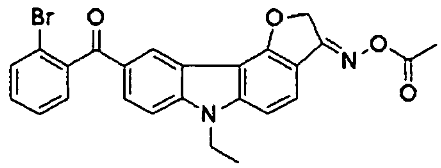

(A-38)

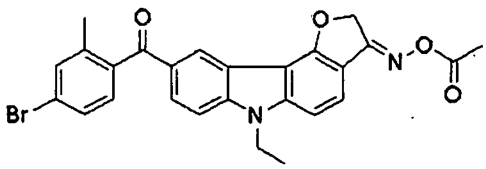

(A-39)

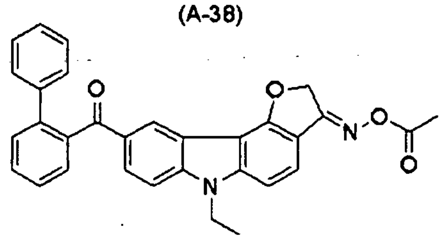

(A-40)

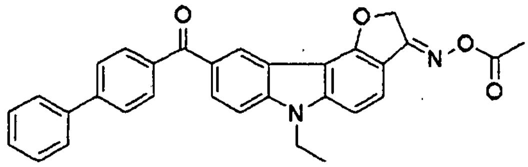

(A-41)

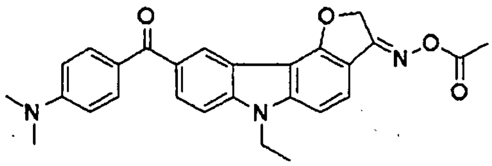

(A-42)

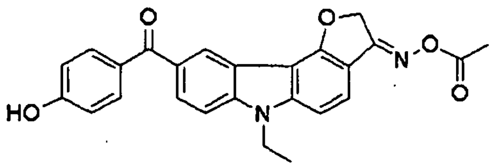

(A-43)

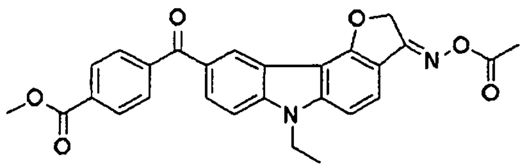

(A-44)

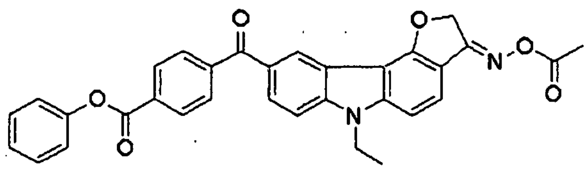

(A-45)

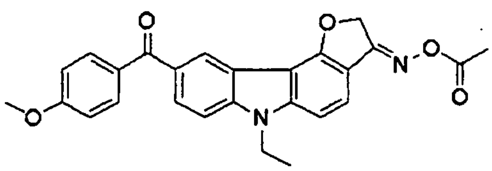

(A-46)

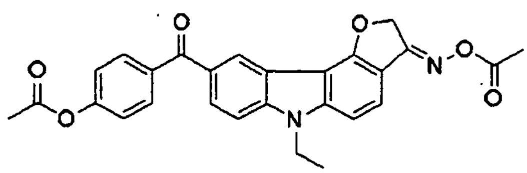

(A-47)

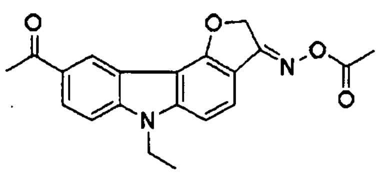

(A-48)

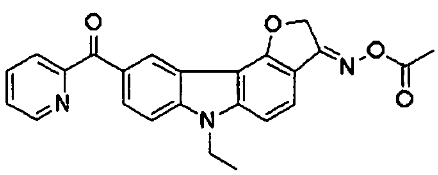

(A-49)

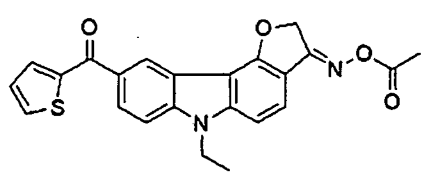

(A-50)

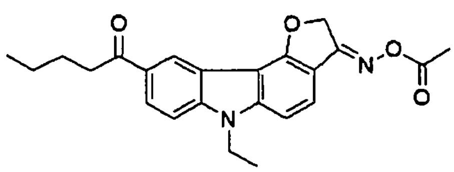

(A-51)

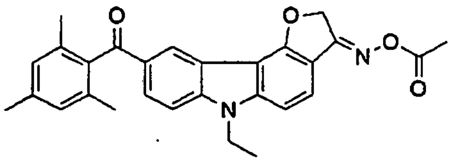

(A-52)

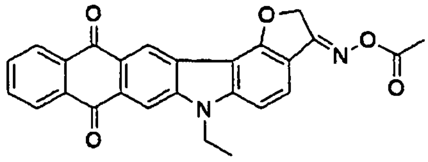

(A-53)

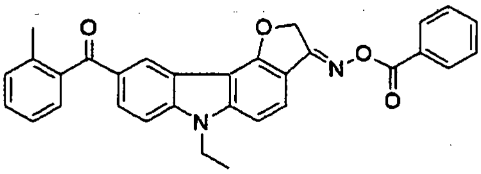

(A-54)

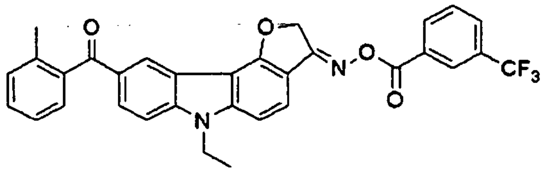

(A-55)

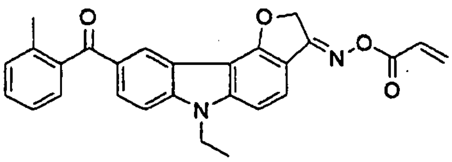

(A-56)

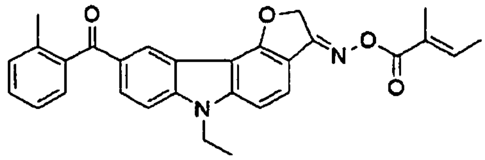

(A-57)

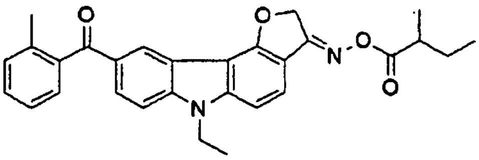

(A-58)

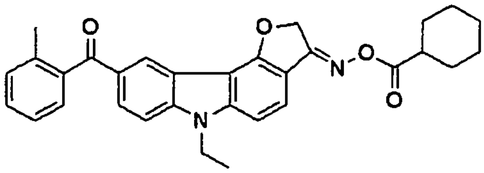

(A-59)

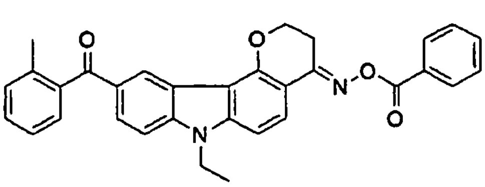

(A-60)

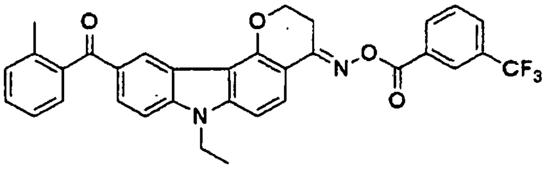

(A-61)

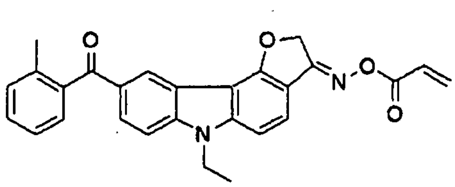

(A-62)

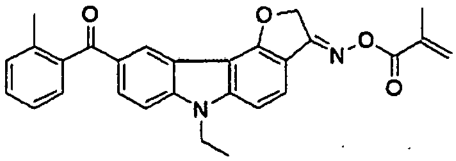

(A-63)

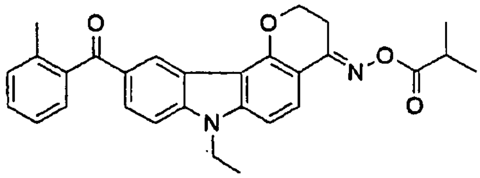

(A-64)

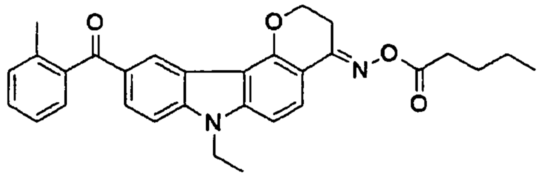

(A-65)

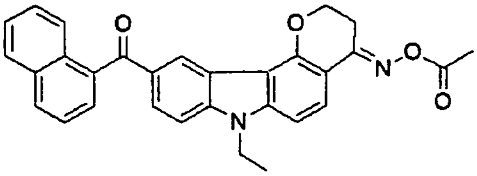

(A-66)

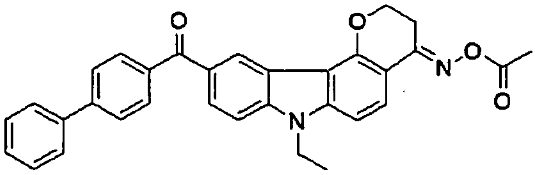

(A-67)

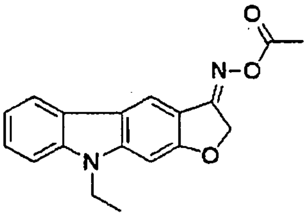

(A-68)

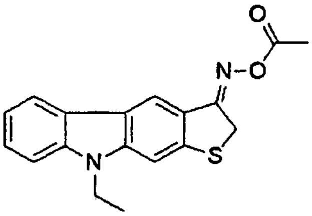

(A-69)

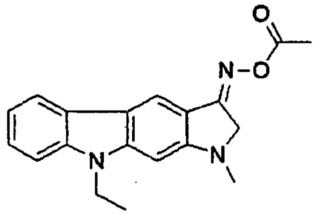

(A-70)

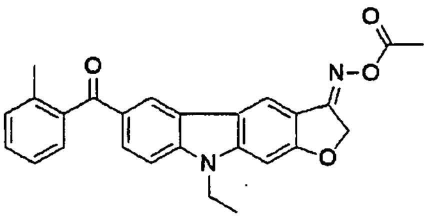

(A-71)

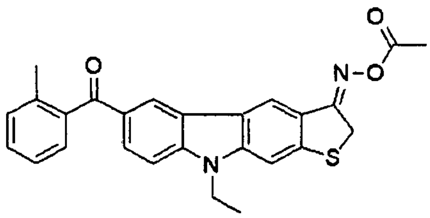

(A-72)

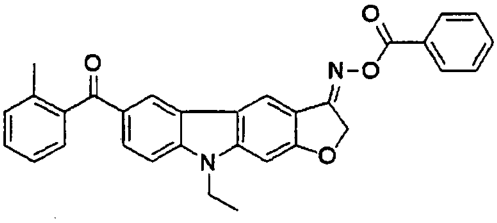

(A-73)

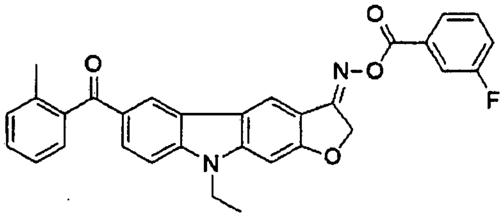

(A-74)

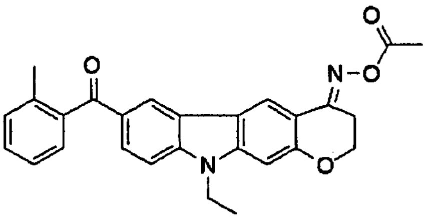

(A-75)

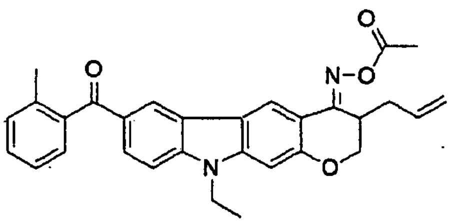

(A-76)

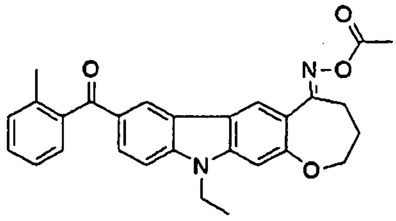

(A-77)

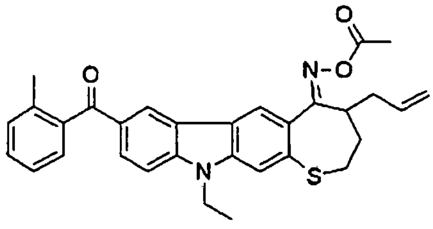

(A-78)

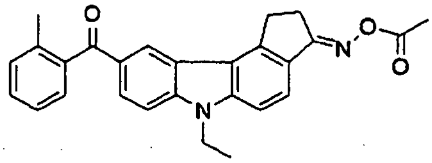

(A-79)

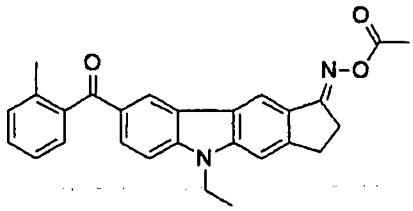

(A-80)

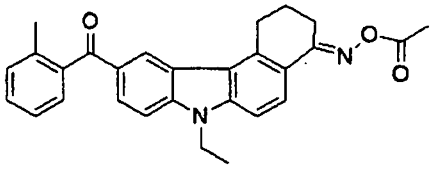

(A-81)

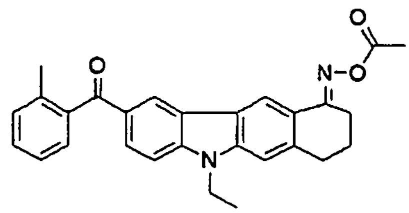

(A-82)

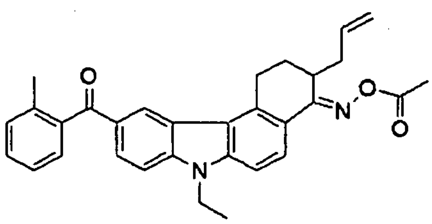

(A-83)

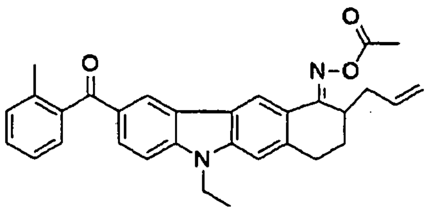

(A-84)

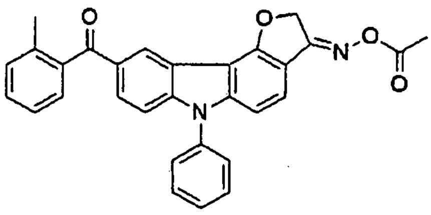

(A-85)

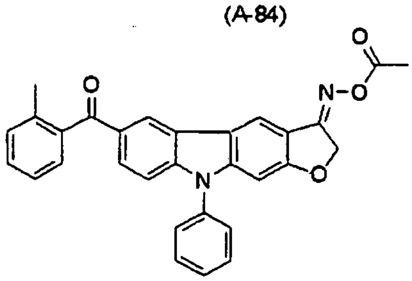

(A-86)

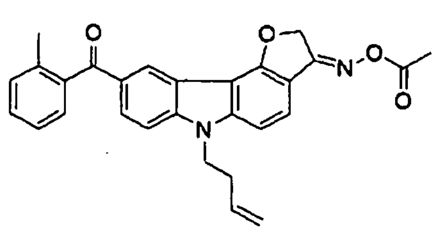

(A-87)

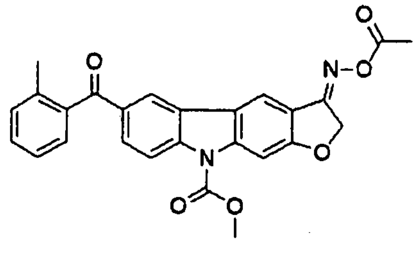

(A-88)

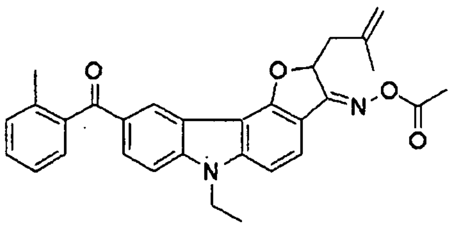

(A-89)

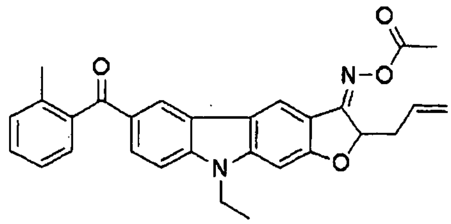

(A-90)

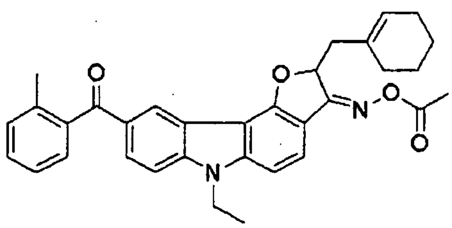

(A-91)

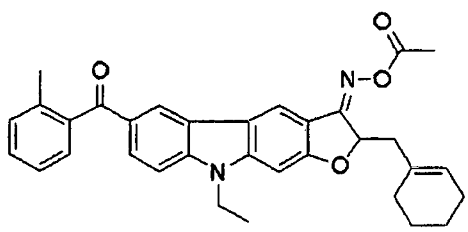

(A-92)

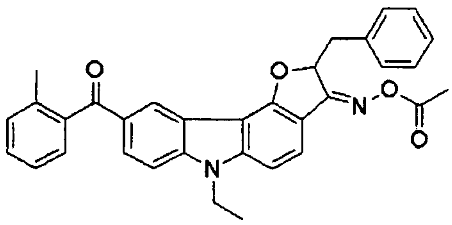

(A-93)

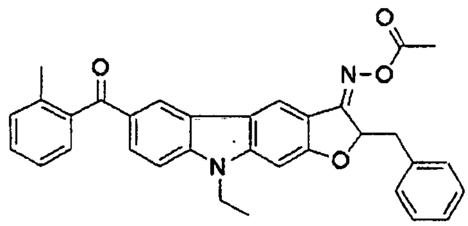

(A-94)

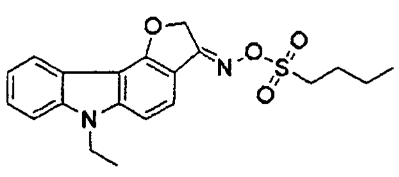

(A-95)

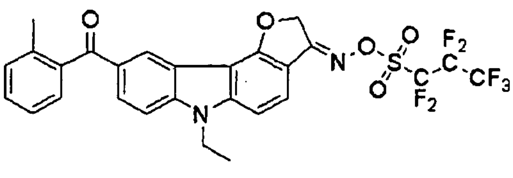

(A-96)

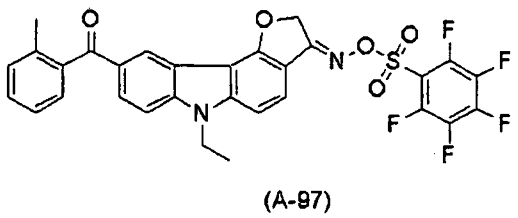

(A-97)

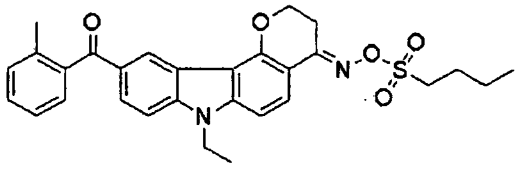

(A-98)

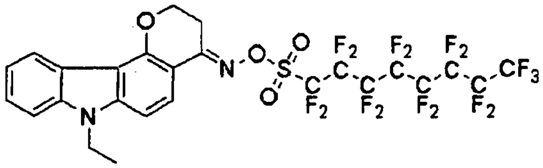

(A-99)

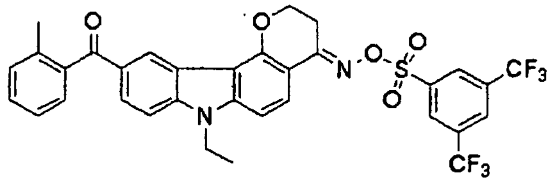

(A-100)

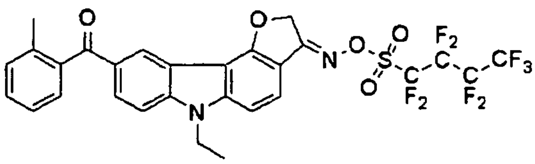

(A-101)

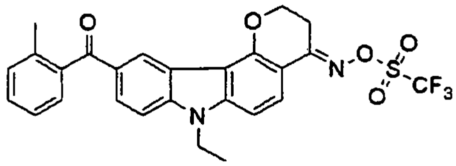

(A-102)

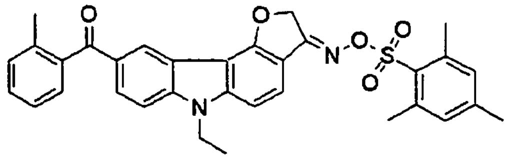

(A-103)

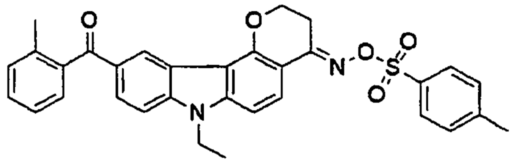

(A-104)

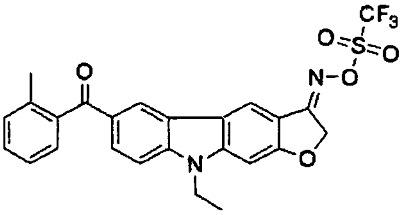

(A-105)

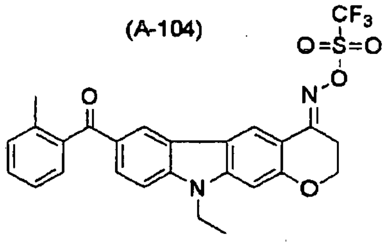

(A-106)

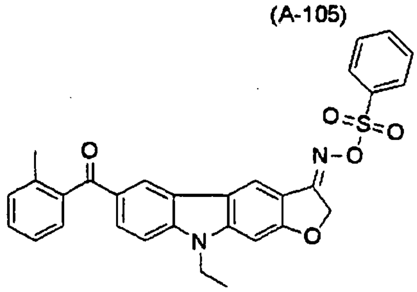

(A-107)

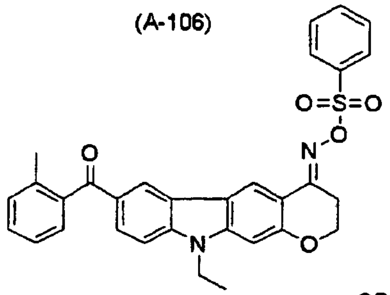

(A-108)

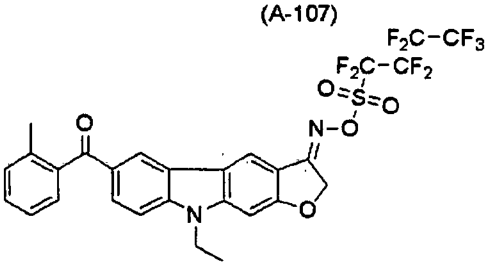

(A-109)

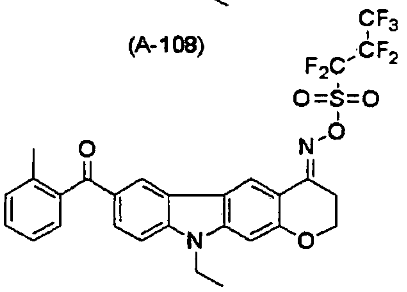

(A-110)

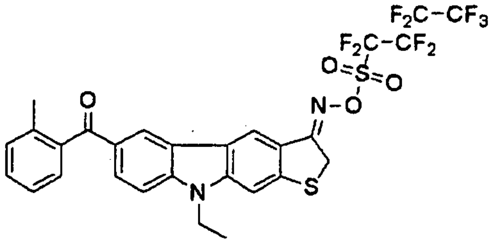

(A-111)

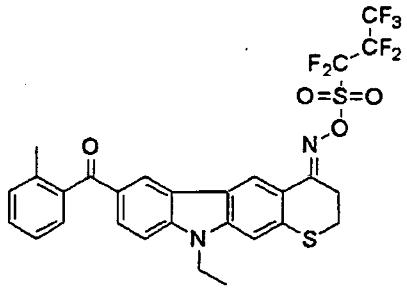

(A-112)

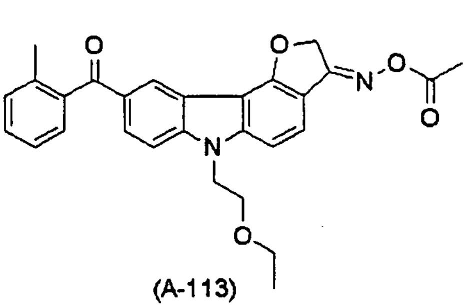

(A-113)

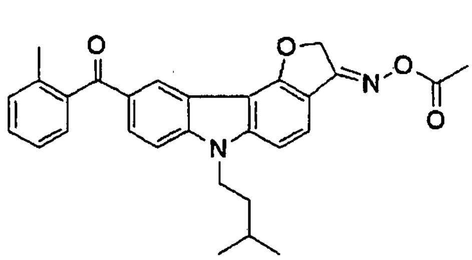

(A-114)

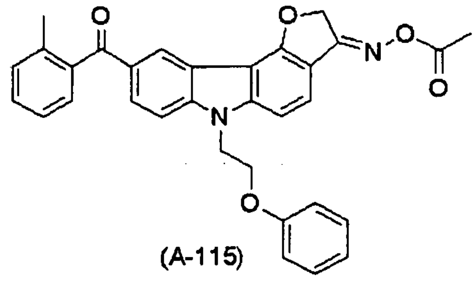

(A-115)

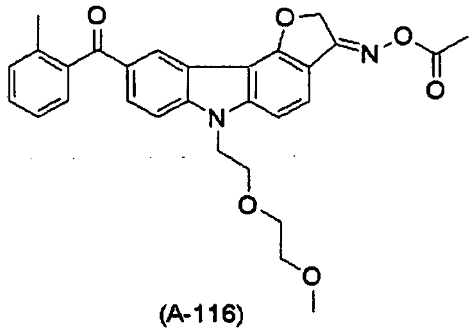

(A-116)

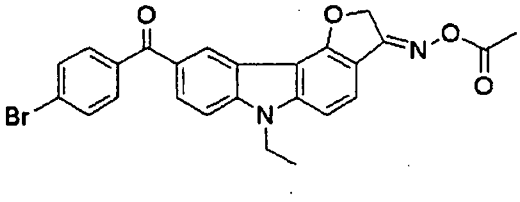

(A-117)

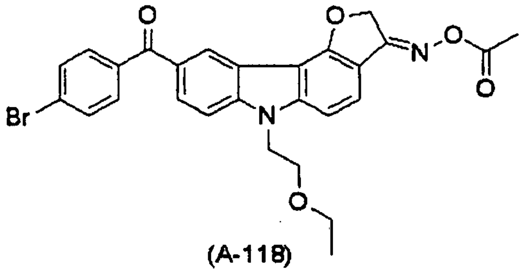

(A-118)

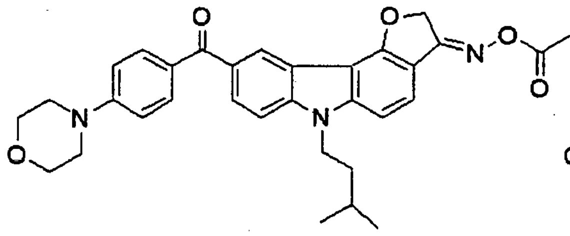

(A-119)

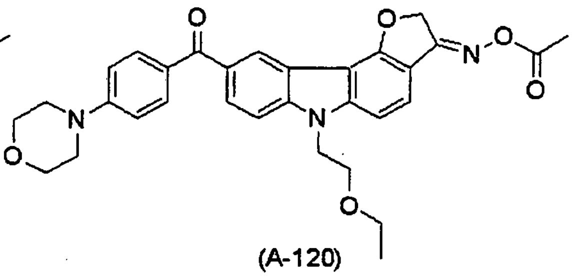

(A-120)

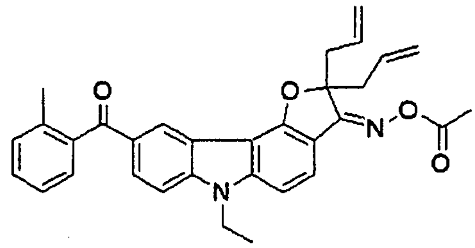

(A-121)

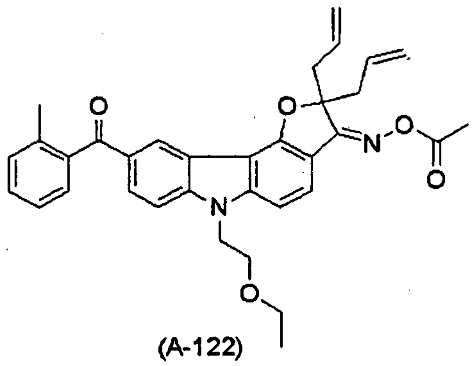

(A-122)

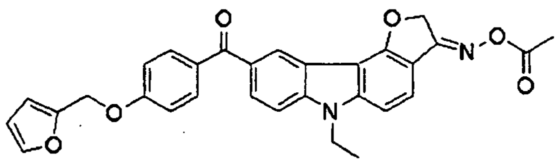

(A-123)

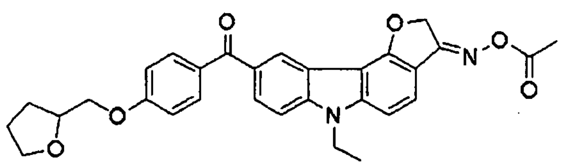

(A-124)

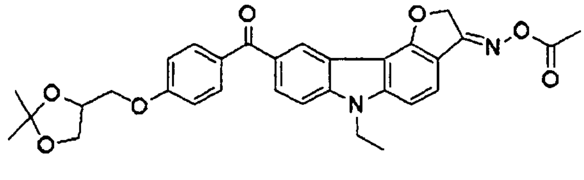

(A-125)

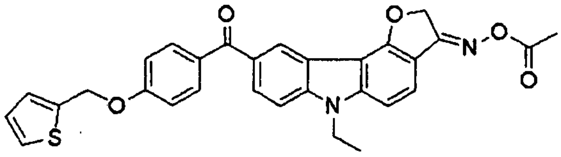

(A-126)

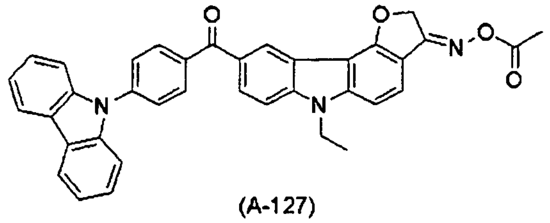

(A-127)

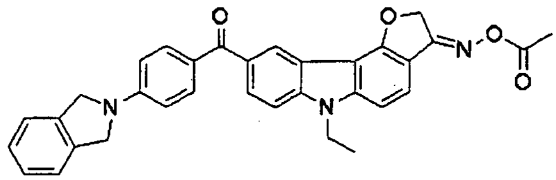

(A-128)

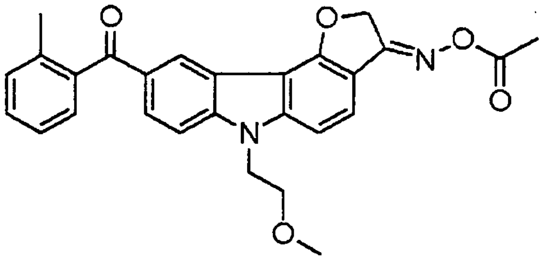

(A-129)

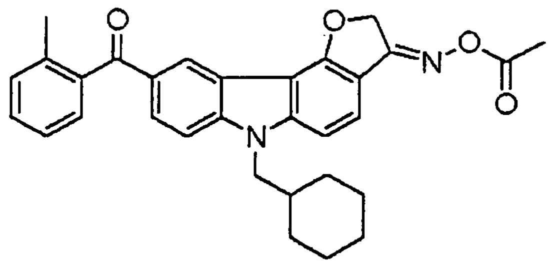

(A-130)

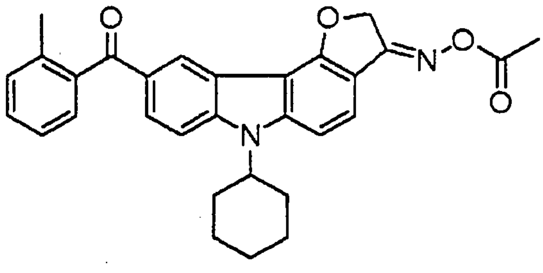

(A-131)

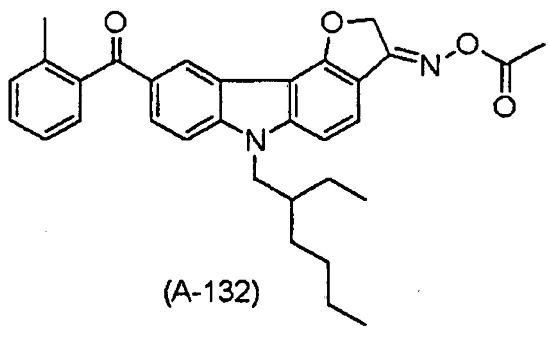

(A-132)

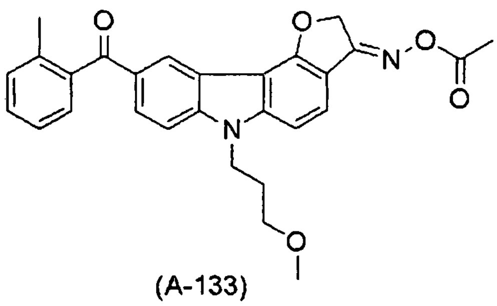

(A-133)

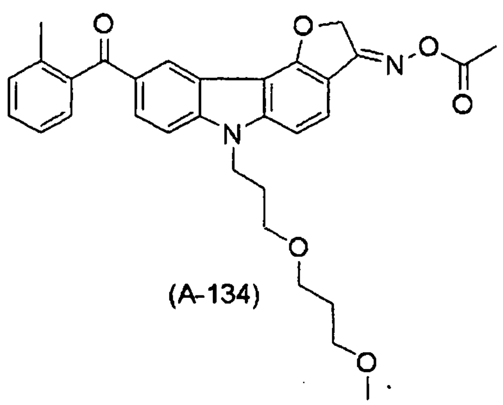

(A-134)

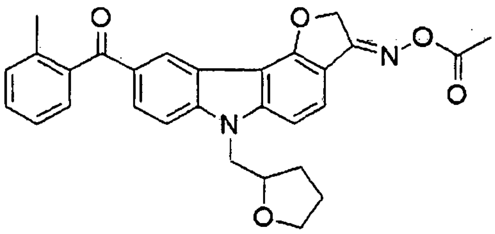

(A-135)

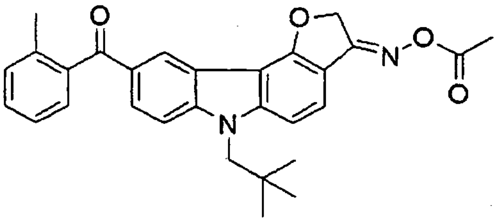

(A-136)

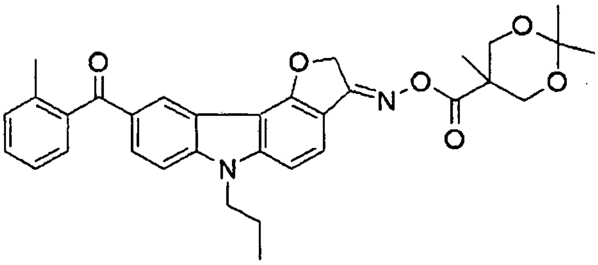

(A-137)

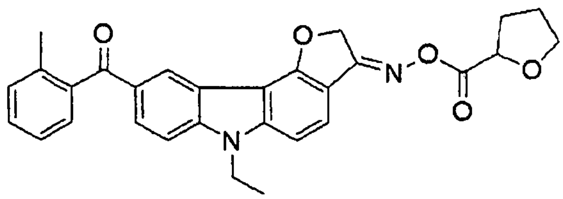

(A-138)

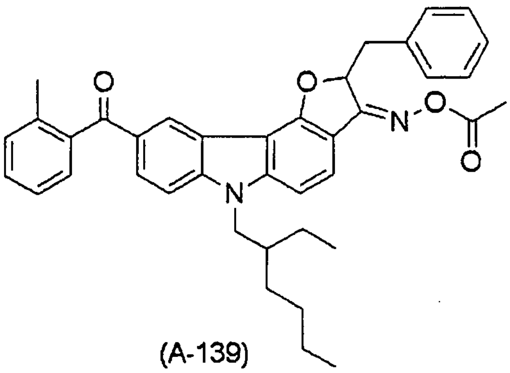

(A-139)

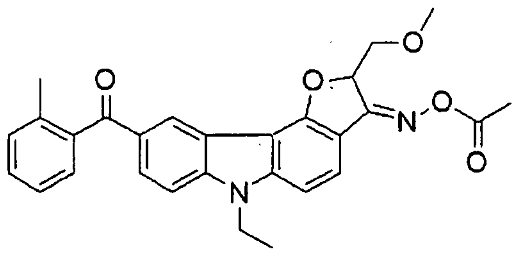

(A-140)

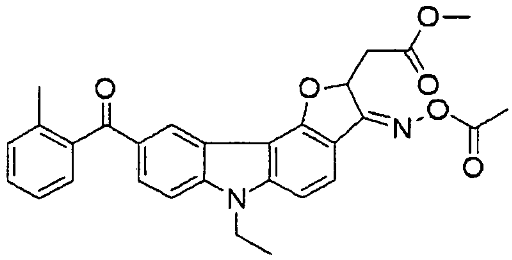

(A-141)

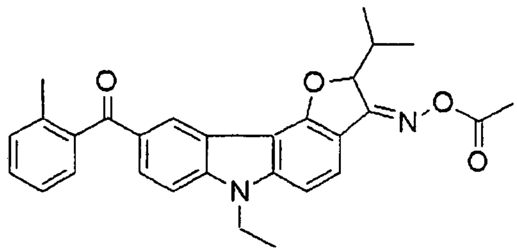

(A-142)

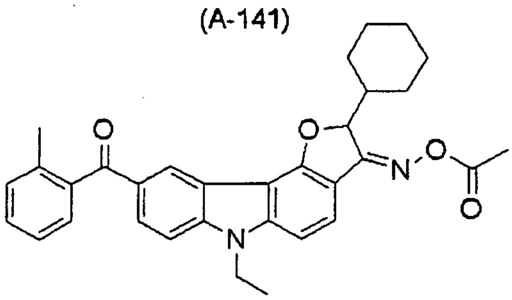

(A-143)

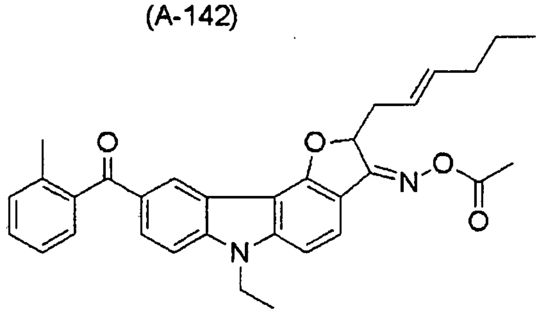

(A-144)

Among the specific examples (A-1) to (A-144), Exemplary Compounds (A-1) to (A-26), (A-38) to (A-68), (A-71), (A-73) to (A-77), (A-85) to (A-94), and (A-129) to (A-144) are preferable, and (A-2), (A-9), (A-11), (A-18), (A-20), (A-71), (A-75), and (A-132) are more preferable, from the viewpoint of improvement in molar absorption coefficient at 365 nm.

The specified oxime compound of the invention has a maximum absorption wavelength in a wavelength region of from 300 nm to 500 nm. The specified oxime compound preferably has an absorption wavelength in a wavelength region of from 300 nm to 480 nm, and has a high sensitivity particularly to a light source having wavelengths of around 365 nm.

The above-mentioned specified oxime compound has, in ethyl acetate, a molar absorption coefficient of 20,000 or more at a λmax within the wavelength range of 300 nm or more and less than 400 nm and a molar absorption coefficient of 400 or less at (λmax + 20 nm).

It is preferable that the specified oxime compound has, in ethyl acetate, a molar absorption coefficient of 20,000 to 100,000 at a λmax within the wavelength range of 300 nm or more and less than 400 nm and a molar absorption coefficient of 0 to 400 at (λmax + 20 nm).

It is more preferable that the specified oxime compound has, in ethyl acetate, a molar absorption coefficient of 20,000 to 60,000 at a λmax within the wavelength range of 300 nm or more and less than 400 nm and a molar absorption coefficient of 0 to 200 at λmax + 20 nm).

Thus, a sharper peak is observed in a UV absorption spectrum of the specified oxime compound in comparison to conventional oxime compounds. Therefore, the specified oxime compound exhibits a high sensitivity when it is exposed to a light source of around 365 nm. Thus, the polymerizable composition of the invention including the specified oxime compound is cured at a high sensitivity.

The molar absorption coefficient of the specified oxime compound is a value measured at a concentration of $2.41 \times 10^{-5}$ mol/L in an ethyl acetate solvent using a spectrophotometer for ultraviolet and visible region (trade name: CARRY-5 spectrophotometer, manufactured by Varian).

The specified oxime compound of the invention [i.e. the compound represented by Formula (1A) and (1B) can be synthesized by, for example, the method shown below. However, the method for the preparation of the compound is not limited to this method.

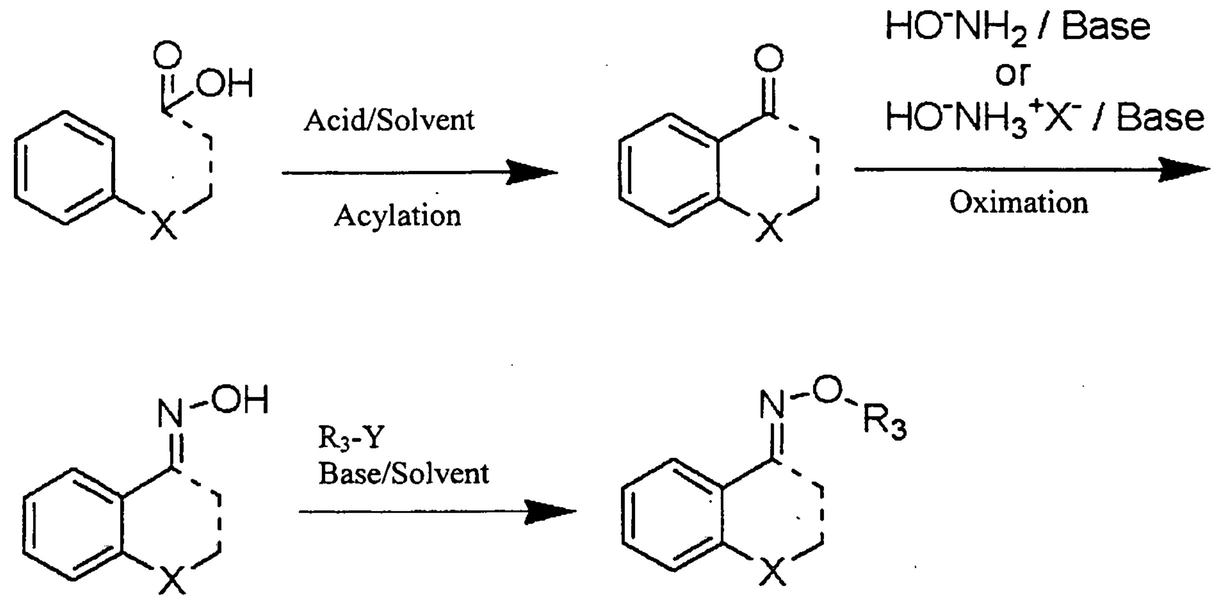


The specified oxime compound of the invention can be applied also to curable materials for the applications shown below by utilizing its photopolymerization initiating function.

Examples of the applications include printing inks such as screen printing inks, offset or flexographic printing inks, and UV-curable inks; white finishing or color finishing for woods or metals; powder coatings, specifically coating materials for paper, wood, metal or plastics; building or road marking; graphic reproduction techniques; holographic recording materials; image recording techniques; manufacture of printing plate precursors developable with organic solvents or aqueous alkali; sunlight-curable coatings for use in manufacture of screen printing masks; dental filling compositions; adhesives; pressure-sensitive adhesives; lamination resins; etching resists for both wet and dry thin films; solder resists; electroplating or permanent resists; photo-forming dielectrics for printed circuit boards and electronic circuits; various displays; optical switches; optical grating (interference grating); manufacture of optical circuits; manufacture of three-dimensional products by large-scale curing (UV curing in a transparent mold) or stereo lithography techniques (for example, as described in U.S. Patent No. 4,575,330); manufacture of composite materials (such as styrene polyesters optionally containing glass fibers and/or other fibers and other aids) or other thick layer compositions; resists for coating or sealing of electronic components and integrated circuits; optical fibers or optical lenses such as coatings for use in the manufacture of contact lenses or Fresnel lenses; manufacture of medical equipment, aids or implants; and manufacture of thermotropic gels such as those disclosed in German Patent No. 19,700,064 and European Patent No. 678,534.

The specified oxime compound of the invention is also suitable for use in other applications in which an acid is generated by irradiation with energy rays, for example light, and then the generated acid serves as a catalyst. For example, the specified oxime compound may also be used for materials for image formation, anti-counterfeit techniques, and detection of energy-ray dose in which a color reaction of a pigment precursor in the presence of the generated acid serving as a catalyst is used, and positive resists for use in manufacture of, for example, semiconductors, TFTs, color filters, and micromachine components in which a decomposition reaction in the presence of the produced acid serving as a catalyst is used.

As described above, the specified oxime compound of the invention can be used as a photopolymerization initiator. Therefore, it is preferable to apply the specified oxime compound together with a polymerizable compound to a photopolymerizable composition which is polymerized to cure in response to light (i.e., a photopolymerizable composition of the invention).

The polymerizable composition of the invention has a high sensitivity to light having a wavelength of around 365 nm and has excellent storage stability. Moreover, the polymerizable composition can form a cured film in which coloration due to heat aging can be prevented. Although the detailed mechanisms thereof are not clear, it is thought that since the specified oxime compound of the invention has such a high molar absorption coefficient, it can absorb light efficiently and generate a large amount of radicals, whereby a high sensitivity can be achieved. This is thought to be because a reaction between decomposition products from the specified oxime compound in heat aging is inhibited due to the inhibition of recombination of radicals and, as a result, coloration caused by such a reaction is inhibited.

In the invention, a color difference $\Delta Eab^*$ may be used for evaluating the coloration of a cured film caused by heat aging. The color difference $\Delta Eab^*$ can be measured with an MCPD-3000 manufactured by Otsuka Electronics Co., Ltd.

The conditions for the evaluation may be as follows. First, a cured film is formed by exposing the photopolymerizable composition of the invention to light at different exposure amounts ranging from 10 $mJ/cm^2$ to 2,500 $mJ/cm^2$ in an ultra-high pressure mercury lamp proximity-type exposure system (manufactured by Hitachi High-Tech Electronics Engineering Co., Ltd.) or an i-line stepper exposure system (trade name: FPA-3000i5+, manufactured by Canon Inc.) (365 nm). The cured film is developed as needed and then heated at 200°C for 1 hour.

The color difference ΔEab* of the cured film between before and after the heating is measured, so that the heat aging-induced coloration of the cured film can be evaluated.

The color difference ΔEab* between before and after the heating can be 5 or less when the photopolymerizable composition of the invention is used.

Hereinafter, the polymerizable composition of the invention is described, taking as examples, Polymerizable Composition (1) suitable for formation of a colored region of a color filter or the like (which may hereinafter referred to as "a polymerizable composition for a color filter"), and Polymerizable Composition (2) suitable for formation of a photosensitive layer of a planographic printing plate precursor. However, the polymerizable composition of the invention is not limited to them.

*Polymerizable Composition (1): Photopolymerizable Composition for Color Filter*

*(1)-(A) Specified Oxime Compound*

Polymerizable Composition (1) includes (A) the specified oxime compound. (A) The specified oxime compound which can be included in Polymerizable Composition (1) functions as a polymerization initiator in the composition.

The amount of the specified compound in Polymerizable Composition (1) is preferably from 0.5 to 40% by mass, more preferably from 1 to 35% by mass, and even more preferably from 1.5 to 30% by mass, with respect to the total solid content of the composition.

One specified oxime compound may be used singly, or two or more specified oxime compounds may be used in combination.

Polymerizable Composition (1) may also include a conventional photopolymerization initiator other than the specified oxime compound, provided it does not impair the effect of the invention. In this case, it is preferable to use a conventional photopolymerization initiator in an amount of 50% by mass or less of the specified oxime compound.

The photopolymerization initiator which can be used in combination with the specified oxime compound may be a compound which is decomposed by light and which starts and promotes the polymerization of the polymerizable compound described later. It preferably is a compound having an absorption in a wavelength region of from 300 nm to 500 nm. Specific examples thereof include organic halogenated compounds, oxadiazole compounds, carbonyl compounds, ketal compounds, benzoin compounds, acridine compounds, organic peroxide compounds, azo compounds, coumarin compounds, azide compounds, metallocene compounds, biimidazole compounds, organic boric acid compounds, disulfonic acid compounds, oxime ester compounds, onium salt compounds, and acyl phosphine oxide compounds.

*(1)-(B) Polymerizable Compound*

The polymerizable compound that may be used in Polymerizable Composition (1) may be an addition-polymerizable compound having at least one ethylenically unsaturated double bond and may be selected from compounds each having at least one ethylenically unsaturated terminal bond, preferably two or more ethylenically unsaturated terminal bonds. Such a class of compounds is widely known in the relevant industrial field, and such compounds may be used in the invention without particular limitations. Such compounds may be in the chemical form of a monomer or a prepolymer, specifically a dimer, a trimer or an oligomer, or any mixture thereof or any copolymer thereof. Examples of monomers and copolymer thereof include unsaturated carboxylic acids (such as acrylic acid, methacrylic acid, itaconic acid, crotonic acid, isocrotonic acid, and maleic acid), esters thereof, and amides thereof. Esters of unsaturated carboxylic acids and aliphatic polyhydric alcohol compounds, or amides of unsaturated carboxylic acids and aliphatic polyamines, are preferably used. Also preferably used are addition reaction products of unsaturated carboxylic acid esters or amides having a nucleophilic substituent such as a hydroxyl group, an amino group or a mercapto group, with monofunctional or polyfunctional isocyanates or epoxy compounds, and dehydration condensation reaction products of such esters or amides with monofunctional or polyfunctional carboxylic acids. Also preferred are addition reaction products of unsaturated carboxylic acid esters or amides having an electrophilic substituent such as an isocyanate group or an epoxy group, with monofunctional or polyfunctional alcohols, amines or thiols, and substitution reaction products of unsaturated carboxylic acid esters or amides having a halogen group or a leaving substituent such as a tosyloxy group, with monofunctional or polyfunctional alcohols, amines, or thiols. Other examples of preferable compounds include compounds obtained by replacing the unsaturated carboxylic acid in the above examples by an unsaturated phosphonic acid, styrene,

vinyl ether, or the like.

Examples of the monomer of the ester of the aliphatic polyhydric alcohol compound and the unsaturated carboxylic acid include acrylates such as ethylene glycol diacrylate, triethylene glycol diacrylate, 1,3-butanediol diacrylate, tetramethylene glycol diacrylate, propylene glycol diacrylate, neopentyl glycol diacrylate, trimethylolpropane triacrylate, trimethylolpropane tri(acryloyloxypropyl)ether, trimethylolethane triacrylate, hexanediol diacrylate, 1,4-cyclohexanediol diacrylate, tetraethylene glycol diacrylate, pentaerythritol diacrylate, pentaerythritol triacrylate, pentaerythritol tetraacrylate, dipentaerythritol diacrylate, dipentaerythritol hexaacrylate, sorbitol triacrylate, sorbitol tetraacrylate, sorbitol pentaacrylate, sorbitol hexaacrylate, tri(acryloyloxyethyl)isocyanurate, polyester acrylate oligomers, and isocyanurate EO-modified triacrylate.

Examples of the monomer of the ester also include methacrylates such as tetramethylene glycol dimethacrylate, triethylene glycol dimethacrylate, neopentyl glycol dimethacrylate, trimethylolpropane trimethacrylate, trimethylolethane trimethacrylate, ethylene glycol dimethacrylate, 1,3-butanediol dimethacrylate, hexanediol dimethacrylate, pentaerythritol dimethacrylate, pentaerythritol trimethacrylate, pentaerythritol tetramethacrylate, dipentaerythritol dimethacrylate, dipentaerythritol hexamethacrylate, sorbitol trimethacrylate, sorbitol tetramethacrylate, bis[p-(3-methacryloxy-2-hydroxypropoxy)phenyl]dimethylmethane, and bis[p-(methacryloxyethoxy)phenyl]dimethylmethane.

Examples of the monomer of the ester also include itaconates such as ethylene glycol diitaconate, propylene glycol diitaconate, 1,3-butanediol diitaconate, 1,4-butanediol diitaconate, tetramethylene glycol diitaconate, pentaerythritol diitaconate, and sorbitol tetraitaconate;
crotonates such as ethylene glycol dicrotonate, tetramethylene glycol dicrotonate, pentaerythritol dicrotonate, and sorbitol tetradicrotonate;
isocrotonates such as ethylene glycol diisocrotonate, pentaerythritol diisocrotonate, and sorbitol tetraisocrotonate; and maleates such as ethylene glycol dimaleate, triethylene glycol dimaleate, pentaerythritol dimaleate, and sorbitol tetramaleate.

Examples of other esters include the aliphatic alcohol esters described in Japanese Patent Application Publication (JP-B) No. 51-47334 and JP-ANo. 57-196231, the aromatic skeleton-containing compounds described in JP-A Nos. 59-5240, 59-5241 and 02-226149, and the amino group-containing compounds described in JP-A No. 01-165613. A mixture of monomers selected from the ester monomers described above may also be used.

Examples of the monomer of the amide of the aliphatic polyamine compound and the unsaturated carboxylic acid include methylenebis-acrylamide, methylenebis-methacrylamide, 1,6-hexamethylenebis-acrylamide, 1,6-hexamethylenebis-methacrylamide, diethylenetriaminetrisacrylamide, xylylenebisacrylamide, and xylylenebismethacrylamide.

Examples of other preferred amide monomers include the cyclohexylene structure-containing compounds described in JP-B No. 54-21726.

Addition-polymerizable urethane compounds produced by an addition reaction of isocyanate with a hydroxyl group are also preferred, examples of which include the vinyl urethane compounds described in JP-B No. 48-41708, which have two or more polymerizable vinyl groups within a molecule and are produced by adding a hydroxyl group-containing vinyl monomer represented by the following Formula (A) to a polyisocyanate compound having two or more isocyanate groups within a molecule.

$$CH_2=C(R^4)COOCH_2CH(R^5)OH \qquad (A)$$

In Formula (A), $R^4$ and $R^5$ each represent H or $CH_3$.

Also preferred are the urethane acrylates described in JP-A No. 51-37193, JP-B No. 02-32293 and JP-B No. 02-16765, and the ethylene oxide skeleton-containing urethane compounds described in JP-B Nos. 58-49860, 56-17654, 62-39417, and 62-39418. Photopolymerizable compositions having excellent photoresponsive speed can also be obtained using addition-polymerizable compounds having an amino or sulfide structure in the molecule, which are disclosed in JP-A Nos. 63-277653, 63-260909 and 01-105238.

Other examples include polyfunctional acrylates and methacrylates such as polyester acrylates and epoxy acrylates produced by a reaction of epoxy resins with (meth)acrylic acid (for example those disclosed in JP-A No. 48-64183, JP-B No. 49-43191 and JP-B No. 52-30490); and the specific unsaturated compounds described in JP-B Nos. 46-43946, 01-40337 and 01-40336, and the vinylphosphonic acid compounds described in JP-A No. 02-25493. In some cases, the perfluoroalkyl group-containing structures described in JP-ANo. 61-22048 are preferably used. Photocurable monomers and oligomers as described in Journal of the Adhesion Society of Japan Vol. 20, No. 7 pp. 300 to 308 (1984) may also be used.

Details of how to use the addition-polymerizable compounds, such as what structure should be used, whether they should be used alone or in combination, or what amount should be added, may be freely determined depending on the final performance design of the polymerizable composition. For example, they may be selected from the following viewpoints.

In view of sensitivity, a structure having a higher content of the unsaturated groups per molecule is preferable,

and difunctional or higher functional structures are preferred in many cases. In order to increase the strength of the cured film, tri- or higher-functional structures are preferred. A method of using a combination of compounds having different numbers of functional groups and/or different types of polymerizable groups (for example, compounds selected from an acrylic ester, a methacrylic ester, a styrene compound, a vinyl ether compound) is also effective for controlling both of sensitivity and strength.

How to select and use the addition-polymerizable compound is also an important factor for the compatibility with or dispersibility to other components of the polymerizable composition (such as a photopolymerization initiator, a colorant (a pigment and/or a dye), and a binder polymer). For example, in some cases, the compatibility may be improved by using a low-purity compound or by using a combination of two or more compounds. A particular structure may also be selected in order to improve adhesion to the hard surface of a support or the like.

The amount of the polymerizable compound in Polymerizable Composition (1) is preferably from 0.1 to 60% by mass, more preferably from 0.2 to 50% by mass, and even more preferably from 0.3 to 40% by mass, with respect to the total solid content of the composition.

The photopolymerizable compounds may be used singly or may be used in combination of two or more of them.

*(1)-(C) Colorant*

Polymerizable Composition (1) may further include (C) a colorant. When Polymerizable Composition (1) includes a colorant, a colored polymerizable composition with a desired color is obtained.

Because Polymerizable Composition (1) includes the specified oxime compound, which is (A) the polymerization initiator of the invention having a high sensitivity to a light source having a short wavelength of around 365 nm, it can be cured at a high sensitivity even when it includes a colorant at a high concentration.

The colorant to be used in Polymerizable Composition (1) is not particularly restricted. Conventionally-known dyes or pigments may be used singly or in combination. These may be selected appropriately depending on the intended application of a photopolymerizable composition. When the photopolymerizable composition of the invention is used for the production of a color filter, both colorants of chromatic colors such as R, G and B (i.e., chromatic color colorants) which form color pixels of color filters and colorants of a black color (i.e., black colorants) which are generally used for the formation of black matrix may be available.

Because the polymerizable composition of the invention including (A) the specified oxime compound can be cured at a high sensitivity even when the amount of light exposure is small, it can be used particularly suitably for a polymerizable composition containing a black colorant which hardly allows light to pass.

Colorants applicable to Polymerizable Composition of the invention are described in detail below, by describing colorants suitable for color filters as examples.

Chromatic pigments to be used may be various conventionally-known inorganic or organic pigments. Higher transparency is preferable regardless of whether the pigment is inorganic or organic. From this point of view, pigment particles are preferably smaller. When also considering handleability, the average particle size of the pigments is preferably from 0.01 μm to 0.1 μm, more preferably from 0.01 μm to 0.05 μm.

Examples of the inorganic pigments include metal compounds, typical examples of which include metal oxides and metal complex salts. Specific examples thereof include oxides of iron, cobalt, aluminum, cadmium, lead, copper, titanium, magnesium, chromium, zinc, antimony, or other metals, and complex oxides of the above metals.

Examples of the organic pigments include:

C.I. Pigment Yellow 11, 24, 31, 53, 83, 93, 99, 108, 109, 110, 138, 139, 147, 150, 151, 154, 155, 167, 180, 185, 199;
C.I. Pigment Orange 36, 38, 43, 71;
C.I. Pigment Red 81, 105, 122, 149, 150, 155, 171, 175, 176, 177, 209, 220, 224,242,254,255,264,270;
C.I. Pigment Violet 19, 23, 32, 39;
C.I. Pigment Blue 1, 2, 15, 15:1, 15:3, 15:6, 16, 22, 60, 66;
C.I. Pigment Green 7, 36, 37;
C.I. Pigment Brown 25, 28;
C.I. Pigment Black 1, 7; and
carbon black.

In the invention, pigments having a basic N atom in their structures are particularly preferably used. The basic N atom-containing pigments exhibit high dispersibility in Polymerizable Composition (1). Although the reason for that has not been clearly understood yet, it is suggested that high affinity between the photosensitive polymerizable component and the pigment may have an effect.

In the invention, examples of the pigments that are preferably used include, but are not limited to, the pigments listed below:

C.I. Pigment Yellow 11, 24, 108, 109, 110, 138, 139, 150, 151, 154, 167, 180, 185;
C.I. Pigment Orange 36, 71;
C.I. Pigment Red 122, 150, 171, 175, 177, 209, 224, 242, 254, 255, 264;
C.I. Pigment Violet 19, 23, 32;
C.I. Pigment Blue 15:1, 15:3, 15:6, 16, 22, 60, 66; and
C.I. Pigment Black 1.

Only a single organic pigment may be used, or a combination of plural organic pigments may be used to increase color purity.
Specific examples of the combination are described below. For example, a red pigment is used which is one of an anthraquinone pigment, a perylene pigment and a diketopyrrolopyrrole pigment, or which is a mixture of at least one of these pigments with a disazo yellow pigment, an isoindolin yellow pigment, a quinophthalone yellow pigment, or a perylene red pigment.
For example, the anthraquinone pigment may be C.I. Pigment Red 177, the perylene pigment may be C.I. Pigment Red 155 or C.I. Pigment Red 224, and the diketopyrrolopyrrole pigment may be C.I. Pigment Red 254. In view of color reproducibility, a mixture of at least one of these pigments and C.I. Pigment Yellow 139 is preferably used.
The mass ratio of the red pigment to the yellow pigment (red pigment / yellow pigment) is preferably in the range of from 100/5 to 100/50. When the mass ratio is within the above range, color purity can be increased, and Polymerizable Composition (1) can be suitably applied to the National Television System Committee (NTSC) target hue.
In particular, the mass ratio is most preferably in the range of from 100/10 to 100/30. In the case of a combination of different red pigments, the mass ratio thereof may be adjusted depending on the hue.
A green pigment of a halogenated phthalocyanine pigment may be used alone or in a form of a mixture with a disazo yellow pigment, a quinophthalone yellow pigment, an azomethine yellow pigment, or an isoindolin yellow pigment.
For example, C.I. Pigment Green 7, 36 or 37 is preferably mixed with C.I. Pigment Yellow 83, C.I. Pigment Yellow 138, C.I. Pigment Yellow 139, C.I. Pigment Yellow 150, C.I. Pigment Yellow 180, or C.I. Pigment Yellow 185. The mass ratio of the green pigment to the yellow pigment (green pigment / yellow pigment) is preferably in the range of from 100/5 to 100/150. When the mass ratio is within the above range, color purity can be increased, and Polymerizable Compound (1) can be suitably applied to the NTSC target hue.
In particular, the mass ratio is preferably in the range of from 100/30 to 100/120.
A blue pigment of a phthalocyanine pigment may be used alone or in a form of a mixture with a dioxazine violet pigment.
For example, C.I. Pigment Blue 15:6 is preferably mixed with C.I. Pigment Violet 23. The mass ratio of the blue pigment to the violet pigment (blue pigment / violet pigment) is preferably in the range of from 100/0 to 100/30, more preferably 100/10 or less.
Carbon, titanium carbon, iron oxide, and titanium oxide may be used alone or in any combination thereof as a pigment for black matrices, and a combination of carbon and titanium carbon is preferred. The mass ratio of carbon to titanium carbon (carbon / titanium carbon) is preferably from 100/0 to 100/60.
Moreover, a titanium black dispersion may also be used as a pigment for black matrices. The titanium black dispersion is described in detail below.
The titanium black dispersion is a dispersion which contains titanium black as a color material.
When a polymerizable composition includes titanium black in the form of a titanium black dispersion prepared in advance, the dispersibility and the dispersion stability of the titanium black are improved.
Titanium black is described below.

*Titanium Black*

The titanium black which can be used in the invention is a black particle having titanium atoms. It preferably is low-order titanium oxide or titanium oxynitride. The surfaces of titanium black particles may be modified as required according to a purpose such as improvement of dispersibility and control of cohesiveness. The particles may be coated with silicon oxide, titanium oxide, germanium oxide, aluminum oxide, magnesium oxide, or zirconium oxide, and they may also be treated with a water-repellent material such as that disclosed in JP-A No. 2007-302836.
Examples of the method for producing titanium black include, but are not limited to, a method in which a mixture of titanium dioxide and metal titanium is heated and reduced in a reducing atmosphere (JP-A No. 49-5432), a method in which ultra-fine titanium dioxide prepared by high-temperature hydrolysis of titanium tetrachloride is reduced in a reducing atmosphere containing hydrogen (JP-A No. 57-205322), a method in which titanium dioxide or titanium hydroxide is reduced at a high temperature in the presence of ammonia (JP-A Nos. 60-65069 and 61-201610), and a method in which a vanadium compound is adhered to titanium dioxide or titanium hydroxide, followed by high-temperature reduction in the presence of ammonia (JP-ANo. 61-201610).

The particle diameter of the particle of titanium black is not particularly limited, but from the viewpoint of dispersibility and coloring property, the particle diameter is preferably from 3 to 2,000 nm, more preferably from 10 to 500 nm, and even more preferably from 20 to 200 nm.

The specific surface area of titanium black is not particularly limited, but in order for the titanium black to have a desired water repellency after being subjected to surface treatment with a water-repellent agent, the specific surface area measured by the BET method is usually from about 5 to about 150 $m^2/g$, and particularly from about 20 to about 100 $m^2/g$.

Examples of commercial products of titanium black include, but not limited to, TITANIUM BLACK 10S, 12S, 13R, 13M, 13M-C, 13R and 13R-N (all trade names, manufactured by Mitsubishi Materials Corporation), and TILACK D (trade name, manufactured by Ako Kasei Co., Ltd).

When the colorant included in Polymerizable Composition (1) is a dye, a colored composition can be obtained in which the dye is uniformly dissolved.

Examples of the dye that may be used as the colorant in Polymerizable Composition (1) include, but are not limited to, known dyes for conventional color filters. Examples of the dyes that may be used include the dyes disclosed in JP-A Nos. 64-90403, 64-91102, 01-94301, and 06-11614, Japanese Patent No. 2592207, U.S. Patent Nos. 4,808,501, 5,667,920 and 5,059,500, and JP-A Nos. 05-333207, 06-35183, 06-51115, 06-194828, 08-211599, 04-249549, 10-123316, 11-302283, 07-286107, 2001-4823, 08-15522, 08-29771, 08-146215, 11-343437, 08-62416, 2002-14220, 2002-14221, 2002-14222, 2002-14223, 08-302224, 08-73758, 08-179120, and 08-151531.

Regarding chemical structure, pyrazole azo dyes, anilino azo dyes, triphenylmethane dyes, anthraquinone dyes, anthrapyridone dyes, benzylidene dyes, oxonol dyes, pyrazolotriazole azo dyes, pyridone azo dyes, cyanine dyes, phenothiazine dyes, pyrrolopyrazole azomethine dyes, xanthene dyes, phthalocyanine dyes, benzopyran dyes, and indigo dyes may be used.

In some resist systems involving water or alkali development, acid dyes and/or derivatives thereof may be preferably used in order to completely remove the binder and/or dye in an unirradiated area (unexposed area) by development.

Other dyes such as direct dyes, basic dyes, mordant dyes, acid mordant dyes, azoic dyes, disperse dyes, oil-soluble dyes, food dyes, and/or derivatives thereof may be effectively used.

Any acid dye having an acidic group such a sulfonic acid group or a carboxylic acid group may be used. The acid dye may be selected taking into account all the necessary properties such as solubility in an organic solvent or developer, ability to form a salt with a basic compound, absorbance, interaction with other components in the composition, light resistance, and heat resistance.

Examples of the acid dye include, but are not limited to, the following dyes: Acid Alizarin Violet N: Acid Black 1, 2, 24, 48; Acid Blue 1, 7, 9, 15, 18, 23, 25, 27, 29, 40, 45, 62, 70, 74, 80, 83, 86, 87, 90, 92, 103, 112, 113, 120, 129, 138, 147, 158, 171, 182, 192, 243, 324:1; Acid Chrome Violet K; Acid Fuchsin; Acid Green 1, 3, 5, 9, 16, 25, 27, 50; Acid Orange 6, 7, 8, 10, 12, 50, 51, 52, 56, 63, 74, 95; Acid Red 1, 4, 8, 14, 17, 18, 26, 27, 29, 31, 34, 35, 37, 42, 44, 50, 51, 52, 57, 66, 73, 80, 87, 88, 91, 92, 94, 97, 103, 111, 114, 129, 133, 134, 138, 143, 145, 150, 151, 158, 176, 183, 198, 211, 215, 216, 217, 249, 252, 257, 260, 266, 274; Acid Violet 6B, 7, 9, 17, 19; Acid Yellow 1, 3, 7, 9, 11, 17, 23, 25, 29, 34, 36, 42, 54, 72, 73, 76, 79, 98, 99, 111, 112, 114, 116, 184, 243; Food Yellow 3; and derivatives of these dyes.

Particularly preferred examples of the acid dye include Acid Black 24; Acid Blue 23, 25, 29, 62, 80, 86, 87, 92, 138, 158, 182, 243, 324:1; Acid Orange 8, 51, 56, 63, 74; Acid Red 1, 4, 8, 34, 37, 42, 52, 57, 80, 97, 114, 143, 145, 151, 183, 217; Acid Violet 7; Acid Yellow 17, 25, 29, 34, 42, 72, 76, 99, 111, 112, 114, 116, 184, 243; Acid Green 25; and derivatives of these dyes.

Acid dyes such as azo, xanthene and phthalocyanine dyes other than the above are also preferred. Acid dyes such as C.I. Solvent Blue 44, 38, C.I. Solvent Orange 45, Rhodamine B, and Rhodamine 110, and derivatives of these acid dyes are also preferably used.

In particular, the colorant is preferably selected from triarylmethane dyes, anthraquinone dyes, azomethine dyes, benzylidene dyes, oxonol dyes, cyanine dyes, phenothiazine dyes, pyrrolopyrazole azomethine dyes, xanthene dyes, phthalocyanine dyes, benzopyran dyes, indigo dyes, pyrazole azo dyes, anilino azo dyes, pyrazolotriazole azo dyes, pyridone azo dyes, and anthrapyridone dyes.

The colorant that may be included in Polymerizable Composition (1) is preferably a dye, or a pigment whose average particle size r (unit: nm) satisfies the relation $20 \leq r \leq 300$, more preferably $125 \leq r \leq 250$, particularly preferably $130 \leq r \leq 200$. Red and green pixels having high contrast ratio and high light transmittance can be obtained by using pigments with such an average particle size r. As used herein, the term "average particle size" refers to the average particle size of secondary particles formed by aggregation of primary particles (single fine crystals) of a pigment.

The particle size distribution of the secondary particles of the pigment for use in the invention (hereinafter, simply referred to as "particle size distribution") is preferably such that 70% by mass or more, more preferably 80% by mass or more, of the whole of the secondary particles fall within (the average particle size $\pm$ 100) nm.

The pigment having the average particle size and the particle size distribution described above may be prepared

by a process including mixing and dispersing a commercially available pigment and another optional pigment (generally having an average particle size more than 300 nm), preferably as a pigment mixture liquid with a dispersing agent and a solvent, while grinding them with a grinding machine such as a bead mill or a roll mill. The resultant pigment is generally in the form of a pigment dispersion liquid.

The amount of the colorant included in Polymerizable Composition (1) is preferably from 30% by mass to 95% by mass, more preferably from 40% by mass to 90% by mass, and even more preferably from 50% by mass to 80% by mass, with respect to the total solid content of the polymerizable composition.

By adjusting the amount of the colorant to be within the above range, a moderate chromaticity can be obtained when a color filter is produced using Polymerizable Composition (1). Moreover, because photo-curing proceeds sufficiently, so that the strength as a film can be maintained, it is possible to prevent a development latitude in alkaline development from narrowing.

That is, since the specified oxime compound, which is (A) the photopolymerization initiator in the invention, has a high light absorption efficiency, a remarkable effect of improving the sensitivity is exhibited even when a colorant is included at a high concentration in a photopolymerizable composition.

*(1)-(D) Pigment Dispersant*

When Polymerizable Composition (1) includes a pigment such as titanium black or an organic pigment as (C) the colorant, it is preferable to add (D) a pigment dispersant from the viewpoint of improving the dispersibility of the pigment.

Examples of the pigment dispersant which may be used in the invention include polymer dispersants such as polyamideamines and salts thereof, polycarboxylic acids and salts thereof, high-molecular-weight unsaturated acid esters, modified polyurethanes, modified polyesters, modified poly(meth)acrylates, (meth)acrylic copolymers, or naphthalenesulfonic acid-formalin condensates, polyoxyethylene alkyl phosphates, polyoxyethylene alkylamines, alkanolamines, and pigment derivatives.

The polymer dispersants can be further classified in accordance with the structure thereof into straight chain polymers, terminal-modified polymers, graft polymers, and block polymers.

The polymer dispersant adsorbs on the surface of the pigments to act so as to prevent re-aggregation of the pigments. Therefore, polymers having an anchor site capable of anchoring to a pigment surface, that is, terminal-modified polymers, graft polymers and block polymers can be listed as preferable structures.

On the other hand, pigment derivatives have an effect of facilitating the adsorption of polymer dispersants by modifying the pigment surface.

Examples of pigment dispersants that may be used in the invention include DISPERBYK 101 (polyamide amine phosphate), 107 (carboxylic acid ester), 110 (acid group-containing copolymer), 130 (polyamide), 161, 162, 163, 164, 165, 166, and 170 (high-molecular-weight polymer), and BYK-P104 and P105 (high-molecular-weight unsaturated polycarboxylic acid) (all trade names, manufactured by BYK Chemie); EFKA 4047, 4050, 4010, and 4165 (polyurethane dispersants), EFKA 4330, 4340 (block copolymer), 4400, 4402 (modified polyacrylate), 5010 (polyester amide), 5765 (high-molecular-weight polycarboxylate), 6220 (fatty acid polyester), 6745 (phthalocyanine derivative), and 6750 (azo pigment derivative) (all trade names, manufactured by EFKA); AJISPER PB821 and PB822 (all trade names, manufactured by Ajinomoto Fine-Techno Co., Inc.); FLOWLEN TG-710 (urethane oligomer) and POLYFLOW Nos. 50E and 300 (acrylic copolymer) (all trade names, manufactured by Kyoeisha Chemical Co., Ltd.); DISPARLON KS-860, 873SN, 874, #2150 (aliphatic polycarboxylic acid), #7004 (polyether ester), DA-703-50, DA-705, and DA-725 (all trade names, manufactured by Kusumoto Chemicals, Ltd.); DEMOL RN, N (naphthalenesulfonic acid-formalin polycondensate), MS, C, SN-B (aromatic sulfonic acid-formalin polycondensate), HOMOGENOL L-18 (high-molecular-weight polycarboxylic acid), EMULGEN 920, 930, 935, and 985 (polyoxyethylene nonyl phenyl ether), and ACETAMIN 86 (stearylamine acetate) (all trade names, manufactured by Kao Corporation); SOLSPERSE 5000 (phthalocyanine derivative), 22000 (azo pigment derivative), 13240 (polyester amine), 3000, 17000, 27000 (polymer having a functional portion at a terminal), 24000, 28000, 32000, and 38500 (graft polymers) (all trade names, manufactured by The Lubrizol Corporation); and NIKKOL T106 (polyoxyethylene sorbitan monooleate) and MYS-IEX (polyoxyethylene monostearate) (all trade names, manufactured by Nikko Chemicals Co., Ltd.).

Only a single pigment dispersant may be used, or two or more of pigment dispersants may be used in combination. In the invention, it is particularly preferable to use a pigment derivative and a polymeric dispersant in combination.

In the invention, the amount of the pigment dispersant in Polymerizable Composition (1) is preferably from 1 to 80% by mass, more preferably from 5 to 70% by mass, even more preferably from 10 to 60% by mass, based on the mass of the pigment.

For example, when a polymeric dispersant is used, the amount thereof is preferably from 5 to 100% by mass, more preferably from 10 to 80% by mass, based on the mass of the pigment.

When a pigment derivative is used, the amount thereof is preferably from 1 to 30% by mass, more preferably from 3 to 20% by mass, particularly preferably from 5 to 15% by mass of the pigment, based on the mass of the pigment.

When a pigment as a colorant and a dispersant are included in Polymerizable Composition (1), the total amount of the colorant and the dispersant is preferably from 30 to 90% by mass, more preferably from 40 to 85% by mass, even more preferably from 50 to 80% by mass, based on the mass of the total solid contents of the polymerizable composition, from the viewpoints of curing sensitivity and color density.

Polymerizable Composition (1) may, if necessary, further include arbitrary components which are described in detail below.

The arbitrary components which may be included in Polymerizable Composition (1) are described below.

*(1)-(E) Sensitizing Agent*

Polymerizable Composition (1) may include a sensitizing agent for the purpose of improving the efficiency of radical generation of a radical initiator and lengthening the sensing wavelength.

As the sensitizing agent which may be used in the invention, a sensitizing agent is preferable which sensitizes (A) the specified oxime compound through an electron transfer mechanism or an energy transfer mechanism.

The sensitizing agent which may be included in Polymerizable Composition (1) may belong to any of the groups of compounds described below and may have an absorption wavelength in the range of 300 nm to 450 nm.

Examples thereof include polynuclear aromatic compounds (such as phenanthrene, anthracene, pyrene, perylene, triphenylene, and 9,10-dialkoxyanthracene), xanthenes (such as fluorescein, eosin, erythrosine, rhodamine B, and rose bengal), thioxanthones (such as isopropylthioxanthone, diethylthioxanthone and chlorothioxanthone), cyanines (such as thiacarbocyanine and oxacarbocyanine), merocyanines (such as merocyanine and carbomerocyanine), phthalocyanines, thiazines (such as thionine, methylene blue and toluidine blue), acridines (such as acridine orange, chloroflavin and acriflavin), anthraquinones (such as anthraquinone), squaryliums (such as squarylium), coumarins (such as 7-diethylamino-4-methylcoumarin), ketocoumarin, phenothiazines, phenazines, styrylbenzenes, azo compounds, diphenylmethane, triphenylmethane, distyrylbenzenes, carbazoles, porphyrin, spiro compounds, quinacridone, indigo, styryl compounds, pyrylium compounds, pyrromethene compounds, pyrazolotriazole compounds, benzothiazole compounds, barbituric acid derivatives, thiobarbituric acid derivatives, aromatic ketone compounds such as acetophenone, benzophenone, thioxanthone, and Michler's ketone, and heterocyclic compounds such as N-aryloxazolidinone.

Examples of more preferred sensitizing agents to be included in Polymerizable Composition (1) include compounds represented by the following Formulae (e-1) to (e-4).

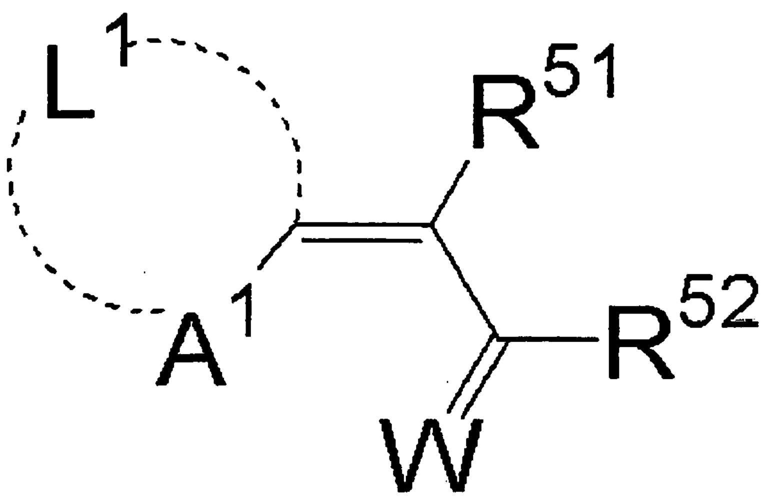

(e-1)

In Formula (e-1), $A^1$ represents a sulfur atom or $NR^{50}$, $R^{50}$ represents an alkyl group or an aryl group, $L^1$ represents a nonmetallic atom group that forms a basic nuclear of the pigment together with $A^1$ and the carbon atom adjacent to $L^1$, $R^{51}$ and $R^{52}$ each independently represent a hydrogen atom or a monovalent nonmetallic atom group, and $R^{51}$ and $R^{52}$ may be bonded to each other to form an acidic nuclear of the pigment, and W represents an oxygen atom or a sulfur atom.

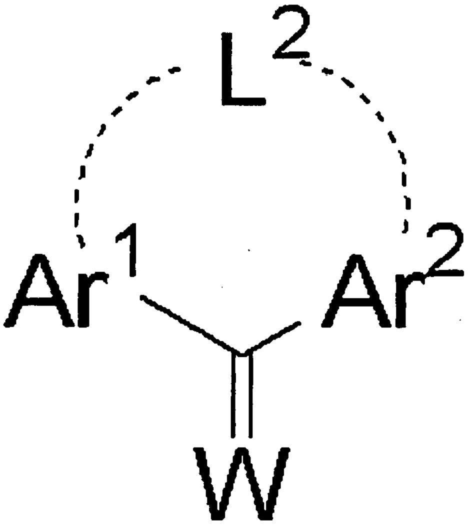

(e-2)

In Formula (e-2), $Ar^1$ and $Ar^2$ each independently represent an aryl group and are linked to each other via $-L^2-$, in which $-L^2-$ represents -O- or -S-, and W has the same definition as in Formula (e-1).

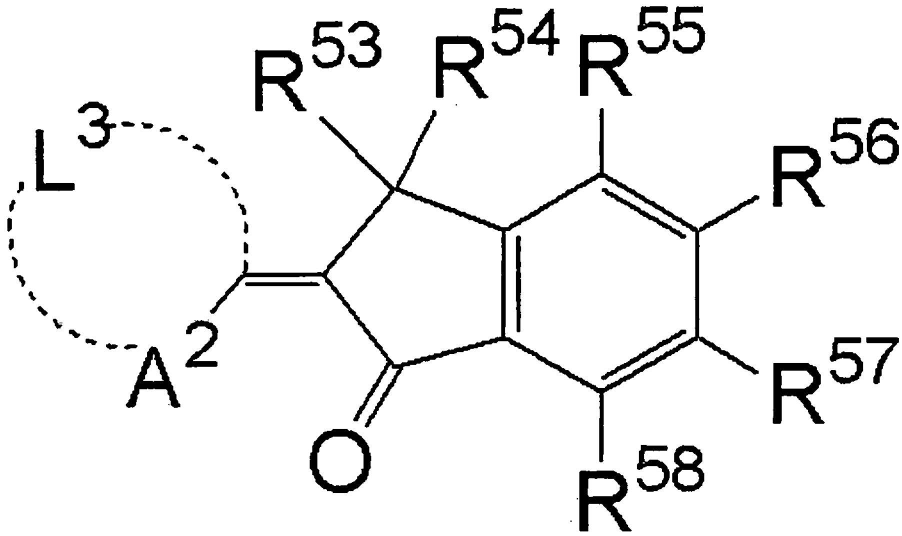

(e-3)

In Formula (e-3), $A^2$ represents a sulfur atom or $NR^{59}$; $L^3$ represents a nonmetallic atom group that forms a basic nuclear of the pigment together with $A^2$ and the carbon atom adjacent to $L^3$; $R^{53}$, $R^{54}$, $R^{55}$, $R^{56}$, $R^{57}$, and $R^{58}$ each independently represent a monovalent nonmetallic atom group; and $R^{59}$ represents an alkyl group or an aryl group.

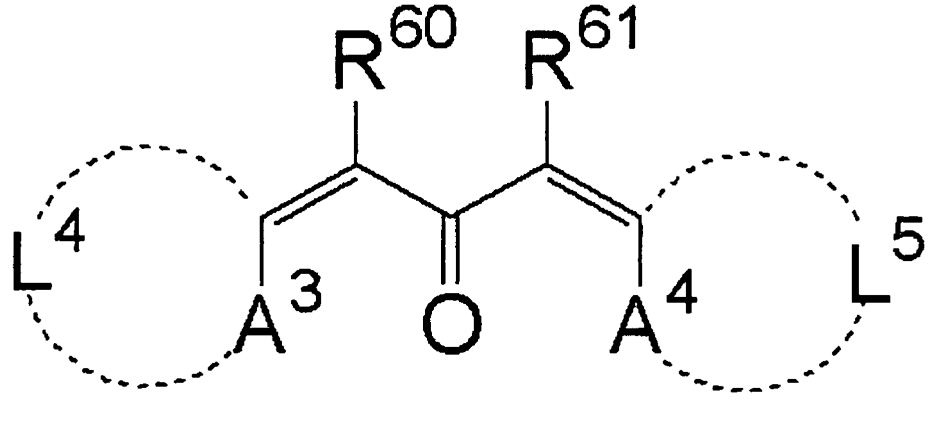

(e-4)

In Formula (e-4), $A^3$ and $A^4$ each independently represent -S- or -$NR^{62}$; $R^{62}$ represents a substituted or unsubstituted alkyl group or a substituted or unsubstituted aryl group; $L^4$ represents a nonmetallic atom group that forms a basic nuclear of the pigment together with adjacent $A^3$ and the carbon atom adjacent to $L^4$; $L^5$ represents a nonmetallic atom group that forms a basic nuclear of the pigment together with adjacent $A^4$ and the carbon atom adjacent to $L^5$;and $R^{60}$ and $R^{61}$ each independently represent a monovalent nonmetallic atom group or may be bonded to each other to form an aliphatic or aromatic ring.

The sensitizing agent which is preferably included in Polymerizable Composition (1) may be at least one selected from a compound represented by the following Formula (II) and a compound represented by the following Formula (III).

A single compound selected from these sensitizing agents may be used, or two or more compounds selected from these sensitizing agents may be used in combination.

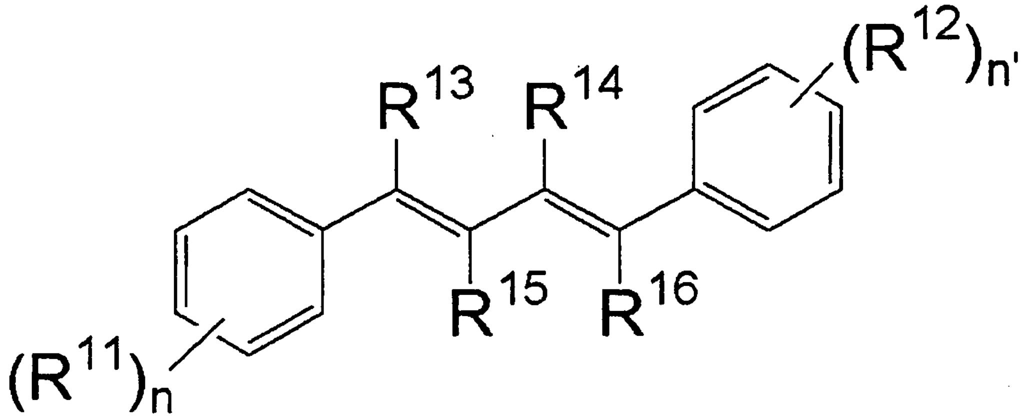

(II)

In Formula (II), $R^{11}$ and $R^{12}$ each independently represent a monovalent substituent, $R^{13}$, $R^{14}$, $R^{15}$, and $R^{16}$ each independently represent a hydrogen atom or a monovalent substituent, n represents an integer of 0 to 5, n' represents an integer of 0 to 5, and n and n' are not simultaneously O. When n is 2 or larger, plural $R^{11}$s may be the same as or different from each other. When n' is 2 or larger, plural $R^{12}{}_S$ may be the same as or different from each other. Formula (II) may represent any of isomers caused by the presence of the double bonds.

The compound represented by Formula (II) preferably has a molar absorption coefficient ε at a wavelength of 365 nm of 500 $mol^{-1}\cdot L\cdot cm^{-1}$ or more, more preferably of 3,000 $mol^{-1}L\cdot cm^{-1}$ or more, most preferably of 20,000 $mol^{-1}\cdot L\cdot cm^{-1}$ or more. When the molar absorption coefficient ε is within the above range at each wavelength, the sensitivity enhancing effect may be high in terms of light absorption efficiency, which is preferable.

Preferred examples of the compound represented by Formula (II) include, but are not limited to, the compounds illustrated below.

In the description, some chemical formulae are simplified structural formulae in which solid lines represent hydrocarbon groups, unless elements and substituents are explicitly indicated.

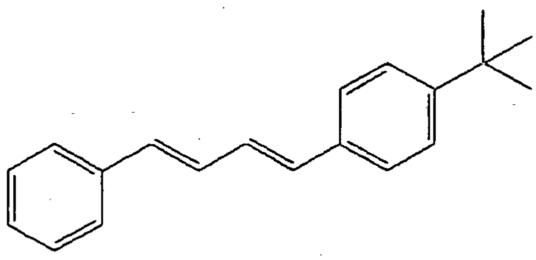

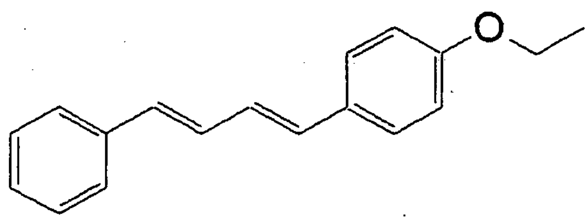

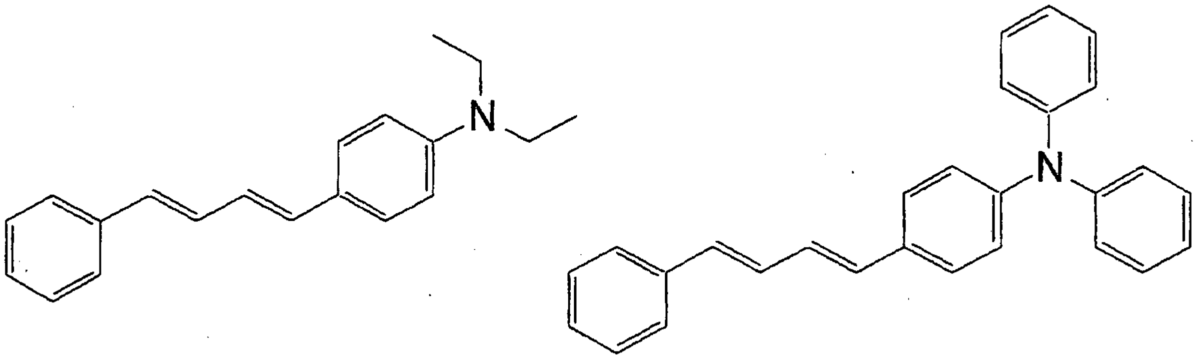

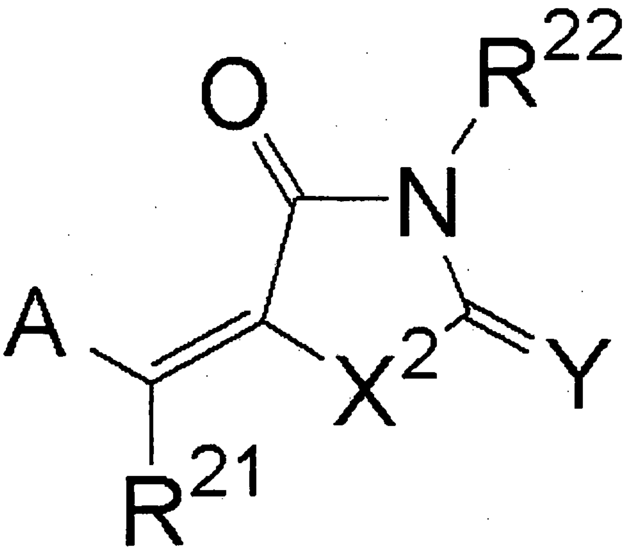

(III)

In Formula (III), A represents an aromatic or heterocyclic group which may have a substituent; $X^2$ represents an oxygen atom, a sulfur atom or -N($R^{23}$)-; Y represents an oxygen atom, a sulfur atom or -N($R^{23}$)-; $R^{21}$, $R^{22}$ and $R^{23}$ each independently represent a hydrogen atom or a monovalent nonmetallic atom group; and A, $R^{21}$, $R^{22}$, and $R^{23}$ may be bonded to one another to form one or more aliphatic or aromatic rings.

In Formula (III), $R^{21}$, $R^{22}$ and $R^{23}$ each independently represent a hydrogen atom or a monovalent nonmetallic atom group. The monovalent nonmetallic atom group represented by $R^{21}$, $R^{22}$ or $R^{23}$ is preferably a substituted or unsubstituted alkyl group, a substituted or unsubstituted aryl group, a substituted or unsubstituted alkenyl group, a substituted or unsubstituted aromatic heterocyclic residue, a substituted or unsubstituted alkoxy group, a substituted or unsubstituted alkylthio group, a hydroxyl group, or a halogen atom.

In the compound represented by Formula (III), Y is preferably an oxygen atom or -N($R^{23}$)- in terms of increasing the efficiency of decomposition of the photopolymerization initiator. Each $R^{23}$ independently represent a hydrogen atom or a monovalent nonmetallic atom group. Y is most preferably -N($R^{23}$)-.

Preferred examples of the compound represented by Formula (III) include, but are not limited to, the following compounds. In the invention, the isomers caused by the presence of the double bond(s) between the acidic nuclear and the basic nuclear are not explicitly specified, and the structure is not limited to a particular isomer. Accordingly, any isomer may be used in the invention.

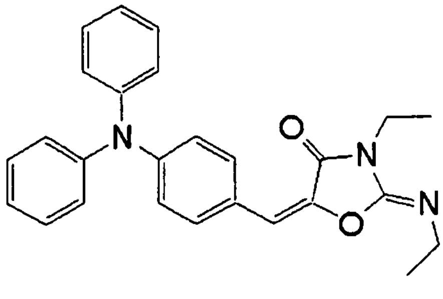

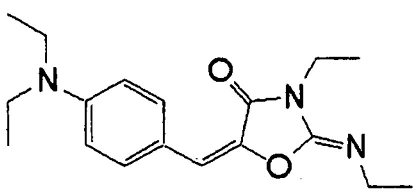

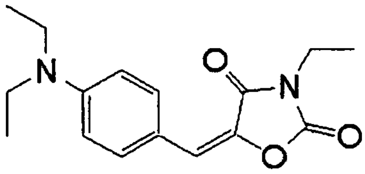
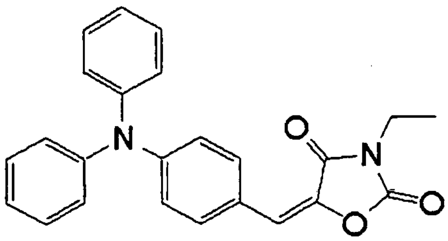
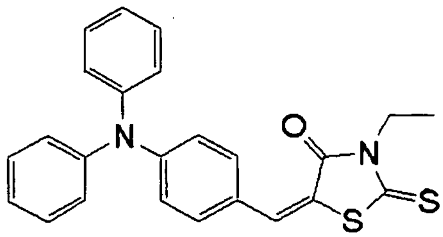
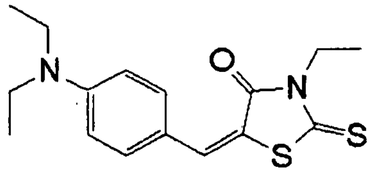
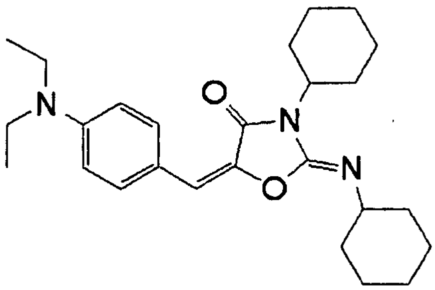
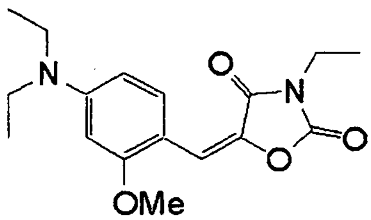
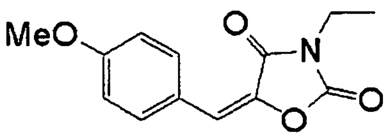
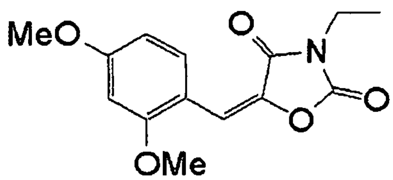
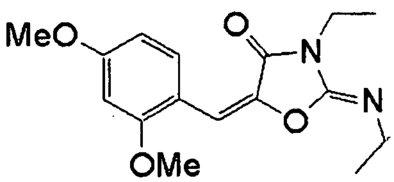
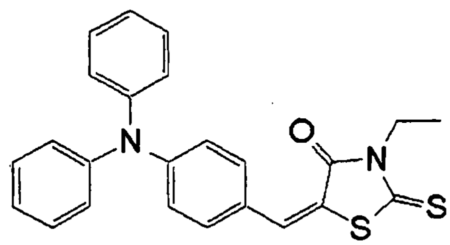

The amount of the sensitizing agent in Polymerizable Composition (1) is preferably from 0.1% by mass to 20% by mass, and more preferably 0.5% by mass to 15% by mass, in terms of solid contents, from the viewpoint of the light absorption efficiency in a deep portion and the initial decomposition efficiency.

The sensitizing agents may be used singly or may be used in combination of two or more of them.

*(1)-(F) Co-sensitizing Agent*

Polymerizable Composition (1) may further contain a co-sensitizing agent.

In the invention, the co-sensitizing agent has an effect on further improving the sensitivity of (A) the specified oxime compound or the sensitizing agent to active radiation, or preventing the inhibition of polymerization of (B) the polymerizable compound due to oxygen.

Examples of the co-sensitizing agent include amines such as the compounds described in M. R. Sander et al.,

Journal of Polymer Society, Vol. 10, p. 3173 (1972), JP-B No. 44-20189, JP-ANos. 51-82102, 52-134692, 59-138205, 60-84305, 62-18537, and 64-33104, and Research Disclosure 33825. Specific examples thereof include triethanolamine, ethyl p-dimethylaminobenzoate, p-formyldimethylaniline, and p-methylthiodimethylaniline.

Other examples of the co-sensitizing agent include thiols and sulfides such as the thiol compounds described in JP-A No. 53-702, JP-B No. 55-500806 and JP-A No. 05-142772 and the disulfide compounds described in JP-A No. 56-75643, and specific examples thereof include 2-mercaptobenzothiazole, 2-mercaptobenzoxazole, 2-mercaptobenzimidazole, 2-mercapto-4(3H)-quinazoline, and β-mercaptonaphthalene.

Other examples of the co-sensitizing agent also include amino acid compounds (such as N-phenylglycine), the organometallic compounds described in JP-B No. 48-42965 (such as tributyltin acetate), the hydrogen donators described in JP-B No. 55-34414, and the sulfur compounds (such as trithiane) described in JP-ANo. 06-308727.

The amount of the co-sensitizing agent is preferably in the range of 0.1 % by mass to 30% by mass, more preferably in the range of 1% by mass to 25% by mass, and even more preferably in the range of 1.5% by mass to 20% by mass, with respect to the mass of the total solid content of Polymerizable Composition (1), from the viewpoint of improving the curing speed according to the balance between a polymerization growing speed and chain transfer.

Polymerizable Composition (1) preferably includes a thiol compound as the co-sensitizing agent.

The thiol compound that may be included in Polymerizable Composition (1) of the invention is preferably a compound represented by the following Formula (IV).

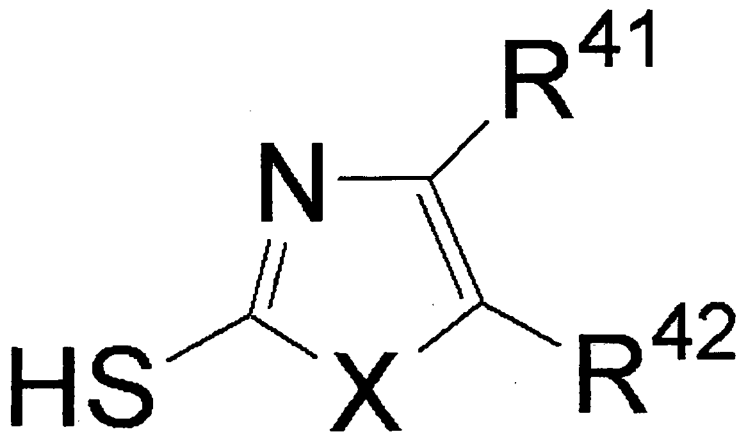

(IV)

In Formula (IV), X represents a sulfur atom, an oxygen atom or $-N(R^{43})-$; $R^{43}$ represents a hydrogen atom, an alkyl group having 1 to 5 carbon atoms or an aryl group having 6 to 13 carbon atoms; $R^{41}$ and $R^{42}$ each independently represent a hydrogen atom, an alkyl group having 1 to 5 carbon atoms, a hydroxyalkyl group having 1 to 3 carbon atoms, a phenyl group which may be substituted by an alkoxy group having 1 to 8 carbon atoms, a nitro group, an alkoxycarbonyl group having an alkyl group having 1 to 8 carbon atoms, a phenoxycarbonyl group, an acetyl group, or a carboxyl group; $R^{41}$, $R^{42}$ and the double bond therebetween together may form a benzene ring; and the double bond between $R^{41}$ and $R^{42}$ may be hydrogenated.

The thiol compound may be, for example, selected from the compounds described in JP-A No. 53-702, JP-B No. 55-500806, and JP-A No. 05-142772.

In the invention, the thiol compound suitable for Polymerization Composition (1) is preferably a compound represented by the following Formula (V).

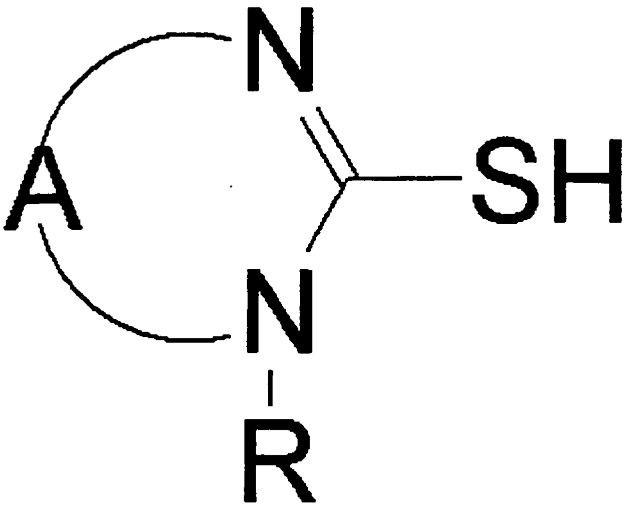

(V)

In Formula (V), R represents an alkyl group or an aryl group, and A represents an atom group that forms a heterocycle together with N=C-N.

In Formula (V), R represents an alkyl group or an aryl group.

The alkyl group may be a linear, branched or cyclic alkyl group having 1 to 20 carbon atoms, more preferably a linear alkyl group having 1 to 12 carbon atoms, a branched alkyl group having 3 to 12 carbon atoms or a cyclic alkyl group having 5 to 10 carbon atoms.

Examples of the alkyl group include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicocyl group, an isopropyl group, an isobutyl group, a sec-butyl group, a tert-butyl group, an isopentyl group, a neopentyl group, a 1-methylbutyl group, an isohexyl group, a 2-ethylhexyl group, a 2-methylhexyl group, a cyclohexyl group, a cyclopentyl group, and a 2-norbornyl group.

The aryl group may have a monocyclic structure, a structure having a condensed ring formed by condensation of 1 to 3 benzene rings, or a structure having a condensed ring formed by condensation of at least one benzene ring and at least one five-membered unsaturated ring. Examples thereof include a phenyl group, a naphthyl group, an anthryl group, a phenanthryl group, an indenyl group, an acenaphthenyl group, and a fluorenyl group. In particular, a phenyl group or a naphthyl group is more preferred.

The alkyl group or the aryl group may further have at least one substituent. Examples of the substituent that may be introduced include a linear, branched or cyclic alkyl group having 1 to 20 carbon atoms, a linear, branched or cyclic alkenyl group having 2 to 20 carbon atoms, an alkynyl group having 2 to 20 carbon atoms, an aryl group having 6 to 20 carbon atoms, an acyloxy group having 1 to 20 carbon atoms, an alkoxycarbonyloxy group having 2 to 20 carbon atoms, an aryloxycarbonyloxy group having 7 to 20 carbon atoms, a carbamoyloxy group having 1 to 20 carbon atoms, a carbonamide group having 1 to 20 carbon atoms, a sulfonamide group having 1 to 20 carbon atoms, a carbamoyl group having 1 to 20 carbon atoms, a sulfamoyl group, a substituted sulfamoyl group having 1 to 20 carbon atoms, an alkoxy group having 1 to 20 carbon atoms, an aryloxy group having 6 to 20 carbon atoms, an aryloxycarbonyl group having 7 to 20 carbon atoms, an alkoxycarbonyl group having 2 to 20 carbon atoms, an N-acylsulfamoyl group having 1 to 20 carbon atoms, an N-sulfamoylcarbamoyl group having 1 to 20 carbon atoms, an alkylsulfonyl group having 1 to 20 carbon atoms, an arylsulfonyl group having 6 to 20 carbon atoms, an alkoxycarbonylamino group having 2 to 20 carbon atoms, an aryloxycarbonylamino group having 7 to 20 carbon atoms, an amino group, a substituted amino group having 1 to 20 carbon atoms, an imino group having 1 to 20 carbon atoms, an ammonio group having 3 to 20 carbon atoms, a carboxyl group, a sulfo group, an oxy group, a mercapto group, an alkylsulfinyl group having 1 to 20 carbon atoms, an arylsulfmyl group having 6 to 20 carbon atoms, an alkylthio group having 1 to 20 carbon atoms, an arylthio group having 6 to 20 carbon atoms, a ureide group having 1 to 20 carbon atoms, a heterocyclic group having 2 to 20 carbon atoms, an acyl group having 1 to 20 carbon atoms, a sulfamoylamino group, a substituted sulfamoylamino group having 1 to 2 carbon atoms, a silyl group having 2 to 20 carbon atoms, an isocyanate group, an isocyanide group, a halogen atom (such as a fluorine, chlorine or bromine atom), a cyano group, a nitro group, and an onium group.

In Formula (V), A represents an atom group that forms a heterocycle together with N=C-N.

The at least one atom that forms the atom group may include atoms selected from carbon, nitrogen, hydrogen, sulfur, and selenium atoms.

The heterocycle formed by A and N=C-N may further have at least one substituent. Examples of introducible substituents may include the same substituents as those introducible into the above-mentioned alkyl group or aryl group.

In the invention, the thiol compound suitably used for Polymerizable Composition (1) is more preferably a compound represented by the following Formula (VI) or (VII).

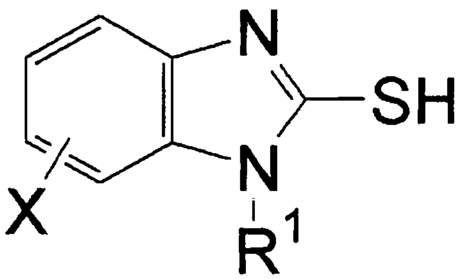

(VI)

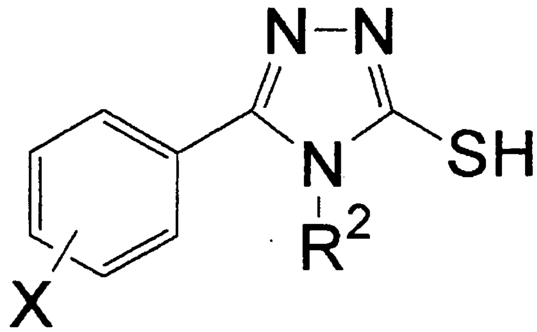

(VII)

In Formula (VI), $R^1$ represents an aryl group, and X represents a hydrogen atom, a halogen atom, an alkoxy group, an alkyl group, or an aryl group.

In Formula (VII), $R^2$ represents an alkyl group or an aryl group, and X represents a hydrogen atom, a halogen atom, an alkoxy group, an alkyl group, or an aryl group.

In Formulae (VI) and (VII), the halogen atom is preferably a fluorine, chlorine, bromine, or iodine atom.

In Formulae (VI) and (VII), examples of the alkoxy group include a methoxy group, an ethoxy group, a propyloxy group, an isopropyloxy group, a butyloxy group, a pentyloxy group, a hexyloxy group, a dodecyloxy group, a benzyloxy group, an allyloxy group, a phenethyloxy group, a carboxyethyloxy group, a methoxycarbonylethyloxy group, an ethoxycarbonylethyloxy group, a methoxyethoxy group, a phenoxyethoxy group, a methoxyethoxyethoxy group, an ethoxyethoxyethoxy group, a morpholinoethoxy group, a morpholinopropyloxy group, an allyloxyethoxyethoxy group, a phenoxy group, a tolyloxy group, a xylyloxy group, a mesityloxy group, a cumetyloxy group, a methoxyphenyloxy group, an ethoxyphenyloxy group, a chlorophenyloxy group, a bromophenyloxy group, an acetyloxy group, a benzoyloxy group, and a naphthyloxy group.

In Formulae (VI) and (VII), the alkyl group has the same definition as the alkyl group represented by R in Formula (V), and their preferred scopes are also the same.

In Formulae (VI) and (VII), the aryl group has the same definition as the aryl group represented by R in Formula (V), and their preferred scopes are also the same.

In Formulae (VI) and (VII), each group may further have at least one substituent. Examples of the substituent include those mentioned above as substituents introducible to the alkyl or aryl group represented by R in Formula (V).

In Formulae (VI) and (VII), X is preferably a hydrogen atom from the viewpoint of solubility in propylene glycol monomethyl ether acetate (PGMEA) that is a solvent suitably used for preparation of the polymerizable composition.

In Formula (VI), $R^1$ is most preferably a phenyl group from the viewpoints of sensitivity and solubility in PGMEA.

In Formula (VII), $R^2$ is more preferably a methyl group, an ethyl group, a phenyl group, or a benzyl group from the viewpoints of sensitivity and solubility in PGMEA.

Among the compounds represented by Formulae (VI) and (VII), the compound represented by Formula (VII) is most preferred in terms of solubility in PGMEA.

Preferred examples of the thiol compound that may be used in an embodiment of the invention include, but are not limited to, the compounds shown below.

Herein, some chemical formulae are simplified structural formulae in which solid lines represent hydrocarbon groups, unless elements and substituents are explicitly indicated. In the examples below, Me represents a methyl group.

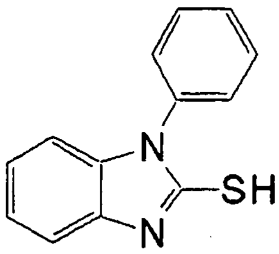
Solubility: 20 g/L or more

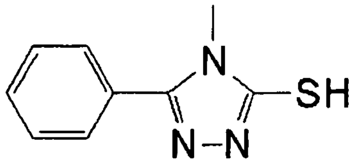
Solubility: 20 g/L or more

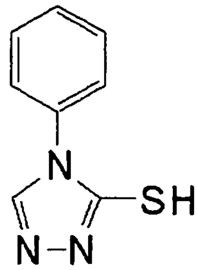
Solubility: 20 g/L or more

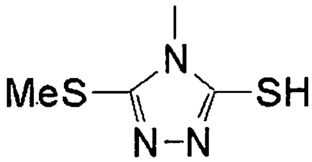
Solubility: 20 g/L or more

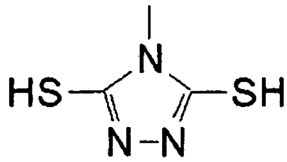
Solubility: 20 g/L or more

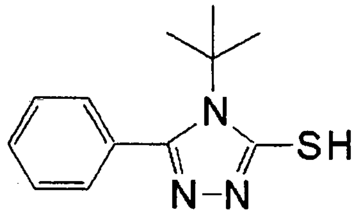
Solubility: 20 g/L or more

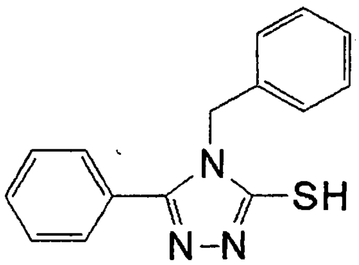
Solubility: 20 g/L or more

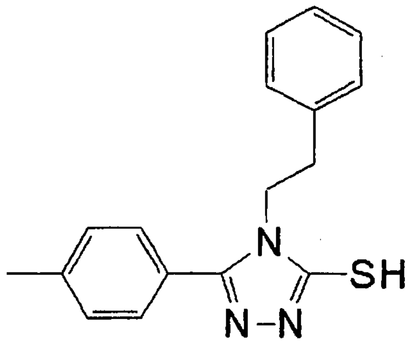
Solubility: 20 g/L or more

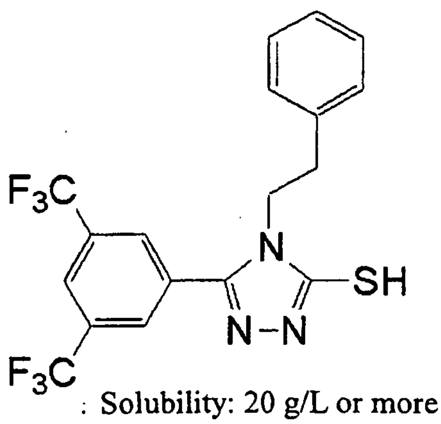
: Solubility: 20 g/L or more

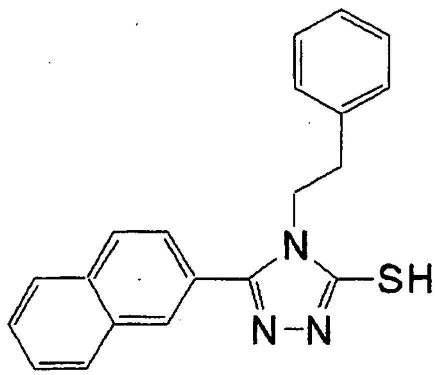
Solubility: 20 g/L or more

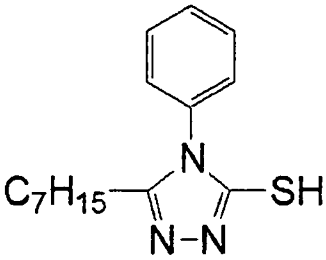

Solubility: 20 g/L or more

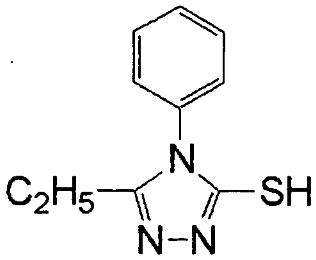

Solubility: 20 g/L or more

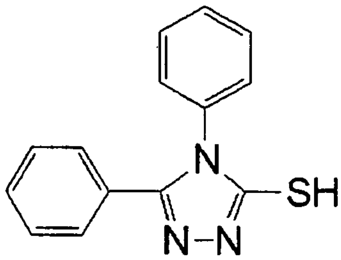

Solubility: 20 g/L or more

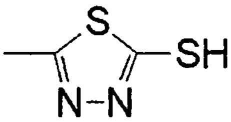

Solubility: 20 g/L or more

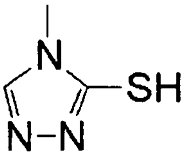

Solubility: 20 g/L or more

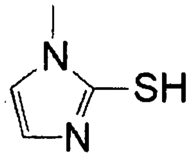

Solubility: 20 g/L or more

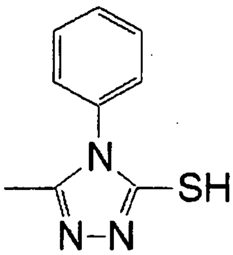

Solubility: 20 g/L or more

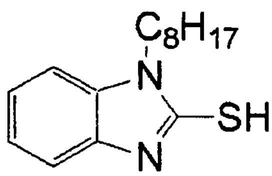

Solubility: 20 g/L or more

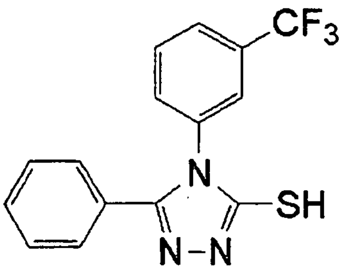

Solubility: 20 g/L or less

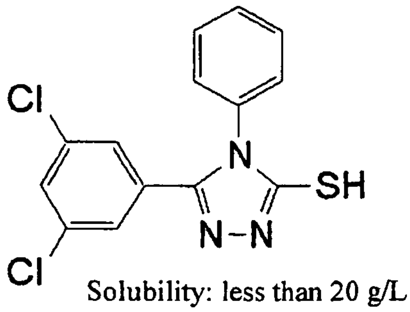

Solubility: less than 20 g/L

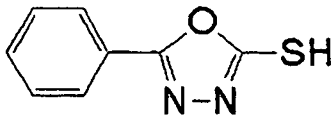

Solubility: less than 20 g/L

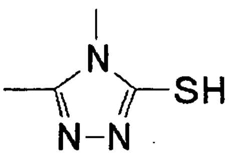

Solubility: less than 20 g/L

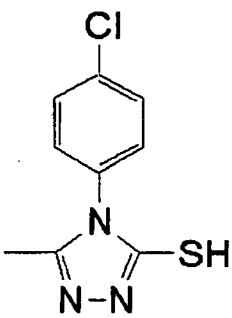

Solubility: less than 20 g/L

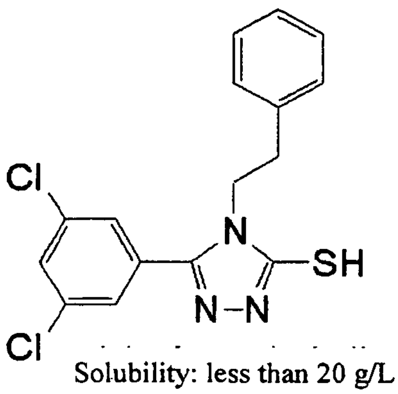

Solubility: less than 20 g/L

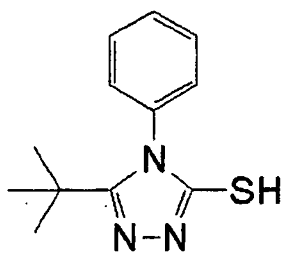

Solubility: less than 20 g/L

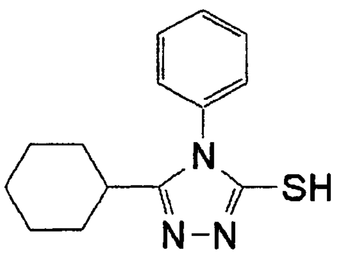

Solubility: less than 20 g/L

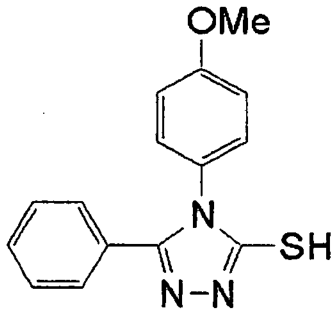

Solubility: less than 20 g/L

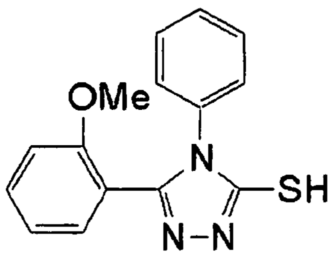

Solubility: less than 20 g/L

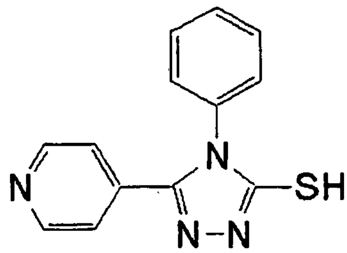

Solubility: less than 20 g/L

The solubility of the thiol compounds in PGMEA solvent is preferably 20 g/L or more, more preferably from 20 g/L to 50 g/L, even more preferably from 20 g/L to 40 g/L, from the viewpoint of coating uniformity.

*Solubility Measurement Method*

Herein, the solubility of the thiol compound is defined as follows.

The specific thiol compound is added to 5 mL of propylene glycol monomethyl ether acetate (PGMEA) solvent and stirred at 25°C for one hour. In this process, the largest amount of the specific thiol compound that can completely dissolve in the solvent is assumed as the solubility.

The thiol compounds may be synthesized by the method described in J. Appl. Chem., 34, 2203-2207 (1961).

The thiol compounds may be used singly or may be used in combination of two or more of them.

When two or more thiol compounds are used in combination, two or more compounds represented by one of the above formulae may be used, or two or more compounds represented by different formulae may be used in combination. For example, there is an aspect in which a compound selected from the compounds represented by Formula (VI) and a compound selected from the compounds represented by Formula (VII) are used in combination.

When Polymerizable Composition (1) includes a thiol compound, the amount of the thiol compound is preferably in the range of 0.5% by mass to 30% by mass, more preferably in the range of 1% by mass to 25% by mass, and even more preferably in the range of 3% by mass to 20% by mass, with respect to the mass of the total solid content of Polymerizable Composition (1), from the viewpoint of improving the curing speed according to the balance between a polymerization growing speed and chain transfer.

*(1)-(G) Binder Polymer*

If necessary, a binder polymer may also be included in Polymerizable Composition (1) of the invention for the purpose of improving film characteristics and the like. A linear organic polymer is preferably used as the binder. Any known "linear organic polymer" may be used. In order to enable development with water or a weakly alkaline aqueous solution, a linear organic polymer soluble or swellable in water or a weakly alkaline aqueous solution is preferably selected. The linear organic polymer may be selected and used as depending on applications not only as a film-forming agent but also in consideration of the developer such as water, a weakly alkaline aqueous solution or an organic solvent. For example, water development can be performed when a water-soluble organic polymer is used. Examples of such a linear organic polymer include radical polymerization products having a carboxylic acid group in a side chain thereof, such as those described in JP-A No. 59-44615, JP-B Nos. 54-34327, 58-12577 and 54-25957, and JP-A Nos. 54-92723, 59-53836 and 59-71048, specifically, resins produced by homopolymerization or copolymerization of carboxyl group-containing monomers, resins produced by homopolymerization or copolymerization of acid anhydride-containing monomers and subsequent hydrolysis, half-esterification or half-amidation of the acid anhydride units, and epoxy acrylates produced by modification of epoxy reins with unsaturated monocarboxylic acids and/or acid anhydrides. Examples of the carboxyl group-containing monomers include acrylic acid, methacrylic acid, itaconic acid, crotonic acid, maleic acid, fumaric acid, and 4-carboxylstyrene. Examples of the acid anhydride-containing monomers include maleic anhydride.

Examples also include acidic cellulose derivatives having carboxylic acid groups in side chains thereof. Besides the above, a product of addition of a cyclic acid anhydride to a hydroxyl group-containing polymer is also useful.

When the alkali-soluble resin to be used is a copolymer, monomers other than the above monomers may also be used as the compounds to be copolymerized. Examples of other monomers include the following compounds of (1) to (12):

(1) aliphatic hydroxyl group-containing acrylates and methacrylates such as 2-hydroxyethyl acrylate, 2-hydroxypropyl acrylate, 3-hydroxypropyl acrylate, 4-hydroxybutyl acrylate, 2-hydroxyethyl methacrylate, 2-hydroxypropyl methacrylate, 3-hydroxypropyl methacrylate, and 4-hydroxybutyl methacrylate;
(2) alkyl acrylates such as methyl acrylate, ethyl acrylate, propyl acrylate, butyl acrylate, isobutyl acrylate, amyl acrylate, hexyl acrylate, 2-ethylhexyl acrylate, octyl acrylate, benzyl acrylate, 2-chloroethyl acrylate, glycidyl acrylate, 3,4-epoxycyclohexylmethyl acrylate, vinyl acrylate, 2-phenylviyl acrylate, 1-propenyl acrylate, allyl acrylate, 2-allyloxyethyl acrylate, and propargyl acrylate;
(3) alkyl methacrylates such as methyl methacrylate, ethyl methacrylate, propyl methacrylate, butyl methacrylate, isobutyl methacrylate, amyl methacrylate, hexyl methacrylate, 2-ethylhexyl methacrylate, cyclohexyl methacrylate, benzyl methacrylate, 2-chloroethyl methacrylate, glycidyl methacrylate, 3,4-epoxycyclohexylmethyl methacrylate, vinyl methacrylate, 2-phenylviyl methacrylate, 1-propenyl methacrylate, allyl methacrylate, 2-allyloxyethyl methacrylate, and propargyl methacrylate;
(4) acrylamides and methacrylamides such as acrylamide, methacrylamide, N-methylolacrylamide, N-ethylacrylamide, N-hexylmethacrylamide, N-cyclohexylacrylamide, N-hydroxyethylacrylamide, N-phenylacrylamide, N-nitrophenylacrylamide, N-ethyl-N-phenylacrylamide, vinyl acrylamide, vinyl methacrylamide, N,N-diallylacrylamide, N,N-diallylmethacrylamide, allylacrylamide, and allylmethacrylamide;
(5) vinyl ethers such as ethyl vinyl ether, 2-chloroethyl vinyl ether, hydroxyethyl vinyl ether, propyl vinyl ether, butyl vinyl ether, octyl vinyl ether, and phenyl vinyl ether;
(6) vinyl esters such as vinyl acetate, vinyl chloroacetate, vinyl butyrate, and vinyl benzoate;
(7) styrenes such as styrene, $\alpha$-methylstyrene, methylstyrene, chloromethylstyrene, and p-acetoxystyrene;
(8) vinyl ketones such as methyl vinyl ketone, ethyl vinyl ketone, propyl vinyl ketone, and phenyl vinyl ketone;
(9) olefins such as ethylene, propylene, isobutylene, butadiene, and isoprene;
(10) N-vinylpyrrolidone, acrylonitrile, methacrylonitrile, and the like;
(11) unsaturated imides such as maleimide, N-acryloylacrylamide, N-acetylmethacrylamide, N-propionylmethacrylamide, and N-(p-chlorobenzoyl)methacrylamide; and
(12) methacrylic acid monomers having a hetero atom at the $\alpha$-position thereof, such as the compounds described in JP-A Nos. 2002-309057 and 2002-311569.

Particularly preferred are (meth)acrylic resins having an allyl or vinyl ester group and a carboxyl group in a side chain thereof, the alkali-soluble resins having a double bond in a side chain thereof described in JP-A Nos. 2000-187322 and 2002-62698, and the alkali-soluble resins having an amide group in a side chain thereof described in JP-A No. 2001-242612, in view of excellent balance between film strength, sensitivity and developability.

The acid group-containing urethane binder polymers described in JP-B Nos. 07-12004, 07-120041, 07-120042, and 08-12424, JP-A Nos. 63-287944, 63-287947 and 01-271741 and the urethane binder polymers having acid groups and double bonds in side chains thereof described in JP-A No. 2002-107918 are advantageous in terms of printing durability or low exposure properties, because they have very high strength.

The acid group-containing, acetal-modified, polyvinyl alcohol binder polymers such as those described in European Patent Nos. 993966 and 1204000 and JP-A No. 2001-318463 are also preferred, because they have an excellent balance between film strength and developability.

Examples of useful water-soluble linear organic polymers also include polyvinyl pyrrolidone and polyethylene oxide. Alcohol-soluble nylon or polyether of 2,2-bis-(4-hydroxyphenyl)-propane and epichlorohydrin is also useful for increasing the strength of the cured film.

Binder polymers that may be used in Polymerizable Composition (1) preferably have a weight average molecular weight of 5,000 or more, more preferably of 10,000 to 300,000, and preferably have a number average molecular weight of 1,000 or more, more preferably of 2,000 to 250,000. The polydispersity (weight average molecular weight/number average molecular weight) thereof is preferably 1 or more, more preferably from 1.1 to 10.

These binder polymers may be any of random, block or graft polymers.

Binder polymers that may be used in the invention may be synthesized by known conventional methods. Examples of solvents that may be used for the synthesis include tetrahydrofuran, ethylene dichloride, cyclohexanone, methyl ethyl ketone, acetone, methanol, ethanol, ethylene glycol monomethyl ether, ethylene glycol monoethyl ether, 2-methoxyethyl acetate, diethylene glycol dimethyl ether, 1-methoxy-2-propanol, 1-methoxy-2-propyl acetate, N,N-dimethylformamide, N,N-dimethylacetamide, toluene, ethyl acetate, methyl lactate, ethyl lactate, dimethylsulfoxide, and water. Only one solvent may be used, or a mixture of two or more solvents may be used.

In the process of synthesizing binder polymers that may be used for Polymerizable Composition (1), a known compound such as an azo initiator or a peroxide initiator may be used as a radical-polymerization initiator.

The amount of the binder polymer is preferably from 1 to 50% by mass, more preferably from 1 to 30% by mass, and even more preferably from 1 to 20% by mass, with respect to the total solid content of Polymerizable Composition (1).

*(1)-(H) Polymerization Inhibitor*

In Polymerizable Composition (1), it is preferable that a small amount of heat polymerization inhibitor be added in order to inhibit undesired heat polymerization of (B) the polymerizable compound during the production or preservation of the polymerizable composition.

Examples of the heat polymerization inhibitors which may be used in the invention include hydroquinone, p-methoxyphenol, di-tert-butyl-p-cresol, pyrogallol, tert-butylcatechol, benzoquinone, 4,4'-thiobis(3-methyl-6-tert-butylphenol), 2,2'-methylenebis(4-methyl-6-tert-butylphenol), and N-nitrosophenyl-hydroxylamine cerium (I) salts.

The amount of the heat polymerization inhibitor to be added is preferably from about 0.01% by mass to about 5% by mass with respect to the total solid content of Polymerizable Composition (1).

If necessary, behenic acid or a higher fatty acid derivative such as behenic acid amide may be added to the composition and may be localized in the surface of a coating film during a drying process after coating, in order to prevent oxygen-induced inhibition of polymerization. The amount of the higher fatty acid derivative to be added is preferably from about 0.5% by mass to about 10% by mass with respect to the total amount of the composition.

*(1)-(I) Adhesion Improving Agent*

Polymerizable Composition (1) may further include an adhesion improving agent for increasing the adhesion to a hard surface of a support or the like. Examples of the adhesion improving agent include silane coupling agents and titanium coupling agents.

Examples of the silane coupling agent include γ-(2-aminoethyl)aminopropyltrimethoxysilane, γ-(2-aminoethyl)aminopropyldimethoxysilane, β-(3,4-epoxycyclohexyl)ethyltrimethoxysilane, γ-aminopropyltrimethoxysilane,γ -aminopropyltriethoxysilane, γ-methacryloxypropyltrimethoxysilane, γ-methacryloxypropyltriethoxysilane, γ-acryloxypropyltrimethoxysilane, γ-acryloxypropyltriethoxysilane, γ-isocyanatopropyltrimethoxysilane, γ-isocyanatopropyltriethoxysilane, N-β-(N-vinylbenzylaminoethyl)-γ-aminopropyltrimethoxysilane hydrochloride, γ-glycidoxypropyltrimethoxysilane, γ-glycidoxypropyltriethoxysilane, aminosilane, γ-mercaptopropyltrimethoxysilane, γ-mercaptopropyltriethoxysilane, methyltrimethoxysilane, methyltriethoxysilane, vinyltriacetoxysilane, γ-chloropropyltrimethoxysilane, hexamethyldisilazane, γ-anilinopropyltrimethoxysilane, vinyltrimethoxysilane, vinyltriethoxysilane, vinyltris(β-methoxyethoxy)silane, octadecyldimethyl[3-(trimethoxysilyl)propyl]ammonium chloride, γ-chloropropylmethyldimethoxysilane, γ-mercaptopropylmethyldimethoxysilane, methyltrichlorosilane, dimethyldichlorosilane, trimethylchlorosilane, 2-(3,4-epoxycyclohexyl)ethyltrimethoxysilane, bisallyltrimethoxysilane, tetraethoxysilane, bis(trimethoxysilyl)hexane, phenyltrimethoxysilane, N-(3-acryloxy-2-hydroxypropyl)-3-aminopropyltriethoxysilane, N-(3-methacryloxy-2-hydroxypropyl)-3-aminopropyltriethoxysilane, (methacryloxymethyl)methyldiethoxysilane, and (acryloxymethyl)methyldimethoxysilane.

In particular, γ-methacryloxypropyltrimethoxysilane, γ-methacryloxypropyltriethoxysilane, γ-acryloxypropyltrimethoxysilane, γ-acryloxypropyltriethoxysilane, γ-mercaptopropyltrimethoxysilane, γ-aminopropyltriethoxysilane, and phenyltrimethoxysilane are preferred, and γ-methacryloxypropyltrimethoxysilane is most preferred.

The amount of the adhesion improving agent is preferably from 0.5 to 30% by mass, more preferably from 0.7 to 20% by mass, based on the mass of the total solid contents of Polymerizable Composition (1).

*(1)-(J) Diluent*

Any of various organic solvents may be used as a diluent for Polymerizable Composition (1).

Examples of the organic solvent that may be used include acetone, methyl ethyl ketone, cyclohexane, ethyl acetate, ethylene dichloride, tetrahydrofuran, toluene, ethylene glycol monomethyl ether, ethylene glycol monoethyl ether, ethylene glycol dimethyl ether, propylene glycol monomethyl ether, propylene glycol monoethyl ether, acetyl acetone, cyclohexanone, diacetone alcohol, ethylene glycol monomethyl ether acetate, ethylene glycol ethyl ether acetate, ethylene glycol monoisopropyl ether, ethylene glycol monobutyl ether acetate, 3-methoxypropanol, methoxymethoxyethanol, diethylene glycol monomethyl ether, diethylene glycol monoethyl ether, diethylene glycol dimethyl ether, diethylene glycol diethyl ether, propylene glycol monomethyl ether acetate, propylene glycol monoethyl ether acetate, 3-methoxypropyl acetate, N,N-dimethylformamide, dimethylsulfoxide, γ-butyrolactone, methyl lactate, and ethyl lactate.
Only a single solvent may be used, or a mixture of two or more solvents may be used. The solid content with respect to the organic solvent is preferably from 2 to 60% by mass.

*(1)-(K) Other Additives*

In addition, known additives for modifying the physical properties of cured films, such as an inorganic filler, a plasticizer or a lipophilizing agent, may also be added to Polymerizable Composition (1).
Examples of the plasticizer include dioctyl phthalate, didodecyl phthalate, triethylene glycol dicaprylate, dimethyl glycol phthalate, tricresyl phosphate, dioctyl adipate, dibutyl sebacate, and triacetylglycerol. When a binder is used, the plasticizer may be added in an amount of 10% by mass or less, based on the total mass of the polymerizable compound and the binder polymer.
Since Polymerizable Composition (1) includes (A) the specified oxime compound, it can be cured at a high sensitivity and has excellent storage stability. In addition, coloration caused by heat aging can be prevented. When Polymerizable Composition (1) is applied to a hard material surface and cured, the cured product exhibits excellent adhesion to the surface.
Such Polymerizable Composition (1) preferably includes (A) a specified oxime compound, (B) a polymerizable compound, and (C) a colorant and is used as a polymerizable composition for color filters (i.e., a polymerizable composition for a color filter of the invention).

*Polymerizable Composition (2): Polymerizable Composition for Photosensitive Planographic Printing Plate Precursors*

When Polymerizable Composition (2) according to another exemplary embodiment of the invention is subjected to pattern exposure, an exposed area thereof is cured due to form a strong film. Therefore, Polymerizable Composition (2) is useful for formation of a photosensitive layer of a planographic printing plate precursor. Preferable exemplary embodiments of the application of the polymerizable composition of the invention to a photosensitive layer of a planographic printing plate precursor are described below.

*(2)-(A) Specified Oxime Compound*

Polymerizable Composition (2) includes (A) the specified oxime compound described above. The specified oxime compound included in Polymerizable Composition (2) may function as a polymerization initiator in the composition.
The amount of the specified oxime compound in Polymerizable Composition (2) is preferably from 0.5% by mass to 40% by mass, more preferably from 1% by mass to 35% by mass, and even more preferably from 1.5% by mass to 30% by mass, with respect to the total solid content of the composition.
The specified oxime compounds may be used singly or may be used in combination of two or more of them.
Also in Polymerizable Composition (2), any conventional polymerization initiator other than the specified oxime compound may be used together with the specified oxime compound, provided it does not impair the effect of the invention.
Examples of other polymerization initiator include (a) aromatic ketones, (b) aromatic onium salt compounds, (c) organic peroxides, (d) thio compounds, (e) hexaarylbiimidazole compounds, (f) ketoxime ester compounds, (g) borate compounds, (h) azinium compounds, (i) metallocene compounds, (j) active ester compounds and (k) compounds having a carbon-halogen bond. More specific examples thereof include the polymerization initiators disclosed in paragraphs [0081] to [0139] of JP-A No. 2006-78749.

*(2)-(B) Polymerizable Compound*

Examples of (B) the polymerizable compound included in Polymerizable Composition (2) include the addition-polymerizable compounds described in the description of Polymerizable Composition (1).
Details of how to use the addition-polymerizable compounds, such as what structure should be used, whether they should be used alone or in combination, or what amount should be added, may be freely determined depending on the final performance design of the curable composition. For example, they may be selected from the following viewpoints.
From the viewpoint of photoresponsive speed, a structure having a higher content of the unsaturated groups per molecule is preferable, and difunctional or higher functional structures are preferred in many cases. In order to increase the strength of the image portion or the cured film, tri- or higher-functional structures are preferred. A method

of using a combination of compounds having different numbers of functional groups and/or different types of polymerizable groups (for example, compounds selected from an acrylic ester, a methacrylic ester, a styrene compound, a vinyl ether compound) is also effective for controlling both of sensitivity and strength. High-molecular-weight compounds or highly hydrophobic compounds can have high sensitive speed or form high-strength films but are not preferred in some cases in view of development speed or precipitation in a liquid developer.

How to select and use the addition-polymerizable compound is also an important factor for the compatibility with and dispersibility to other components in the photosensitive layer (for example, a binder polymer, an initiator, and a colorant). For example, the compatibility may be improved by using a low-purity compound or by using a combination of two or more compounds.

Moreover, a particular structure may also be selected for the purpose of improving adhesion to a support, an overcoat layer, or the like. A larger amount of the addition-polymerizable compound in the photosensitive layer is advantageous in terms of sensitivity. However, when the amount is excessively high, undesired phase separation, problems in a production process due to tackiness of the photosensitive layer (for example, production failures resulting from transfer or adhesion of the photosensitive material components), precipitation from a liquid developer, and the like may occur.

From such points of view, the amount of the addition-polymerizable compound is preferably from 5% by mass to 80% by mass, and more preferably from 25% by mass to 75% by mass, with respect to the total solid content of Polymerizable Composition (2).

Addition-polymerizable compounds may be used singly or may be used in combination of two or more of them. Besides, details of use of an addition-polymerizable compound may be determined in consideration of the degree of polymerization inhibition by oxygen, resolution, fogging, change in refractive index, and surface adhesiveness, and the like. An appropriate structure, an appropriate composition, and an adequate amount to be added of the polymerizable compound may be determined in consideration of the above factors. In some cases, a layer structure and/or a coating method such as undercoating and overcoating may be adopted.

*(2)-(C) Binder Polymer*

Photopolymerizable Composition (2) preferably includes a binder polymer. The binder polymer may be included in terms of improving film characteristics. Any binder polymer having the function of improving film characteristics may be used.

A linear organic high-molecular weight polymer is preferably included as the binder polymer. Such a linear organic high-molecular-weight polymer to be used is not particularly limited, and may be any linear organic polymer. In order to enable development with water or a weakly alkaline aqueous solution, a linear organic high-molecular-weight polymer soluble or swellable in water or a weakly alkaline aqueous solution is preferably selected.

The linear organic high-molecular-weight polymer may be selected and used not only as a film-forming agent for the polymerizable composition but also in consideration of the specifications of the developer such as water, a weakly alkaline aqueous solution or an organic solvent. For example, water development can be performed when a water-soluble organic high-molecular-weight polymer is used. Examples of such a linear organic high-molecular-weight polymer include addition polymerization products having a carboxylic acid group in a side chain thereof, such as those described in JP-A No. 59-44615, JP-B Nos. 54-34327, 58-12577 and 54-25957, and JP-A Nos. 54-92723, 59-53836 and 59-71048, specifically, methacrylic acid copolymers, acrylic acid copolymers, itaconic acid copolymers, crotonic acid copolymers, maleic acid copolymers, and partially-esterified maleic acid copolymers. Examples also include acidic cellulose derivatives having a carboxylic acid group in a side chain thereof. Besides the above, a product of an addition of a cyclic acid anhydride to a hydroxyl group-containing addition polymer is also useful.

Among these, copolymers of [benzyl (meth)acrylate/(meth)acrylic acid/optionally other addition-polymerizable vinyl monomer] and copolymers of [allyl (meth)acrylate/(meth)acrylic acid/optionally other addition-polymerizable vinyl monomer] are particularly suitable because they provide excellent balance between the film strength, the sensitivity and the developability.

An amide bond-containing binder polymer or a urethane bond-containing binder polymer may also be included. The amide bond-containing binder polymer or urethane bond-containing binder polymer preferably is an amide bond-containing linear organic high-molecular-weight polymer or a urethane bond-containing linear organic high-molecular-weight polymer. Any amide bond-containing linear organic high-molecular-weight polymer or any urethane bond-containing linear organic high-molecular-weight polymer" may be used. It is preferable to select an amide bond- or urethane bond-containing linear high-molecular-weight organic polymer soluble or swellable in water or a weakly alkaline aqueous solution, which enables development with water or a weakly alkaline aqueous solution.

The amide bond- or urethane bond-containing linear organic high-molecular-weight polymer may be selected and used not only as a film-forming agent for the polymerizable composition but also in consideration of the specifications of the developer such as water, a weakly alkaline aqueous solution or an organic solvent. For example, water development can be performed when a water-soluble high-molecular-weight organic polymer is used. Examples of the amide bond-

or urethane bond-containing linear organic high-molecular-weight polymer include the amide group-containing binders described in JP-ANo. 11-171907 because such a binder can provide both excellent developability and high film strength.

The acid group-containing urethane-based binder polymers described in JP-B Nos. 7-120040, 7-120041, 7-120042, 8-12424, JP-ANos. 63-287944, 63-287947 and 1-271741 are advantageous in view of printing durability and low exposure property because they provide highly excellent strength. The amide group-containing binders described in JP-A No. 11-171907 are suitable because they provide both excellent developability and high film strength.

In addition, polyvinylpyrrolidone, polyethylene oxide, and the like are useful as the water-soluble linear organic polymer. Furthermore, for the purpose of increasing the strength of the cured film, alcohol-soluble nylons, polyethers between 2,2-bis-(4-hydroxyphenyl)-propane and epichlorohydrin, and the like are also useful.

An arbitrary amount of the binder polymer may be mixed in Polymerizable Composition (2). The amount of the binder polymer is preferably in the range of from 30% by mass to 85% by mass with respect to the total solid content in the photosensitive layer, from the viewpoint of image strength, etc. It is preferable that the mass ratio of the addition-polymerizable compound to the binder polymer (addition-polymerizable compound / binder polymer) be in the range of from 1/9 to 7/3.

In a preferred exemplary embodiment, the binder polymer to be used is substantially water-insoluble but alkali-soluble. This can eliminate the use of an environmentally-unfriendly organic solvent for a developer or limit the used amount of the solvent to a very low level. In such a method of use, the acid value (the acid content per g of polymer expressed in chemical equivalent numbers) and the molecular weight of the binder polymer may be appropriately selected in consideration of image strength and developability. The acid value is preferably from 0.4 to 3.0 meq/g, more preferably from 0.6 to 2.0 meq/g, and the molecular weight is preferably from 3,000 to 500,000, more preferably from 10,000 to 300,000.

*(2)-(D) Sensitizing Agent*

Polymerizable Composition (2) preferably includes a sensitizing agent together with (A) the specified oxime compound or the like as a polymerization initiator. Examples of the sensitizing agent that may be used in the invention include spectral sensitizing dyes or pigments, and pigments or dyes capable of interacting with polymerization initiators upon absorption of light from a light source.

Examples of preferred spectral sensitizing dyes or pigments include polycyclic aromatic compounds (such as pyrene, perylene and triphenylene), xanthenes (such as fluorescein, eosin, erythrosine, rhodamine B, and rose bengal), cyanines (such as thiacarbocyanine and oxacarbocyanine), merocyanines (such as merocyanine and carbomerocyanine), thiazines (such as thionine, methylene blue and toluidine blue), acridines (such as acridine orange, chloroflavin, and acriflavin), phthalocyanines (such as phthalocyanine and metallophthalocyanine), porphyrins (such as tetraphenylporphyrin and center metal-substituted porphyrin), chlorophylls (such as chlorophyll, chlorophyllin and center metal-substituted chlorophyll), metal complexes (such as the compound shown below), anthraquinones (such as anthraquinone), and squaryliums (such as squarylium).

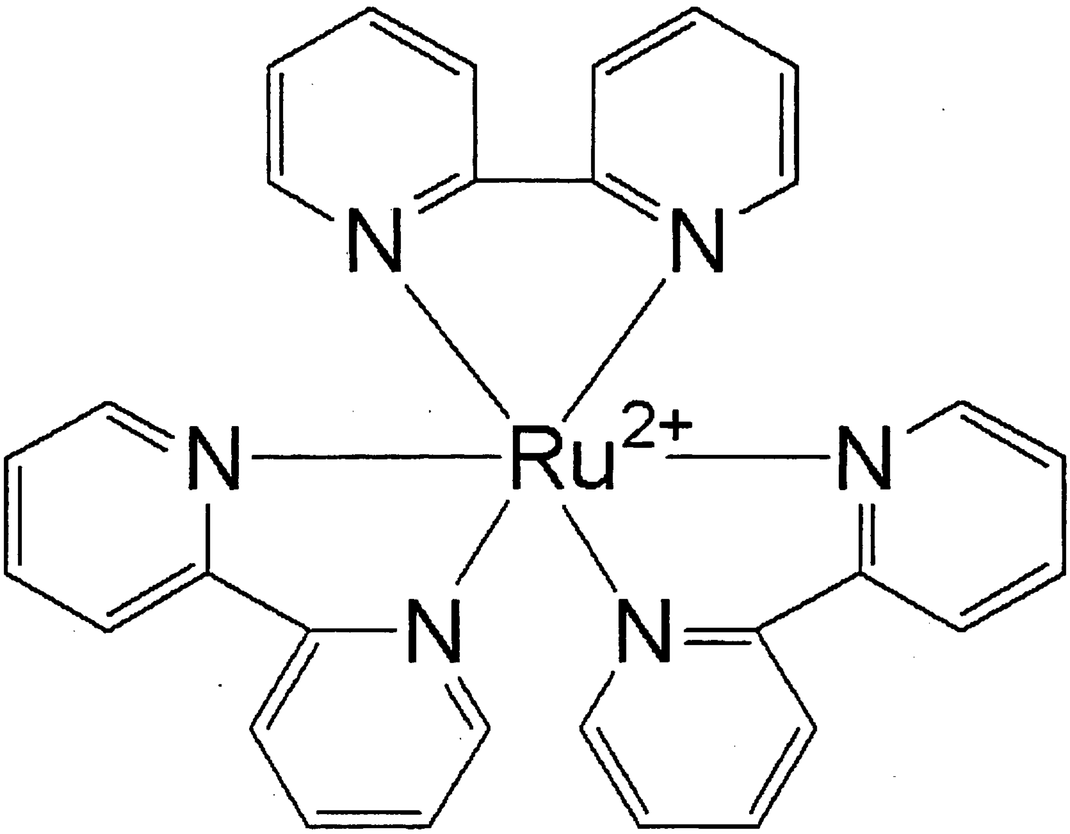

Examples of more preferred spectral sensitizing dyes or pigments include the compounds described in Paragraphs [0144] to [0202] of JP-A No. 2006-78749.

Examples of the sensitizing agent that may be used for Polymerizable Composition (2) also include those described above for Polymerizable Composition (1).

Only one sensitizing agent may be used, or two or more sensitizing agents may be used in combination. In Polymerizable Composition (2), the molar ratio of all the polymerization initiator(s) to the sensitizing dye (all the polymerization initiator(s) / sensitizing dye) may be from 100/0 to 1/99, more preferably from 90/10 to 10/90, most preferably from 80/20 to 20/80.

*(2)-(E) Co-sensitizing Agent*

Polymerizable Composition (2) may further include, as a co-sensitizing agent, a conventionally-known compound having a function of additionally improving the sensitivity of the composition or preventing the polymerization inhibition of the composition caused by oxygen.

Examples of the co-sensitizing agent also include those described above in the description of Polymerizable Composition (1). In addition, the phosphorus compounds (diethyl phosphite, etc.) disclosed in JP-A No. 6-250387 are also available.

When using a co-sensitizing agent, the amount thereof is preferably from 0.01 parts by mass to 50 parts by mass with respect to the total mass of the polymerization initiator included in Polymerizable Composition (2).

*(2)-(F) Polymerization Inhibitor*

Polymerizable Composition (2) preferably further includes a small amount of heat polymerization inhibitor in order to inhibit the undesired heat polymerization of a compound having an ethylenically unsaturated double bond which is polymerizable during the production or preservation of the composition. Examples of the heat polymerization inhibitor include hydroquinone, p-methoxyphenol, di-tert-butyl-p-cresol, pyrogallol, tert-butylcatechol, benzoquinone, 4,4'-thiobis(3-methyl-6-tert-butylphenol), 2,2'-methylenebis(4-methyl-6-tert- butylphenol), and N-nitrosophenyl-hydroxylamine cerium (I) salts.

The amount of the heat polymerization inhibitor to be added is preferably from about 0.01 % by mass to about 5% by mass with respect to the mass of the entire composition. If necessary, behenic acid or a higher fatty acid derivative

such as behenic acid amide may be added to the composition and may be localized in the surface of a sensitive layer during a drying process after coating, in order to prevent oxygen-induced inhibition of polymerization. The amount of the higher fatty acid derivative to be added is preferably from about 0.5% by mass to about 10% by mass with respect to the total amount of the composition.

(2)-(G) Colorant and the like

In order to form a colored photosensitive layer, a dye or a pigment may be added to the composition. This allows an improvement in the so-called plate checking capability of a printing plate, such as the visibility of the photosensitive layer after plate making and suitability for measurements with image density measuring machines. Many dyes used as colorants can reduce the sensitivity of the photopolymerizable photosensitive layer. In particular, therefore, pigments are preferably used as colorants. Examples of colorants include pigments such as phthalocyanine pigments, azo pigments, carbon blacks, and titanium oxide; and dyes such as ethyl violet, crystal violet, azo dyes, anthraquinone dyes, and cyanine dyes. The amount of the dye or the pigment is preferably from about 0.5 to about 5% by mass with respect to the total amount of the composition.

*(2)-(H) Other Additives*

In addition, known additives for modifying physical properties of cured films, such as an inorganic filler, a plasticizer or a lipophilizing agent, may also be added to the polymerizable composition.
Examples of the plasticizer include dioctyl phthalate, didodecyl phthalate, triethylene glycol dicaprylate, dimethyl glycol phthalate, tricresyl phosphate, dioctyl adipate, dibutyl sebacate, and triacetyl glycerin. When a binder is used, the plasticizer may be added in an amount of 10% by mass or less, based on the total mass of the ethylenically unsaturated double bond-containing compound and the binder.
Furthermore, in order to increase heating and exposure effects after the development for the purpose of enhancing the film strength (printing durability), a UV initiator, a thermal crosslinking agent, or the like may be added.
By applying such Polymerizable Composition (2) onto a support to form a photosensitive layer, a planographic printing plate precursor of the invention is obtained. The planographic printing plate precursor of the invention will be described later.
The photosensitive composition of the invention may be used for molding resins, casting resins, photo-molding resins, sealing materials, dental polymerizing materials, printing inks, paints, photosensitive resins for printing plates, color proofs for printing, resists for color filters, resists for black matrices, resists for printed boards, resists for semiconductor production, resists for microelectronics, resists for manufacture of micromachine components, insulating materials, hologram materials, waveguide materials, overcoat materials, adhesives, tackifiers, pressure-sensitive adhesives, and release coating agents.

*Color Filter and Production Method Thereof*

The color filter of the invention and the method for producing the same are described below.
A color filter according to an exemplary embodiment of the invention has a support and a colored pattern produced by using the polymerizable composition for a color filter of the invention (i.e., Polymerizable Composition (1)).
The color filter of the invention is described in detail below together with the method for the production thereof (the method of the invention for producing a color filter).
The method for producing a color filter includes: applying, to a support, the polymerizable composition for a color filter of the invention (i.e., Polymerizable Composition (1)) to form a polymerizable composition layer (hereinafter may also be abbreviated as "colored polymerizable composition layer forming step"); subjecting the polymerizable composition layer to pattern exposure with light (hereinafter may also be abbreviated as "exposure step"); and developing the exposed polymerizable composition layer to form a colored pattern (hereinafter may also be abbreviated as "development step").
Specifically, the polymerizable composition for a color filter of the invention is applied to a support (substrate) directly or with another layer interposed therebetween to form a polymerizable composition layer (i.e., colored polymerizable composition layer forming step); the coating film is exposed to light through a specified masking pattern to cure a portion of the film, which is exposed to the light (i.e., exposure step); and the coating film is developed with a liquid developer (i.e., development step) to form a patterned film having pixels of individual colors (three or four colors), so that the color filter of the invention can be produced.
The pattern exposure may be performed by scanning exposure with a laser as well as the method of exposing through a specified masking pattern.
Each step of the method of the invention for producing a color filter is described below.

*Colored Polymerizable Composition Layer Forming Step*

In the colored polymerizable composition layer forming step, the polymerizable composition for a color filter of the invention is applied to a support to form a colored polymerizable composition layer.
Examples of the support that may be used in this step include soda glass substrates, PYREX (registered trademark) glass substrates, quartz glass substrates, and substrates obtained by attaching transparent conductive films onto the foregoing glass substrates, which are for use in liquid crystal displays and the like, and optoelectric converting substrates for use in imaging devices, such as silicon substrates and complementary metal oxide film semiconductors (CMOS). In some cases, these substrates may have black stripes that separate pixels from one another.
If necessary, an undercoat layer may be formed on the support in order to improve adhesion to the upper layer, to prevent the substance diffusion, or to flatten the substrate surface.
The polymerizable composition for a color filter of the invention may be applied to the support by various coating methods such as slit coating, inkjet method, spin coating, cast coating, roll coating, and screen printing.
The thickness of the coating film of the polymerizable composition for a color filter is preferably from 0.1 μm to 10 μm, more preferably from 0.2 μm to 5 μm, and even more preferably from 0.2 μm to 3 μm.
When a color filter for a solid-state imaging device is produced, the thickness of the coating film of the polymerizable composition for a color filter is preferably from 0.35 μm to 1.5 μm, and more preferably from 0.40 μm to 1.0 μm, from the viewpoints of resolution and developability.
The polymerizable composition for a color filter which has been applied to the support generally is dried under conditions of from 70°C to 110°C for about 2 to about 4 minutes, whereby a colored polymerizable composition layer is formed.

*Exposure Step*

In the exposure step, the colored polymerizable composition layer formed in the colored polymerizable composition layer forming step is exposed to light through a mask, so that only the portion exposed to the light of the coating film is cured.
It is preferable that the exposure be performed by irradiation with a radiation. In particular, ultraviolet rays, such as g-line or i-line, are used suitably as the radiation to be used for the exposure. High-pressure mercury lamps are more preferable. The irradiation intensity is preferably from 5 mJ/cm$^2$ to 1,500 mJ/cm$^2$, more preferably from 10 mJ/cm$^2$ to 1,000 mJ/cm$^2$, and most preferably from 10 mJ/cm$^2$ to 800 mJ/cm$^2$.

*Development Step*

After the exposure step, alkali development (the development step) is performed, in which an unexposed portion resulting from the exposure step is dissolved in an aqueous alkali solution, whereby only a photo-cured portion remains.
The liquid developer preferably is an organic alkali developer that does not damage underlying circuits or the like. The development generally is performed at a temperature of from 20°C to 30°C for a time period of from 20 seconds to 90 seconds.
Examples of the alkali for use in the liquid developer include ammonia water and alkaline aqueous solutions prepared by diluting an organic alkaline compound, such as ethylamine, diethylamine, dimethylethanolamine, tetramethylammonium hydroxide, tetraethylammonium hydroxide, choline, pyrrole, piperidine and 1,8-diazabicyclo-[5,4,0]-7-undecene, with pure water so that the concentration of the compound would become 0.00 1 % by mass to 10% by mass, preferably of 0.01 % by mass to 1% by mass. When a liquid developer composed of such an alkaline aqueous solution is used, washing (rinsing) with pure water generally is performed after the development.
If necessary, the method for producing a color filter of the invention may further include curing the resultant colored pattern by heating and/or light exposure after the polymerizable composition layer forming step, the exposure step and the development step have been performed.
The colored polymerizable composition layer forming step, the exposure step and the development step (and optionally the curing step) are repeated at the number of times equal to the number of the desired hues, whereby a color filter having the desired hues is produced.

*Solid-state Imaging Device*

The solid-state imaging device of the invention includes the color filter of the invention.
The color filter according to an exemplary embodiment of the invention is produced by using the polymerizable composition for a color filter of the invention. In the color filter, therefore, the resultant colored pattern exhibits excellent adhesion to the substrate, and the cured composition has excellent resistance to development. Therefore, the exposure

sensitivity is high, the adhesion to the substrate of the exposed portion is excellent, and a high-resolution pattern having a desired cross sectional shape can be formed. Thus, the color filter of the invention is suitable for use in liquid crystal display devices and solid state imaging devices such as CCDs, and is particularly suitable for use in high-resolution CCD devices or CMOS devices having more than a million pixels. That is, it is preferable that the color filter of the invention be applied to solid-state imaging devices.

The color filter of the invention can be used, for example, as a color filter placed between a light-receiving portion of each pixel of a CCD and a microlens that collect light.

*Planographic Printing Plate Precursor*

Next, the planographic printing plate precursor of the invention is described.

The planographic printing plate precursor according to an exemplary embodiment of the invention at least has a photosensitive layer containing the polymerizable composition of the invention on a support.

A printing plate may be produced using the planographic printing plate precursor of the invention as follows. Polymerizable Composition (2) of the invention is applied to a support for a planographic printing plate directly or with another layer interposed therebetween to form a polymerizable composition layer, whereby a planographic printing plate precursor is produced. The photosensitive layer of this planographic printing plate precursor is subjected to pattern exposure, whereby only the exposed portion thereof is cured. After that, development is performed using a liquid developer, whereby a remaining photosensitive layer becomes an ink-receiving layer for printing and the area of the hydrophilic support which has been exposed by the removal of a photosensitive layer is wetted to become a water-receiving region. Thus, a planographic printing plate is obtained.

The planographic printing plate precursor of the invention may, if necessary, have another layer, such as a protective layer or an intermediate layer. Since the planographic printing plate precursor of the invention includes the polymerizable composition of the invention in the photosensitive layer, the planographic printing plate precursor has high sensitivity, excellent stability over time and excellent printing durability. Each of the elements which constitute the planographic printing plate precursor of the invention are described below.

*Photosensitive Layer*

The photosensitive layer is a layer containing the polymerizable composition of the invention. Specifically, the photosensitive layer may be formed by using Polymerizable Composition (2), which is one of the preferable exemplary embodiments of the polymerizable composition of the invention, as a composition for photosensitive layer formation (hereinafter also referred to as "composition for a photosensitive layer"), applying a coating liquid containing the composition to a support, and then drying the composition.

When the composition for a photosensitive layer is applied to the support, the components to be included in the composition may be used after being dissolved in various organic solvents. Examples of the solvent to be used include acetone, methyl ethyl ketone, cyclohexane, ethyl acetate, ethylene dichloride, tetrahydrofuran, toluene, ethylene glycol monomethyl ether, ethylene glycol monoethyl ether, ethylene glycol dimethyl ether, propylene glycol monomethyl ether, propylene glycol monoethyl ether, acetylacetone, cyclohexanone, diacetone alcohol, ethylene glycol monomethyl ether acetate, ethylene glycol ethyl ether acetate, ethylene glycol monoisopropyl ether, ethylene glycol monobutyl ether acetate, 3-methoxypropanol, methoxymethoxyethanol, diethylene glycol monomethyl ether, diethylene glycol monoethyl ether, diethylene glycol dimethyl ether, diethylene glycol diethyl ether, propylene glycol monomethyl ether acetate, propylene glycol monoethyl ether acetate, 3-methoxypropyl acetate, N,N-dimethylformamide, dimethyl sulfoxide, γ-butyrolactone, methyl lactate, and ethyl lactate. Such solvents may be used singly or in combination of two or more of them. The solid concentration in the coating solution preferably is from 2 to 50% by mass.

The coating amount of the photosensitive layer on the support may influence mainly the sensitivity and the developability of the photosensitive layer and the strength and the printing durability of an exposed film. It is desirable that the coating amount be adequately selected depending upon the intended application. When the coating amount is excessively small, the printing durability becomes insufficient. On the other hand, an excessively large coating amount is undesirable because it leads not only to decrease in sensitivity and a long period of time for the exposure to light, but also to a longer period of time for the development treatment. For a planographic printing plate for scanning exposure, which is a major purpose of the invention, the coating amount is preferably in the range of 0.1 $g/m^2$ to 10 $g/m^2$, and more preferably is 0.5 $g/m^2$ to 5 $g/m^2$ in terms of the mass after drying.

*Support*

In the planographic printing plate precursor of the invention, the support preferably has a hydrophilic surface. The hydrophilic support may be selected from conventionally-known hydrophilic supports used for planographic printing

plates, without particular limitations.

The support is preferably a dimensionally stable plate-shaped material, examples of which include paper, paper laminated with plastic (such as polyethylene, polypropylene, or polystyrene), a metal plate (such as of an aluminum, zinc or cupper plate), a plastic film (such as a film of cellulose diacetate, cellulose triacetate, cellulose propionate, cellulose butyrate, cellulose acetate butyrate, cellulose nitrate, polyethylene terephthalate, polyethylene, polystyrene, polypropylene, polycarbonate, or polyvinyl acetal), and paper or a plastic film on which metal such as those listed above is laminated or vapor-deposited. If necessary, the surface of these materials may be subjected to an appropriate known physical and/or chemical treatment for the purpose of imparting hydrophilicity, improving strength or the like.

In particular, examples of a preferable support include paper, polyester films and aluminum plates. Among them, aluminum plates are particularly preferable, which are dimensionally stable and relatively inexpensive and which can provide a surface having excellent hydrophilicity and strength by surface treatment optionally performed. A composite sheet having an aluminum sheet coupled on a polyethylene terephthalate film as described in JP-B No. 48-18327 is also preferable.

Examples of preferable aluminum plates include pure aluminum plates and alloy plates containing aluminum as the major component and a slight amount of foreign elements. Plastic films having aluminum laminated or vapor-deposited thereon may also be used. Examples of foreign elements which are included in the aluminum alloys include silicon, iron, manganese, copper, magnesium, chromium, zinc, bismuth, nickel, and titanium. The amount of foreign elements in the alloy is 10% by mass or less at most. The aluminum which is especially suitable in the invention is pure aluminum. However, since it is difficult to produce completely pure aluminum according to the current refining technology, a slight amount of foreign elements may be included. The composition of the aluminum plate which is applied in the invention in this way is not specified, and aluminum plates made of a material which has hitherto been publicly known and used can be properly utilized. The thickness of the aluminum plate which is used in the invention is from about 0.1 mm to 0.6 mm, preferably from 0.15 mm to 0.4 mm, and particularly preferably from 0.2 mm to 0.3 mm.

Moreover, when a support has a surface of a metal, particularly of aluminum, it is preferable that the support be subjected to surface treatment such as roughing (sand blasting) treatment, immersing treatment in an aqueous solution of sodium silicate, zirconium potassium fluoride, a phosphate, or the like, or anodic oxidation treatment.

The roughing treatment of the surface of the aluminum plate is performed by a variety of methods. For example, the roughing treatment is performed by a method of mechanically roughing the surface, a method of electrochemically dissolving and roughing the surface, or a method of selectively chemically dissolving the surface. Examples of employable mechanical methods include known methods such as a ball polishing method, a brush polishing method, a blast polishing method, and a buff polishing method. Examples of the electrochemical roughing method include a method in which roughing is performed in an electrolytic solution of hydrochloric acid, nitric acid, or the like by an alternating current or a direct current. A combination of both of these methods as disclosed in JP-A No. 54-63902 can also be utilized. Prior to roughing of the aluminum plate, if desired, degreasing treatment with, for example, a surfactant, an organic solvent or an alkaline aqueous solution, is carried out in order to remove a rolling oil on the surface.

An aluminum plate that has undergone immersion in an aqueous sodium silicate solution after surface roughening is preferably used. An aluminum plate that has undergone the processes of anodic oxidation of the aluminum plate and immersion in an aqueous alkali metal silicate solution as described in JP-B No. 47-5125 may also preferably be used. For example, the anodic oxidation may be performed by allowing a current to flow through the aluminum plate serving as an anode in an electrolyte solution containing one or more of aqueous or nonaqueous solutions of inorganic acids such as phosphoric acid, chromic acid, sulfuric acid, and boric acid, organic acids such as oxalic acid and sulfamic acid, or salts thereof.

The silicate electrodeposition described in U.S. No. 3,658,662 is also effective.

The surface treatment disclosed in JP-B No. 46-27481 and JP-A Nos. 52-58602 and 52-30503 is also useful which uses an electrolytically grained support in combination with the anodic oxidation and sodium silicate treatment.

In a preferred treatment, mechanical surface roughening, chemical etching, electrolytic graining, anodic oxidation, and sodium silicate treatment are sequentially performed as described in JP-A No. 56-28893.

After these treatments, undercoating may be preferably performed. The undercoating may include a water-soluble resin such as polyvinyl phosphonic acid, a polymer or copolymer having a sulfonic acid group in a side chain, or polyacrylic acid; a water-soluble metal salt (such as zinc borate); a yellow dye; or an amine salt.

A sol-gel-treated substrate is also preferably used in which a functional group capable of undergoing a radical addition reaction is covalently bonded as disclosed in JP-A No. 07-154983.

In another preferred example, a water-resistant hydrophilic layer may be formed as a surface layer on an arbitrary support. Examples of such a surface layer include a layer including an inorganic pigment and a binding agent as described in U.S. Patent No. 3,055,295 and JP-A No. 56-13168, a hydrophilic swelling layer as described in JP-A No. 09-80744, and a sol-gel film of titanium oxide, polyvinyl alcohol or silicates as described in Japanese Patent Application National Publication (Laid-Open) No. 08-507727.

The hydrophilization treatment is performed not only to render the support surface hydrophilic but also to prevent

a harmful reaction of the photopolymerizable composition provided thereon and, at the same time, to improve adhesion of the photosensitive layer.

*Protective Layer*

It is preferable for the planographic printing plate precursor of the invention to further have a protective layer on the photosensitive layer. The protective layer prevents low-molecular-weight compounds, for example, oxygen or basic substance present in the air, which inhibit the image forming reaction generated upon the exposure in the photosensitive layer, from permeating into the photosensitive layer and enables exposure in the air. Accordingly, the protective layer is required to have the following properties, that is, to have low permeability to oxygen or low-molecular-weight compounds, not to substantially inhibit the transmission of light used for the exposure, to exhibit good adhesion to the photosensitive layer, and to be easily removable in the development step after the exposure.

Contrivances on the protective layer have been heretofore made and described in detail in U.S. Patent No. 3,458,311 and JP-B No. 55-49729. Preferable examples of the material which can be used for the protective layer include water-soluble polymer compounds having a relatively high crystallinity. Specifically, water-soluble polymers such as polyvinyl alcohol, polyvinyl pyrrolidone, acidic celluloses, gelatin, gum arabic and polyacrylic acid are known. Among these, when polyvinyl alcohol is used as a main component, the best results can be obtained with respect to fundamental properties, for example, oxygen blocking property and removability by development.

Polyvinyl alcohol for use in the protective layer may be partially substituted by at least one of an ester, an ether or an acetal, as long as it has an unsubstituted vinyl alcohol unit for imparting necessary oxygen barrier characteristics and water solubility. The polyvinyl alcohol may partially have at least one other copolymerization component. Specifically, the polyvinyl alcohol may be hydrolyzed to a degree of 71 to 100 mol% and may have a mass average molecular weight in the range of from 300 to 2,400. Examples of the polyvinyl alcohol include PVA-105, PVA-110, PVA-117, PVA-117H, PVA-120, PVA-124, PVA-124H, PVA-CS, PVA-CST, PVA-HC, PVA-203, PVA-204, PVA-205, PVA-210, PVA-217, PVA-220, PVA-224, PVA-217EE, PVA-217E, PVA-220E, PVA-224E, PVA-405, PVA-420, PVA-613, and L-8 (all trade names, manufactured by Kuraray Co., Ltd.).

The components (choice of PVAs, use of additives and the like), the coating amount and the like of the protective layer may be selected in consideration of on oxygen barrier characteristics, removability by development, fogging, adhesion property, or scratch resistance. In general, a higher rate of hydrolysis of the PVA to be used (a higher content of unsubstituted vinyl alcohol units in the protective layer) or a larger thickness of the film leads to a higher level of oxygen barrier characteristics, which is advantageous in terms of sensitivity. When oxygen barrier characteristics are increased excessively, problems may occur such as unnecessary polymerization reactions during manufacture or storage and unnecessary fogging or thickening of image lines during pattern exposure. The adhesion to the image portion and the scratch resistance are also very important for the handleability of the printing plate. Methods for applying the protective layer are described in detail in, for example, U.S. Patent No. 3,458,313 and JP-A No. 55-49729.

The protective layer may also have another function. For example, a colorant (such as a water-soluble dye) capable of efficiently transmitting light in the range of 350 nm to 450 nm to be used for exposure and efficiently absorbing light at 500 nm or more may be added to the protective layer so that safe light suitability can be further improved without a reduction in sensitivity.

*Other Layers*

Besides, a layer for improving the adhesion between the photosensitive layer and the support or for increasing the removability of an unexposed photosensitive layer by development may be provided. For example, the adhesion is improved by the addition of a compound which exhibits a relatively strong interaction with the support, such as compounds having a diazonium structure or phosphonic compounds, or by an undercoating layer, leading successfully to improvement in printing durability. In the meantime, the developability of a non-image portion is improved by the addition of a hydrophilic polymer such as polyacrylic acid or polysulfonic acid or by an undercoating layer, leading successfully to improvement in stain resistance.

*Plate Making*

A planographic printing plate precursor usually is subjected to image exposure, and then unexposed portions of the photosensitive layer are removed using a liquid developer, whereby an image is obtained.

Any known light exposure method may be applied to the planographic printing plate precursor of the invention, without limitations. The wavelength of the light source is preferably from 350 nm to 450 nm, and specifically InGaN semiconductor lasers are preferred. The exposure mechanism may be of any system such as an internal drum system, an external drum system or a flathead system. A highly water-soluble component may be used for the photosensitive

layer so that the layer can dissolved in neutral water or weakly alkaline water. The planographic printing plate having such a constitution may be subjected to on-press exposure and development after mounted on a printing machine.

Available laser light sources in the range of 350 nm to 450 nm include the lasers described below:

Gas lasers including Ar ion lasers (364 nm, 351 nm, 10 mW to 1 W), Kr ion lasers (356 nm, 351 nm, 10 mW to 1 W) and He-Cd lasers (441 nm, 325 nm, 1 to 100 mW); Solid-state lasers including a combination of Nd:YAG ($YVO_4$) and SHG crystal (twice) (355 nm, 5 mW to 1 W) and a combination of Cr:LiSAF and SHG crystal (430 nm, 10 mW); Semiconductor lasers including $KNbO_3$ ring resonators (430 nm, 30 mW), a combination of a waveguide type wavelength converting device and an AlGaAs or InGaAs semiconductor (380 to 450 nm, 5 to 100 mW), a combination of a waveguide type wavelength converting device and an AlGaInP or AlGaAs semiconductor (300 to 350 nm, 5 to 100 mW), and AlGaInN lasers (350 to 450 nm, 5 to 30 mW); Other lasers including pulsed lasers such as $N_2$ lasers (337 nm, 0.1 to 10 mJ pulse) and XeF lasers (351 nm, 10 to 250 mJ pulse).

Above all, AlGaInN semiconductor lasers (commercially available InGaN semiconductor lasers) (400 to 410 nm, 5 to 30 mW) are particularly preferred in view of wavelength characteristics and cost.

For scanning exposure type planographic printing plate exposure systems, internal drum, external drum, or flathead systems may be used as exposure mechanisms, and all of the light sources describe above, except for pulsed lasers, may be used as light sources. Practically, the exposure systems described below are particularly preferred in view of the relationship between the sensitivity of the material and plate making time.

**[0360]** - An exposure device of single beam using one gas laser or solid laser light source according to the internal drum system.

- An exposure device of multi-beams using many (ten or more) semiconductor lasers according to the flat bed system.
- An exposure device of multi-beams using many (ten or more) semiconductor lasers according to the external drum system.

In laser direct drawing type planographic printing plates such as those described above, the following equation (eq 1) generally is valid among the sensitivity X ($J/cm^2$) of a photosensitive material, the exposed area S ($cm^2$) of a photosensitive material, the power q (W) per one laser light source, the number n of lasers, and the entire exposure time t (s).

$$X \cdot S = n \cdot q \cdot t \qquad (eq1)$$

*i) Case of Internal Drum (Single Beam) System:*

In general, the following equation (eq 2) is valid among the revolution number f (radian/s) of laser, the sub-scanning length Lx (cm) of photosensitive material, the resolution Z (dots/cm), and the entire exposure time t (s).

$$f \cdot Z \cdot t = Lx \qquad (eq2)$$

*ii) Case of External Drum (Multi-Beam) System:*

In general, the following equation (eq 3) is valid among the revolution number F (radian/s) of drum, the sub-scanning length Lx (cm) of photosensitive material, the resolution Z (dots/cm), the entire exposure time t (s), and the number (n) of beams.

$$F \cdot Z \cdot n \cdot t = Lx \qquad (eq3)$$

*iii) Case of Flat Bed (Multi-Beam) System:*

In general, the following equation (eq 4) is valid among the revolution number H (radian/s) of a polygon mirror, the sub-scanning length Lx (cm) of photosensitive material, the resolution Z (dots/cm), the entire exposure time t (s), and the number (n) of beams.

$$H \cdot Z \cdot n \cdot t = Lx \qquad (eq4)$$

Substitution of the resolution (2560 dpi) required of a practical printing plate, the size of the plate (A1/B1, 42 inches in sub-scanning length), the exposure condition of about 20 sheets per hour, and the photosensitive characteristics of the photopolymerizable composition of the invention (the photosensitive wavelength, and a sensitivity of about 0.1 $mJ/cm^2$) into the equations suggests that the photosensitive material of the invention should preferably be used in combination with a semiconductor laser of a multi-beam exposure type. Also taking handleability and cost into account, it is suggested that the photosensitive material of the invention is most preferably used in combination with a semiconductor laser, multi-beam exposure system of an external drum system.

Furthermore, other exposure light sources such as mercury vapor lamps including ultra-high pressure mercury vapor lamps, high pressure mercury vapor lamps, medium pressure mercury vapor lamps and low pressure mercury vapor lamps, chemical lamps, carbon arc lamps, xenon lamps, metal halide lamps, visible laser lamps, ultraviolet laser lamps, fluorescent lamps, tungsten lamps, or sunlight may also be used.

Examples of a liquid developer suitable for the planographic printing plate precursor of the invention include the liquid developers disclosed in JP-B No. 57-7427. Suitable examples thereof include aqueous solutions of inorganic alkali agents (for example, sodium silicate, potassium silicate, sodium hydroxide, potassium hydroxide, lithium hydroxide, sodium triphosphate, sodium diphosphate, ammonium triphosphate, ammonium diphosphate, sodium metasilicate, sodium bicarbonate, or ammonia water) and aqueous solutions of organic alkali agents (for example, monoethanolamine or diethanolamine). Such an alkali solution is added so that a concentration of 0.1% by mass to 10% by mass, and preferably 0.5% by mass to 5% by mass will be achieved.

The alkaline aqueous solution may, if necessary, include a small amount of a surfactant or an organic solvent, such as benzyl alcohol, 2-phenoxyethanol or 2-butoxyethanol. Examples thereof include those described in U.S. Patents No. 3,375,171 and 3,615,480.

Furthermore, the liquid developers disclosed in JP-A Nos. 50-26601, 58-54341, JP-B Nos. 56-39464 and 56-42860 are also preferable.

A particularly preferable liquid developer may be a liquid developer containing a nonionic compound represented by Formula ($\alpha$) shown below and having a pH of 11.5 to 12.8 and a conductivity of 3 mS/cm to 30 mS/cm, as described in JP-A No. 2002-202616.

$$A\text{-}W \qquad \text{Formula } (\alpha)$$

In Formula ($\alpha$), A represents a hydrophobic organic group, which may form A-H having log P of 1.5 or more, and W represents a nonionic hydrophilic organic group which may form W-H having log P of less than 1.0.

The term "log P" is generally used as a hydrophobicity parameter which is described in C. Hansch and A. Leo, Substituent Constants for Correlation Analysis in Chemistry and Biology, J. Wiley & Sons (1979). The log P is defined as a logarithm of an equilibrium concentration ratio P calculated from the proportion of objective molecules (A-H and W-H) distributed to each layer of an octanol/water two-layer system. The log P value is used here as an index for specifying each organic group of A and W in Formula ($\alpha$), and for the convenience's sake, assuming that an A-H or W-H structure is formed by bonding a hydrogen atom to each organic group A or W, the log P value is determined by calculation from known data according to the method described in A. K. Ghose, et al., J. Comput. Chem., 9, 80 (1988).

The components of the liquid developer are described in detail in paragraphs (0024) to (0067) of JP-A No. 2002-202616.

It is effective to add the nonionic compound represented by Formula ($\alpha$) in an amount of from 0.1% by mass to 15% by mass, preferably from 1.0% by mass to 8.0% by mass, to the liquid developer.

In a plate-making process of the planographic printing plate precursor, the entire surface may, if necessary, be heated before or during exposure or between exposure and development. By the heating, an image forming reaction in the photosensitive layer is accelerated, and effects such as improvement in sensitivity or in printing durability and stabilization of sensitivity may be achieved. For the purpose of improving the image strength and the printing durability, it is also effective to apply entire post-heating or entire exposure to the image after development. Usually, the heating before development preferably is performed under a mild condition of 150°C or lower. When the temperature is 150°C or lower, no fogging problem occurs in non-image portions. The heating after development uses very strong conditions, usually within the range of 200°C to 500°C. A sufficient effect of strengthening images is obtained at a temperature of 200°C or higher, whereas problems, for example, deterioration of a support and thermal decomposition of image areas fail to occur at a temperature of 500°C or lower.

## EXAMPLES

Hereinafter, the present invention is described more specifically by way of examples. However, the invention

is not limited to the examples unless departing from the gist of the invention. Unless otherwise stated, "part" is based on mass and "%" is "% by mass".

First, details of the specified oxime compounds to be used in Examples (Specified Compounds 1 to 9) and the comparative compounds to be used in Comparative Examples (Comparative Compounds 1 to 4) are shown below.

| Compound No. | Structure |
|---|---|
| Specified Compound 1 | 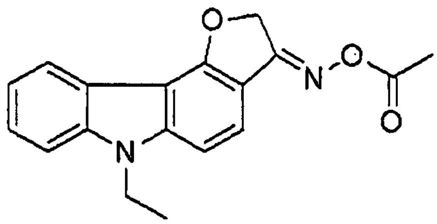 |
| Specified Compound 2 | 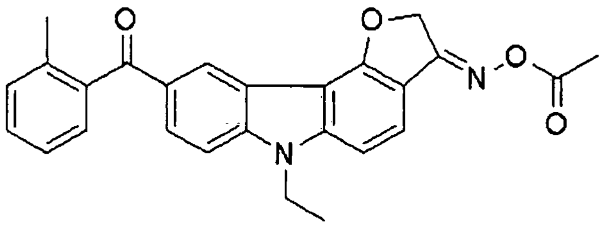 |
| Specified Compound 3 | 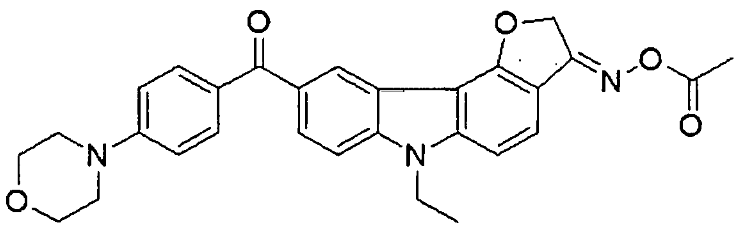 |
| Specified Compound 4 | 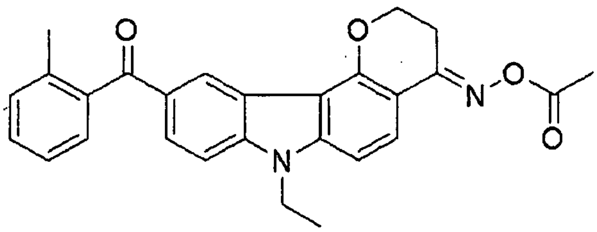 |
| Specified Compound 5 | 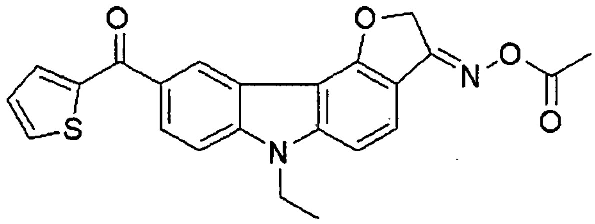 |
| Specified Compound 6 | 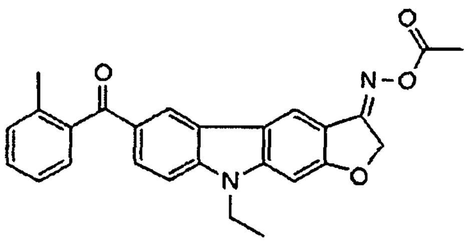 |

(continued)

| Compound No. | Structure |
|---|---|
| Specified Compound 7 | 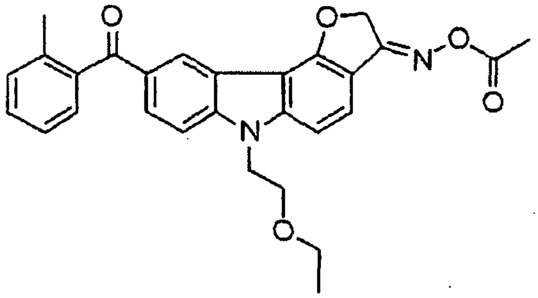 |
| Specified Compound 8 | 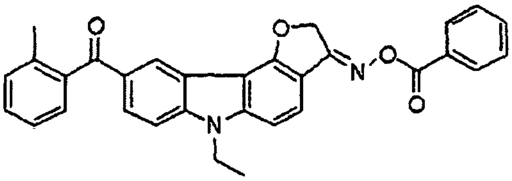 |
| Specified Compound 9 | 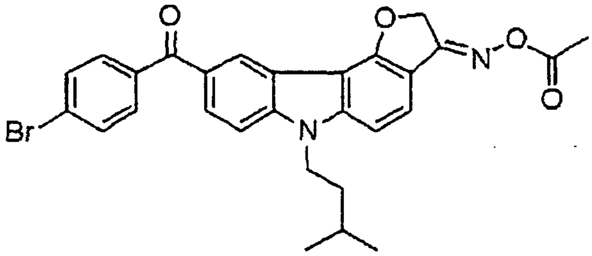 |
| Comparative Compound 1 | IRGACURE OXE 01 (manufactured by Ciba Specialty Chemicals) |
| Comparative Compound 2 | IRGACURE OXE 02 (manufactured by Ciba Specialty Chemicals) |
| Comparative Compound 3 | 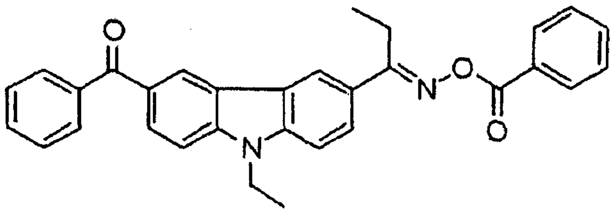 |
| Comparative Compound 4 | 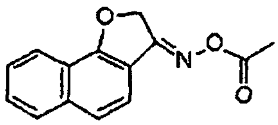 |

Specified Compounds 1 to 9 which are the specified oxime compounds in the list above were synthesized by the methods described below.

*Synthesis Example 1: Synthesis of Specified Compound 1 (Specified Oxime Compound)*

Compound A (2.0 g, 7.5 mmol) shown below was dissolved in 50 ml of pyridine, followed by the addition of triethylamine (1.14 g, 11.3 mmol). After cooling to 0°C, acetyl chloride (0.77 g, 9.8 mmol) was added by dropping over 20 minutes, followed by increasing the temperature to room temperature and stirring for 2 hours. The reaction solution was added by dropping to 150 ml of distilled water having been cooled to 0°C, followed by extraction of the organic layer using ethyl acetate. Then, magnesium sulfate was added to dry the extraction, followed by evaporation of the solvent under reduced pressure. The resultant crystals were washed with methanol and then recrystallized using ethyl acetate, thereby obtaining Specified Compound 1 having the following structure (yield: 1.1 g, 48%).

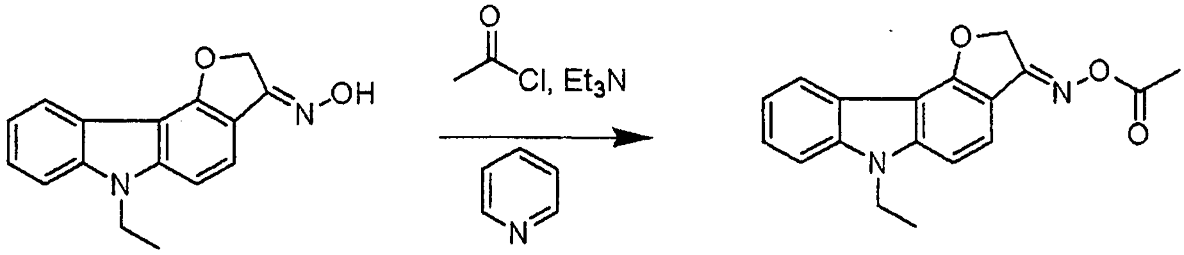


Compound A

Specified Compound 1

The structure of Specified Compound 1 thus obtained was identified by NMR. (1H-NMR, 400 MHz, deuterated chloroform): 8.15 (d, 1H, J= 7.8 Hz), 7.85 (d, 1H, J = 7.8 Hz), 7.50-7.47 (m, 2H), 7.32 (t, 1H, J = 7.6 Hz), 7.12 (d, 1H, J = 8.0 Hz), 5.43 (s, 2H), 4.41 (q, 2H, 7.2 Hz), 2.26 (s, 3H),1.46 (t, 3H, 7.2 Hz)

The molar absorption coefficient at 365 nm of Specified Compound 1 was 7,410 in ethyl acetate.

$$\lambda max \text{ in a range between 300 nm or more and less than 400 nm} = 360 \text{ nm}$$

$$\text{Molar absorption coefficient } (\lambda max) = 31{,}207, \text{ molar absorption coefficient } (\lambda max + 20 \text{ nm}) = 76$$

*Synthesis Example 2: Synthesis of Specified Compound 2 (Specified Oxime Compound)*

Compound B (3.0 g, 7.8 mmol) shown below was dissolved in 50 ml of pyridine, followed by the addition of triethylamine (1.18 g, 11.7 mmol). After cooling to 0°C, acetyl chloride (0.79 g, 10.1 mmol) was added by dropping over 20 minutes, followed by increasing the temperature to room temperature and stirring for 2 hours. The reaction solution was added by dropping to 150 ml of distilled water having been cooled to 0°C, followed by extraction of the organic layer using ethyl acetate. Then, magnesium sulfate was added to dry the extraction, followed by evaporation of the solvent under reduced pressure. The resultant crystals were washed with 2-propanol and then recrystallized using 2-propanol, thereby obtaining Specified Compound 2 having the following structure (yield: 2.2 g, 66%).

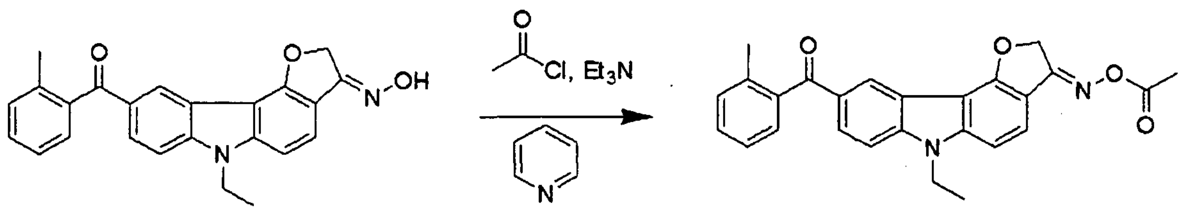


Compound B

Specified Compound 2

The structure of Specified Compound 2 thus obtained was identified by NMR. (1H-NMR, 400 MHz, deuterated chloroform): 8.65 (s, 1H), 8.00(d, 1H, J= 7.8 Hz), 7.92 (d, 1H, J = 7.8 Hz), 7.50-7.20 (m, 5H), 7.15 (d, 1H, J = 7.8 Hz), 5.41 (s, 2H), 4.43 (q, 2H, J = 6.8 Hz), 2.36 (s, 3H), 2.26 (s, 3H), 1.49 (t, 3H, J = 6.8 Hz).

The molar absorption coefficient at 365 nm of Specified Compound 2 was 29,934 in ethyl acetate.

$$\lambda max \text{ in a range between 300 nm or more and less than 400 nm} = 365 \text{ nm}$$

$$\text{Molar absorption coefficient } (\lambda max) = 29{,}934, \text{ molar absorption coefficient } (\lambda max + 20 \text{ nm}) = 383$$

Specified Compounds 3 to 9, which are the specified compounds shown in the above list, were synthesized by methods similar to those described in Synthesis Examples 1 and 2. The molar absorption coefficients of the obtained compounds in ethyl acetate are shown below, respectively.

Table 1

| | λ max (nm) | Molar absorption coefficient at λmax | Molar absorption coefficient at (λmax + 20 nm) |
|---|---|---|---|
| Specified Compound 1 | 360 | 31207 | 76 |
| Specified Compound 2 | 365 | 29934 | 383 |
| Specified Compound 3 | 371 | 31400 | 210 |
| Specified Compound 4 | 367 | 32101 | 323 |
| Specified Compound 5 | 383 | 28709 | 214 |
| Specified Compound 6 | 357 | 30557 | 264 |
| Specified Compound 7 | 364 | 31298 | 156 |
| Specified Compound 8 | 365 | 29967 | 56 |
| Specified Compound 9 | 373 | 34221 | 198 |
| Comparative Compound 1 | 328 | 17577 | 10172 |
| Comparative Compound 2 | 323 | 20516 | 5496 |
| Comparative Compound 3 | 328 | 19878 | 6908 |
| Comparative Compound 4 | 368 | 15030 | 1121 |

The structures of the Comparative Compound 1 and the Comparative Compound 2 shown in the list are as follows.

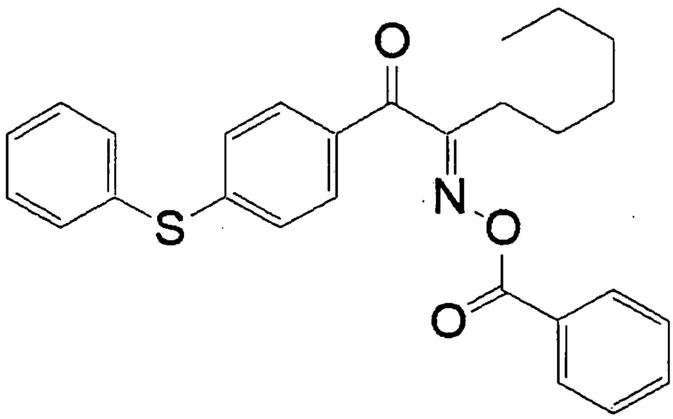

IRGACURE OXE 01

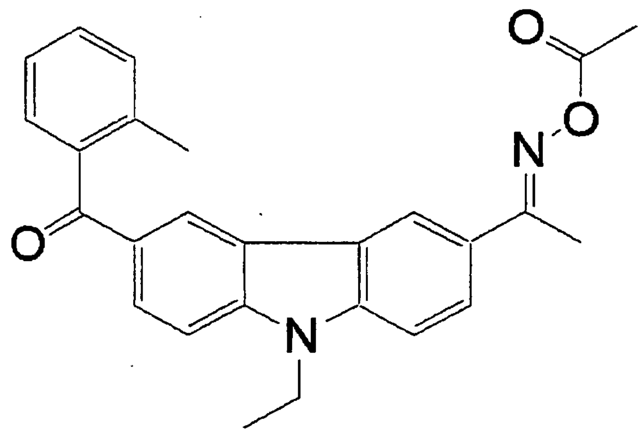

IRGACURE OXE 02

*Example 1-1*

*Preparation and Evaluation of Polymerizable Composition 1*

Polymerizable Composition 1 was prepared as described below, and its sensitivity was evaluated.

A uniform composition was prepared which included 0.08 mmol of Specified Compound 1 as a specific oxime compound, 1 g of pentaerythritol tetraacrylate as a radical-polymerizable compound, 1 g of polymethyl methacrylate (c.a. 996,000 in molecular weight, manufactured by Aldrich) as a binder resin, and 16 g of cyclohexanone as a solvent. The resultant composition was used as a coating liquid, applied to a glass plate using a spin coater, and dried at 40°C for 10 minutes, whereby a 1.5 μm-thick coating film was formed. A21 √2 step tablet (a gray scale film manufactured by Dainippon Screen Mfg. Co., Ltd.) was placed on the coating film. The coating film was exposed to light from a 500 mW high-pressure mercury lamp (manufactured by Ushio Inc.) through a heat-ray-cutting filter for 30 seconds, and then immersed in toluene for 60 seconds for development. The step number of the step tablet where the film was completely cured and insolubilized was evaluated as the sensitivity. The sensitivity was step No. 7.

The larger the step number, the higher the sensitivity.

*Examples 1-2 to 1-9 and Comparative Examples 1-1 to 1-4*

Polymerizable Compositions 2 to 13 were each prepared in the same manner as in Example 1-1, except that 0.08 mmol of each of the compounds (Specified Compounds 2 to 9 and Comparative Compounds 1 to 4) shown in the above list was used in place of 0.08 mmol of Specified Compound 1 used as the specified oxime compound. Then, the step number for the sensitivity was evaluated in the same manner as in Example 1-1.

The results of the evaluation of Examples 1-1 to 1-9 and Comparative Examples 1-1 to 1-4 are shown in Table 2.

Table 2

| | Polymerizable Composition | Compound | Polymerizable Composition | Sensitivity Step Number |
|---|---|---|---|---|
| Example 1-1 | 1 | Specified Compound 1 | 1 | 7 |
| Example 1-2 | 2 | Specified Compound 2 | 2 | 9 |
| Example 1-3 | 3 | Specified Compound 3 | 3 | 8 |
| Example 1-4 | 4 | Specified Compound 4 | 4 | 9 |
| Example 1-5 | 5 | Specified Compound 5 | 5 | 9 |
| Example 1-6 | 6 | Specified Compound 6 | 6 | 9 |

(continued)

| | Polymerizable Composition | Compound | Polymerizable Composition | Sensitivity Step Number |
|---|---|---|---|---|
| Example 1-7 | 7 | Specified Compound 7 | 7 | 9 |
| Example 1-8 | 8 | Specified Compound 8 | 8 | 7 |
| Example 1-9 | 9 | Specified Compound 9 | 9 | 8 |
| Comparative Example 1-1 | 10 | Comparative Compound 1 | 10 | 5 |
| Comparative Example 1-2 | 11 | Comparative Compound 2 | 11 | 5 |
| Comparative Example 1-3 | 12 | Comparative Compound 3 | 12 | 5 |
| Comparative Example 1-4 | 13 | Comparative Compound 4 | 13 | 6 |

*Example 2-1*

*1. Preparation of Colored Polymerizable Composition A-1*

A negative colored polymerizable composition (A-1) containing a colorant (pigment) was prepared as a polymerizable composition for formation of a color filter. A color filter was prepared using the colored polymerizable composition.

*1-1. Preparation of Pigment Dispersion Liquid (P1)*

A liquid mixture composed of 40 parts by mass of a pigment mixture of C.I. Pigment Green 36 and C.I. Pigment Yellow 219 (30/70 in mass ratio), 10 parts by mass (45.1 parts by mass in terms of solid content) of BYK 2001 (DISPERBYK, manufactured by BYK-Chemie, 45.1% by mass in solid content) as a dispersant, and 150 parts by mass of ethyl 3-ethoxypropionate as a solvent was mixed and dispersed in a bead mill for 15 hours, whereby Pigment Dispersion Liquid (P1) was prepared.

The average particle size of the pigment in the resultant Pigment Dispersion Liquid (P1) was measured to be 200 nm by dynamic light scattering.

*1-2. Preparation of Colored Polymerizable Composition A-1 (Coating Liquid)*

The components for Composition A-1 shown below were mixed and dissolved to form a colored polymerizable composition A-1.

Composition A-1

- Pigment Dispersion Liquid (P1): 600 parts by mass
- Alkali-soluble resin: 200 parts by mass (benzyl methacrylate/methacrylic acid/hydroxyethyl methacrylate copolymer, 80/10/10 in molar ratio, Mw: 10,000)
- Polyfunctional monomer (dipentaerythritol hexaacrylate): 60 parts by mass
- Specified oxime compound (Specified Compound 1): 60 parts by mass
- Solvent (propylene glycol monomethyl ether acetate): 1,000 parts by mass
- Surfactant (trade name: TETRONIC 150R[1], manufactured by BASF): 1 part by mass
- $\gamma$-methacryloxypropyltriethoxysilane: 5 parts by mass

*2. Preparation of Color Filter*

*2-1. Formation of Colored Polymerizable Composition Layer*

The resulting pigment-containing colored polymerizable composition A-1 was used as a resist solution. The resist solution was applied to a glass substrate of 550 mm × 650 mm by slit coating under the conditions described below and then allowed to stand for 10 minutes. The solution was then dried under vacuum and pre-baked (100°C for 80 seconds) to form a polymerizable composition coating film (polymerizable composition layer).

*Slit Coating Conditions*

Opening gap of the top of the coating head: 50 μm
Coating speed: 100 mm/second
Clearance between the substrate and the coating head: 150 μm
Coating thickness (dry thickness): 2 μm
Coating temperature: 23°C

*2-2. Exposure and Development*

The colored polymerizable composition layer was then subjected to pattern exposure using a 2.5 kW ultra-high pressure mercury lamp. After the exposure to light, the entire surface of the exposed colored polymerizable composition layer was covered with an aqueous 10% solution of a liquid organic developer (trade name: CD, manufactured by Fuji Film Electronics Materials Co., Ltd.) and was allowed to stand for 60 seconds.

*2-3. Heat Treatment*

A shower of pure water was then sprayed on the colored polymerizable composition layer to wash away the liquid developer. The composition was then heated in an oven at 220°C for 1 hour (post-baking), thereby obtaining a color filter having a colored pattern on the glass substrate.

*3. Performance Evaluation*

Evaluations were performed as described below with respect to the storage stability and exposure sensitivity of the colored polymerizable composition, the developability of a colored patterned product formed on a glass substrate by using the colored polymerizable composition, the coloration caused by heat aging and the adhesion to the substrate of the colored patterned product, and the cross-sectional pattern shape and the post-heating cross-sectional pattern shape. A summary of the evaluation results is shown in Table 3.

*3-1. Storage Stability of Colored Polymerizable Composition*

After the colored polymerizable composition was stored at room temperature for one month, the degree of precipitation of matters was visually evaluated according to the following criteria.

*Criteria:*

A: No precipitation was observed.
B: Precipitation was slightly observed.
C: Precipitation was observed.

*3-2. Exposure Sensitivity of Colored Polymerizable Composition*

The colored polymerizable composition was applied to a glass substrate by spin coating and then was dried to form a coating film having a thickness of 1.0 μm. Spin coating conditions were 300 rpm for 5 seconds and then 800 rpm for 20 seconds. Drying conditions were 100°C for 80 seconds. The resulting coating film was then exposed to light

through a test photomask with a line width of 2.0 μm at different exposure amounts in the range of 10 $mJ/cm^2$ to 1,600 $mJ/cm^2$ using a proximity exposure system equipped with an ultra-high pressure mercury lamp (manufactured by Hitachi High-Tech Electronics Engineering Co., Ltd.). The exposed coating film was then developed with a liquid developer 60% CD-2000 (manufactured by Fuji Film Electronics Materials Co., Ltd.) at 25°C for 60 seconds. Thereafter, the film was rinsed with running water for 20 seconds and then spray-dried so that the patterning was completed.

Regarding the evaluation of exposure sensitivity, the minimum exposure amount at which the post-development thickness of the region that was irradiated with light in the exposure step became 95% or more of the thickness (100%) of the film before the exposure was evaluated as the exposure sensitivity. A smaller exposure sensitivity indicates a higher sensitivity.

*3-3. Developability, Cross-Sectional Shape of Pattern, Adhesion to Substrate*

After the post-baking described in the section 2-3 "Heat Treatment", the surface and the cross-sectional shape of the substrate were observed by common methods with an optical microscope and SEM photographs, so as to evaluate the developability, the adhesion to the substrate, the color change due to forced heat aging and the cross-sectional pattern shape. Details of the evaluation method are as described below.

*Developability*

The presence or absence of residues in the region (unexposed portion) which was not irradiated with light in the exposure step was observed, so as to evaluate the developability. The evaluation was preformed according to the following criteria.

*Criteria*

A: No residue was observed in the unexposed portion.
B: Residues were slightly observed in the unexposed portion, but the residue level was practically acceptable.
C: Residues were significantly observed in the unexposed portion.

*Adhesion to Substrate*

The presence or absence of pattern defects was observed, and the adhesion to the substrate was evaluated according to the following criteria.

*Criteria*

A: No pattern defect was observed.
B: Pattern defects were hardly observed but observed in part.
C: Many pattern defects were significantly observed.

*Evaluation of Coloration by Forced Heat Aging*

The exposed and developed polymerizable composition layer (the colored pattern) was heated on a hot plate at 200°C for 1 hour, and the color difference $\Delta Eab^*$ between before and after the heating was evaluated with MCPD-3000 manufactured by Otsuka Electronics Co., Ltd. according to the following criteria.

*Criteria*

A: $\Delta Eab^* \leq 5$

B: $5 < \Delta Eab^* < 8$

C: ΔEab* ≥ 8

*Cross-Sectional Shape of Pattern*

The cross-sectional shape of the pattern was observed and evaluated. The cross-sectional shape of the pattern preferably is forward-tapered, and most preferably is rectangular. The reverse-tapered shape is undesirable.

*Post-Heating Cross-Sectional Shape of Pattern*

After the post-baking described in the section 2-3 "Heat Treatment", the cross-sectional shape of the resulting pattern was observed and evaluated. The cross-sectional shape of the pattern preferably is forward-tapered, and most preferably is rectangular. The reverse-tapered shape is undesirable.

*Examples 2-2 to 2-17 and Comparative Examples 2-1 to 2-3*

Colored polymerizable compositions A-2 to A-17 and A'-1 to A'-3 and color filters were prepared in the same manner as in Example 2-1, except that the respective compounds in the amounts shown in Table 2 below were used in place of 60 parts by mass of Specified Compound 1 (the specific oxime compound) of the composition A-1 used in the preparation of the colored photopolymerizable composition A-1 and that a sensitizing agent and a co-sensitizing agent of the type and in the amount shown in Table 2 was further added in each of Examples 2-10 to 2-17. Evaluations were also performed in the same way as in Example 2-1. The results are shown in Table 3.

Table 3

| | Colored Polymerizable Composition | Compound | | | Sensitizing Agent | | Co-sensitizing Agent | |
|---|---|---|---|---|---|---|---|---|
| | | Specified | Comparative | Content (parts by mass) | Kind | Content (parts by mass) | Kind | Content (parts by mass) |
| Example 2-1 | A-1 | 1 | - | 60 | - | - | - | - |
| Example 2-2 | A-2 | 2 | - | 60 | - | - | - | - |
| Example 2-3 | A-3 | 3 | - | 60 | - | - | - | - |
| Example 2-4 | A-4 | 4 | - | 60 | - | - | - | - |
| Example 2-5 | A-5 | 5 | - | 60 | - | - | - | - |
| Example 2-6 | A-6 | 6 | - | 60 | - | - | - | - |
| Example 2-7 | A-7 | 7 | - | 60 | - | - | - | - |
| Example 2-8 | A-8 | 8 | - | 60 | - | - | - | - |
| Example 2-9 | A-9 | 9 | - | 60 | - | - | - | - |
| Example 2-10 | A-10 | 2 | - | 50 | A1 | 10 | - | - |
| Example 2-11 | A-11 | 2 | - | 50 | A2 | 10 | - | - |
| Example 2-12 | A-12 | 2 | - | 50 | A3 | 10 | - | - |
| Example 2-13 | A-13 | 2 | - | 50 | - | - | F1 | 10 |
| Example 2-14 | A-14 | 2 | - | 40 | A2 | 10 | F1 | 10 |
| Example 2-15 | A-15 | 2 | - | 40 | A2 | 10 | F2 | 10 |
| Example 2-16 | A-16 | 2 | - | 40 | A2 | 10 | F3 | 10 |
| Example 2-17 | A-17 | 2 | - | 40 | A2 | 10 | F3<br>LD-5 | 10<br>10 |
| Comparative Example 2-1 | A'-1 | - | 1 | 60 | - | - | - | - |
| Comparative Example 2-2 | A'-2 | - | 2 | 60 | - | - | - | - |
| Comparative Example 2-3 | A'-3 | - | 3 | 60 | - | - | - | - |

Table 3 (continued)

| | Storage Stability | Exposure Sensitivity (mJ/cm²) | Developability | Color Change by Forced Heat Aging | Adhesion to Substrate | Cross-sectional Shape of Pattern | Post-heating Cross-sectional Shape of Pattern |
|---|---|---|---|---|---|---|---|
| Example 2-1 | A | 110 | A | A | A | Rectangular | Rectangular |
| Example 2-2 | A | 90 | A | A | A | Rectangular | Rectangular |
| Example 2-3 | A | 90 | A | A | A | Rectangular | Rectangular |
| Example 2-4 | A | 90 | A | A | A | Rectangular | Rectangular |
| Example 2-5 | A | 90 | A | A | A | Rectangular | Rectangular |
| Example 2-6 | A | 90 | A | A | A | Rectangular | Rectangular |
| Example 2-7 | A | 90 | A | A | A | Rectangular | Rectangular |
| Example 2-8 | A | 120 | A | A | A | Rectangular | Rectangular |
| Example 2-9 | A | 100 | A | A | A | Rectangular | Rectangular |
| Example 2-10 | A | 90 | A | A | A | Rectangular | Rectangular |
| Example 2-11 | A | 80 | A | A | A | Rectangular | Rectangular |
| Example 2-12 | A | 90 | A | A | A | Rectangular | Rectangular |
| Example 2-13 | A | 80 | A | A | A | Rectangular | Rectangular |
| Example 2-14 | A | 80 | A | A | A | Rectangular | Rectangular |
| Example 2-15 | A | 80 | A | A | A | Rectangular | Rectangular |
| Example 2-16 | A | 70 | A | A | A | Rectangular | Rectangular |
| Example 2-17 | A | 70 | A | A | A | Rectangular | Rectangular |
| Comparative Example 2-1 | A | 150 | A | B | C | Reverse Tapered | Tapered |
| Comparative Example 2-2 | A | 140 | A | C | B | Reverse Tapered | Tapered |
| Comparative Example 2-3 | A | 140 | A | B | B | Reverse Tapered | Tapered |

In Table 3, the numbers 1 to 9 in the "Specified" column in the "Compound" column represent Specified Compound 1 to Specified Compound 9 and the numbers 1 to 3 in the "Comparative" column represent Comparative Compound 1 to Comparative Compound 3. Sensitizing agents A1 to A3 and Co-Sensitizing agents F1 to F3 and LD-5 shown in Table 3 are the following compounds:

A1: 4,4-Bisdiethylaminobenzophenone

A2: Diethylthioxanthone

A3 :

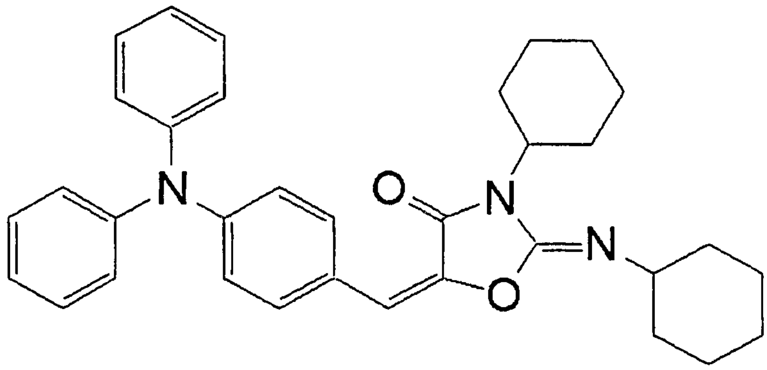

F1: 2-Mercaptobenzimidazole

F2: 2-Mercaptobenzothiazole

F3: N-Phenyl-2-mercaptobenzimidazole

LD-5: 2,2'-Bis(o-chlorophenyl)-4,4',5,5'-tetraphenylbiimidazole

The results shown in Table 3 indicate that the colored photopolymerizable composition of each Example containing the specified oxime compound (each of Compounds 1 to 9) has excellent storage stability (stability over time). Moreover, it is also shown that these colored photopolymerizable compositions have high exposure sensitivity and high developability when being used for the formation of colored patterns of a color filter, that the resulting colored patterns exhibit no coloration by heat aging, and that the colored patterns are excellent in adhesion to a substrate, cross-sectional pattern shape and post-heating cross-sectional pattern shape.

*Example 3-1*

*1. Preparation of Resist Liquid*

The components for the composition described below were mixed and dissolved to form a resist liquid.

*Composition of Resist Liquid*

- Propylene glycol monomethyl ether acetate (PGMEA): 19.20 parts by mass
- Ethyl lactate: 36.67 parts by mass
- Resin: 30.51 parts by mass
  [a 40% PGMEA solution of a benzyl methacrylate/methacrylic acid/2-hydroxyethyl methacrylate copolymer (60/22/18 in molar ratio)]
- Dipentaerythritol hexaacrylate (polymerizable compound): 12.20 parts by mass
- Polymerization inhibitor (p-methoxyphenol): 0.0061 parts by mass
- Fluorosurfactant: 0.83 parts by mass
  (F-475, manufactured by Dainippon Ink & Chemicals, Inc.)
- Photopolymerization initiator: 0.586 parts by mass
  [TAZ-107 (a trihalomethyltriazine-based photopolymerization initiator), manufactured by Midori Kagaku Co., Ltd.]

*2. Preparation of Silicon Wafer Substrate having Undercoat Layer*

A 6 inch silicon wafer was heated in an oven at 200°C for 30 minutes. The resist liquid was applied to the silicon wafer so as to provide a dry thickness of 2 μm and then heated and dried in an oven at 220°C for 1 hour to form an undercoat layer. As a result, a silicon wafer substrate having an undercoat layer was obtained.

*3. Preparation of Colored Photopolymerizable Composition B-1*

The compounds for the composition B-1 described below were mixed and dissolved to form a colorant (dye)-containing colored photopolymerizable composition B-1.

*Composition B-1*

- Cyclohexanone: 80 parts by mass
- Colorant C.I. Acid Blue 108: 7.5 parts by mass
- Colorant C.I. Solvent Yellow 162: 2.5 parts by mass
- Radical-polymerizable monomer (polymerizable compound): 7.0 parts by mass
  [a mixture of pentaerythritol triacrylate and dipentaerythritol hexaacrylate (3:7)]
- Specified Compound 1 (specified oxime compound): 2.5 parts by mass
- Glycerol propoxylate: 0.5 parts by mass
  [number average molecular weight Mn: 1,500, molar absorption coefficient [ε] = 0]

*4. Evaluation of Storage Stability of Colored Photopolymerizable Composition*

*B-1 (Coating Liquid)*

After the colored photopolymerizable composition B-1 was stored at room temperature for one month, the degree of precipitation of matters was visually evaluated according to the following criteria. The results are shown in Table 4.

*Criteria*

A: No precipitation was observed.
B: Precipitation was slightly observed.
C: Precipitation was observed.

*5. Preparation of Color Filter by Using Colored Photopolymerizable*

*Composition B-1 and Evaluation Thereof*

The colored photopolymerizable composition B-1 prepared in the section 3. was applied to the undercoat layer of the silicon wafer substrate having an undercoat layer obtained in the section 2. so that a photocurable coating film was formed. The coating film was then heated (pre-baked) with a hot plate at 100°C for 120 seconds so as to provide a dry thickness of 0.9 μm.

The film was then exposed to light with a wavelength of 365 nm through a mask having a 2 μm square island pattern at exposure amounts in the range of 10 to 1,600 mJ/$cm^2$ using an i-line stepper exposure system (trade name: FPA-3000i5+, manufactured by Cannon Inc.).

The silicon wafer substrate having the irradiated coating film was then mounted on a horizontal rotary table of a spin-shower developing machine (trade name: Model DW-30, manufactured by Chemitronics Co., Ltd.) and subjected to a paddle development process at 23°C for 60 seconds with CD-2000 (manufactured by Fuji Film Electronics Materials Co., Ltd.) so that a colored pattern was formed on the silicon wafer substrate).

The silicon wafer substrate having the colored pattern was fixed on the horizontal rotary table by vacuum chucking. The colored pattern was rinsed with a shower of pure water supplied from above the rotation center by using a spray nozzle, while the silicon wafer substrate was rotated at a rotation number of 50 rpm by means of a rotator, and

then the colored pattern was spray-dried.

Thus, a color filter having the substrate and the colored pattern formed thereon was obtained.

*Exposure Sensitivity and Size of Pattern*

The minimum exposure amount at which the post-development thickness of the region that was irradiated with light in the exposure step became 95% or more of the thickness (100%) of the film before the exposure was evaluated as the exposure sensitivity. A smaller exposure sensitivity indicates a higher sensitivity.

In this process, the size of the colored pattern was measured using a length measuring SEM (trade name: S-9260A, manufactured by Hitachi High-Technologies Corporation). A pattern size that is closer to 2 μm indicates sufficient curing, and higher sensitivity.

The results are shown in Table 4.

*Developability, Coloration on Heating, Adhesion to Substrate, Cross-Sectional Shape of Pattern, and Post-Heating Cross-Sectional Shape of Pattern*

Developability, coloration on heating, adhesion to the substrate, the cross-sectional shape of the pattern, and the post-heating cross-sectional shape of the pattern were evaluated according to the methods and the criteria used in Example 2-1. The results are shown in Table 4.

*Examples 3-2 to 3-17 and Comparative Examples 3-1 to 3-3*

Colored polymerizable compositions B-2 to B-17 and B'-1 to B'-3 and color filters were prepared in the same manner as in Example 3-1, except that each compound in the amount shown in Table 4 below were used in place of 2.5 parts by mass of Specified Compound 1 (the specified oxime compound) of the composition B-1 used in the preparation of the colored polymerizable composition B-1 and that a sensitizing agent and a co-sensitizing agent of the type and in the amount shown in Table 4 were further added in Examples 3-10 to 3-17. Evaluations were also performed in the same way as in Example 3-1. The results were shown in Table 4.

Table 4

| | Colored Polymerizable Composition | Compound | | | Sensitizing Agent | | Co-sensitizing Agent | |
|---|---|---|---|---|---|---|---|---|
| | | Specified | Comparative | Content (parts by mass) | Kind | Content (parts by mass) | Kind | Content (parts by mass) |
| Example 3-1 | B-1 | 1 | - | 2.5 | - | - | - | - |
| Example 3-2 | B-2 | 2 | - | 2.5 | - | - | - | - |
| Example 3-3 | B-3 | 3 | - | 2.5 | - | - | - | - |
| Example 3-4 | B-4 | 4 | - | 2.5 | - | - | - | - |
| Example 3-5 | B-5 | 5 | - | 2.5 | - | - | - | - |
| Example 3-6 | B-6 | 6 | - | 2.5 | - | - | - | - |
| Example 3-7 | B-7 | 7 | - | 2.5 | - | - | - | - |
| Example 3-8 | B-8 | 8 | - | 2.5 | - | - | - | - |
| Example 3-9 | B-9 | 9 | - | 2.5 | - | - | - | - |
| Example 3-10 | B-10 | 2 | - | 2 | A1 | 0.5 | - | - |
| Example 3-11 | B-11 | 2 | - | 2 | A2 | 0.5 | - | - |
| Example 3-12 | B-12 | 2 | - | 2 | A3 | 0.5 | - | - |
| Example 3-13 | B-13 | 2 | - | 2 | - | - | F1 | 0.5 |
| Example 3-14 | B-14 | 2 | - | 1.5 | A2 | 0.5 | F1 | 0.5 |
| Example 3-15 | B-15 | 2 | - | 1.5 | A2 | 0.5 | F2 | 0.5 |
| Example 3-16 | B-16 | 2 | - | 1.5 | A2 | 0.5 | F3 | 0.5 |
| Example 3-17 | B-17 | 2 | - | 1.5 | A2 | 0.5 | F3<br>LD-5 | 0.5<br>0.5 |
| Comparative Example 3-1 | B'-1 | - | 1 | 2.5 | - | - | - | - |
| Comparative Example 3-2 | B'-2 | - | 2 | 2.5 | - | - | - | - |
| Comparative Example 3-3 | B'-3 | - | 3 | 2.5 | - | - | - | - |

Table 4 (continued)

| | Storage Stability | Exposure Sensitivity (mJ/cm$^2$) | Size of Pattern (μm) | Developability | Color Change by Forced Heating | Adhesion to Substrate | Cross-Sectional Shape of Pattern | Post-Heating Cross-Sectional Shape of Pattern |
|---|---|---|---|---|---|---|---|---|
| Example 3-1 | A | 1000 | 1.96 | A | A | A | Rectangular | Rectangular |
| Example 3-2 | A | 900 | 1.97 | A | A | | Rectangular | Rectangular |
| Example 3-3 | A | 900 | 1.97 | A | A | A | Rectangular | Rectangular |
| Example 3-4 | A | 900 | 1.97 | A | A | | Rectangular | Rectangular |
| Example 3-5 | A | 900 | 1.97 | A | A | A | Rectangular | Rectangular |
| Example 3-6 | A | 900 | 1.97 | A | A | | Rectangular | Rectangular |
| Example 3-7 | A | 1200 | 1.94 | A | A | A | Rectangular | Rectangular |
| Example 3-8 | A | 900 | 1.97 | A | A | | Rectangular | Rectangular |
| Example 3-9 | A | 900 | 1.97 | A | A | A | Rectangular | Rectangular |
| Example 3-10 | A | 850 | 1.97 | A | A | | Rectangular | Rectangular |
| Example 3-11 | A | 850 | 1.97 | A | A | A | Rectangular | Rectangular |
| Example 3-12 | A | 800 | 1.98 | A | A | | Rectangular | Rectangular |
| Example 3-13 | A | 850 | 1.98 | A | A | A | Rectangular | Rectangular |
| Example 3-14 | A | 800 | 1.98 | A | A | | Rectangular | Rectangular |
| Example 3-15 | A | 800 | 1.98 | A | A | A | Rectangular | Rectangular |
| Example 3-16 | A | 750 | 1.98 | A | A | | Rectangular | Rectangular |
| Example 3-17 | A | 750 | 1.98 | A | A | A | Rectangular | Rectangular |
| Comparative Example 3-1 | A | 1400 | 1.92 | A | B | C | Reverse Tapered | Tapered |
| Comparative Example 3-2 | A | 1300 | 1.92 | A | C | C | Reverse Tapered | Tapered |
| Comparative Example 3-3 | A | 1300 | 1.92 | A | B | B | Reverse Tapered | Tapered |

In Table 4, the numbers 1 to 9 in the "Specified" column in the "Compound" column represent Specified Compound 1 to Specified Compound 9 and the numbers 1 to 3 in the "Comparative" column represent Comparative Compound 1 to Comparative Compound 3. Sensitizing agents A1 to A3 and Co-Sensitizing agents F1 to F3 and LD-5 shown in Table 4 are the compounds described above.

*Example 3-18*

The compounds for the composition C-1 described below were mixed and dissolved to form a colorant (pigment)-containing colored polymerizable composition C-1.

*Composition C-1*

- Ethyl 3-ethoxypropionate [solvent]: 17.9 parts by mass
- Dispersion liquid of colorant C. I. Pigment Red 254: 26.7 parts by mass
  (Solid content: 15% by weight, pigment content in the solid: 60%)
- Dispersion liquid of colorant C. I. Pigment Yellow 139: 17.8 parts by mass
  (Solid content: 15% by weight, pigment content in the solid: 60%)
- Radical-polymerizable monomer (polymerizable compound): 3.5 parts by mass
  [a mixture of pentaerythritol triacrylate and dipentaerythritol hexaacrylate (3:7)]
- Specified Compound 1 (specified oxime compound): 0.5 parts by mass
- Benzyl methacrylate/methacrylic acid copolymer: 2.0 parts by mass
  (molar ratio = 70/30)

*Examples 3-19 to 3-34 and Comparative Examples 3-4 to 3-6*

Colored polymerizable compositions C-2 to C-17, C'-1 to C'-3 were prepared in the same manner as in Example 3-18, except that each compound in the amount shown in Table 5 below were used in place of 0.5 parts by mass of Specified Compound 1 (the specified oxime compound) of the composition C-1 used in the preparation of the colored polymerizable composition C-1 and that a sensitizing agent and a co-sensitizing agent of the type and in the amount shown in Table 5 were further added in Examples 3-27 to 3-34.

For the colored polymerizable compositions obtained, the same evaluation as that of Example 3-1 was performed. The results are shown in Table 5.

Table 5

| | Colored Polymerizable Composition | Compound | | | Sensitizing Agent | | Cosensitizing agent | |
|---|---|---|---|---|---|---|---|---|
| | | Specified | Comparative | Content (parts by mass) | Kind | Content (parts by mass) | Kind | Content (parts by mass) |
| Example 3-18 | C-1 | 1 | - | 0.5 | - | - | - | - |
| Example 3-19 | C-2 | 2 | - | 0.5 | - | - | - | - |
| Example 3-20 | C-3 | 3 | - | 0.5 | - | - | - | - |
| Example 3-21 | C-4 | 4 | - | 0.5 | - | - | - | - |
| Example 3-22 | C-5 | 5 | - | 0.5 | - | - | - | - |
| Example 3-23 | C-6 | 6 | - | 0.5 | - | - | - | - |
| Example 3-24 | C-7 | 7 | - | 0.5 | - | - | - | - |
| Example 3-25 | C-8 | 8 | - | 0.5 | - | - | - | - |
| Example 3-26 | C-9 | 9 | - | 0.5 | - | - | - | - |
| Example 3-27 | C-10 | 2 | - | 0.4 | A1 | 0.1 | - | - |
| Example 3-28 | C-11 | 2 | - | 0.4 | A2 | 0.1 | - | - |
| Example 3-29 | C-12 | 2 | - | 0.4 | A3 | 0.1 | - | - |
| Example 3-30 | C-13 | 2 | - | 0.4 | - | - | F1 | 0.1 |
| Example 3-31 | C-14 | 2 | - | 0.3 | A2 | 0.1 | F1 | 0.1 |
| Example 3-32 | C-15 | 2 | - | 0.3 | A2 | 0.1 | F2 | 0.1 |
| Example 3-33 | C-16 | 2 | - | 0.3 | A2 | 0.1 | F3 | 0.1 |
| Example 3-34 | C-17 | 2 | - | 0.3 | A2 | 0.1 | F3<br>LD-5 | 0.1<br>0.1 |
| Comparative Example 3-4 | C'-1 | - | 1 | 0.5 | - | - | - | - |
| Comparative Example 3-5 | C'-2 | - | 2 | 0.5 | - | - | - | - |
| Comparative Example 3-6 | C'-3 | - | 3 | 0.5 | - | - | - | - |

Table 5 (continued)

| | Storage Stability | Exposure Sensitivity (mJ/cm$^2$) | Size of Pattern (μm) | Developability | Color Change by Forced Heating | Adhesion to Substrate | Cross-Sectional Shape of Pattern | Post-Heating Cross-Sectional Shape of Pattern |
|---|---|---|---|---|---|---|---|---|
| Example 3-18 | A | 900 | 1.97 | A | A | A | Rectangular | Rectangular |
| Example 3-19 | A | 700 | 1.98 | A | A | A | Rectangular | Rectangular |
| Example 3-20 | A | 700 | 1.98 | A | A | A | Rectangular | Rectangular |
| Example 3-21 | A | 700 | 1.98 | A | A | A | Rectangular | Rectangular |
| Example 3-22 | A | 700 | 1.98 | A | A | A | Rectangular | Rectangular |
| Example 3-23 | A | 700 | 1.98 | A | A | A | Rectangular | Rectangular |
| Example 3-24 | A | 700 | 1.96 | A | A | A | Rectangular | Rectangular |
| Example 3-25 | A | 1000 | 1.98 | A | A | A | Rectangular | Rectangular |
| Example 3-26 | A | 800 | 1.98 | A | A | A | Rectangular | Rectangular |
| Example 3-27 | A | 650 | 1.98 | A | A | A | Rectangular | Rectangular |
| Example 3-28 | A | 650 | 1.98 | A | A | A | Rectangular | Rectangular |
| Example 3-29 | A | 650 | 1.98 | A | A | A | Rectangular | Rectangular |
| Example 3-30 | A | 650 | 1.98 | A | A | A | Rectangular | Rectangular |
| Example 3-31 | A | 650 | 1.98 | A | A | A | Rectangular | Rectangular |
| Example 3-32 | A | 650 | 1.98 | A | A | A | Rectangular | Rectangular |
| Example 3-33 | A | 600 | 1.98 | A | A | A | Rectangular | Rectangular |
| Example 3-34 | A | 600 | 1.98 | A | A | A | Rectangular | Rectangular |
| Comparative Example 3-4 | A | 1200 | 1.92 | A | B | C | Reverse Tapered | Tapered |
| Comparative Example 3-5 | A | 1100 | 1.92 | A | C | B | Reverse Tapered | Tapered |
| Comparative Example 3-6 | A | 1100 | 1.92 | A | A | B | Reverse Tapered | Tapered |

In Table 5, the numbers 1 to 9 in the "Specified" column in the "Compound" column represent Specified Compound 1 to Specified Compound 9 and the numbers 1 to 3 in the "Comparative" column represent Comparative Compound 1 to Comparative Compound 3.

Sensitizing agents A1 to A3 and Co-Sensitizing agents F1 to F3 and LD-5 shown in Table 5 are the compounds described above.

*Example 3-35*

The compounds for the composition D-1 described below were mixed and dissolved to form a colorant (pigment)-containing colored polymerizable composition D-1.

*Composition D-1*

- Ethyl 3-ethoxypropionate [solvent]: 17.9 parts by mass
- Dispersion liquid of colorant C. I. Pigment Red 254: 33.34 parts by mass
  (Solid content: 15% by weight, pigment content in the solid: 60%)
- Dispersion liquid of colorant C. I. Pigment Yellow 139: 22.23 parts by mass
  (Solid content: 15% by weight, pigment content in the solid: 60%)
- Radical-polymerizable monomer (polymerizable compound): 2.5 parts by mass
  [a mixture of pentaerythritol triacrylate and dipentaerythritol hexaacrylate (3:7)]
- Specified Compound 1 (specified oxime compound): 0.5 parts by mass
- Benzyl methacrylate/methacrylic acid copolymer: 2.0 parts by mass
  (molar ratio = 70/30)

*Examples 3-36 to 3-51 and Comparative Examples 3-7 to 3-9*

Colored polymerizable compositions D-2 to C-17, D'-1 to D'-3 were prepared in the same manner as in Example 3-35, except that each compound in the amount shown in Table 6 below were used in place of 0.5 parts by mass of Specified Compound 1 (the specified oxime compound) of the composition D-1 used in the preparation of the colored polymerizable composition D-1 and that a sensitizing agent and a co-sensitizing agent of the type and in the amount shown in Table 6 were further added in Examples 3-44 to 3-51.

For the colored polymerizable compositions obtained, the same evaluation as that of Example 3-1 was performed. The results are shown in Table 6.

Table 6

| | Colored Polymerizable Composition | Compound | | | Sensitizing Agent | | Co-sensitizing Agent | |
|---|---|---|---|---|---|---|---|---|
| | | Specified | Comparative | Content (parts by mass) | Kind | Content (parts by mass) | Kind | Content (parts by mass) |
| Example 3-35 | D-1 | 1 | - | 0.5 | - | - | - | - |
| Example 3-36 | D-2 | 2 | - | 0.5 | - | - | - | - |
| Example 3-37 | D-3 | 3 | - | 0.5 | - | - | - | - |
| Example 3-38 | D-4 | 4 | - | 0.5 | - | - | - | - |
| Example 3-39 | D-5 | 5 | - | 0.5 | - | - | - | - |
| Example 3-40 | D-6 | 6 | - | 0.5 | - | - | - | - |
| Example 3-41 | D-7 | 7 | - | 0.5 | - | - | - | - |
| Example 3-42 | D-8 | 8 | - | 0.5 | - | - | - | - |
| Example 3-43 | D-9 | 9 | - | 0.5 | - | - | - | - |
| Example 3-44 | D-10 | 2 | - | 0.4 | A1 | 0.1 | - | - |
| Example 3-45 | D-11 | 2 | - | 0.4 | A2 | 0.1 | - | - |
| Example 3-46 | D-12 | 2 | - | 0.4 | A3 | 0.1 | - | - |
| Example 3-47 | D-13 | 2 | - | 0.4 | - | - | F1 | 0.1 |
| Example 3-48 | D-14 | 2 | - | 0.3 | A2 | 0.1 | F1 | 0.1 |
| Example 3-49 | D-15 | 2 | - | 0.3 | A2 | 0.1 | F2 | 0.1 |
| Example 3-50 | D-16 | 2 | - | 0.3 | A2 | 0.1 | F3 | 0.1 |
| Example 3-51 | D-17 | 2 | - | 0.3 | A2 | 0.1 | F3<br>LD-5 | 0.1<br>0.1 |
| Comparative Example 3-7 | D'-1 | - | 1 | 0.5 | - | - | - | - |
| Comparative Example 3-8 | D'-2 | - | 2 | 0.5 | - | - | - | - |
| Comparative Example 3-9 | D'-3 | - | 3 | 0.5 | - | - | - | - |

Table 6 (continued)

| | Storage Stability | Exposure Sensitivity ($mJ/cm^2$) | Size of Pattern (μm) | Developability | Color Change by Forced Heating | Adhesion to Substrate | Cross-Sectional Shape of Pattern | Post-Heating Cross-Sectional Shape of Pattern |
|---|---|---|---|---|---|---|---|---|
| Example 3-35 | A | 1500 | 1.96 | A | A | A | Rectangular | Rectangular |
| Example 3-36 | A | 1400 | 1.96 | A | A | A | Rectangular | Rectangular |
| Example 3-37 | A | 1400 | 1.96 | A | A | A | Rectangular | Rectangular |
| Example 3-38 | A | 1400 | 1.96 | A | A | A | Rectangular | Rectangular |
| Example 3-39 | A | 1400 | 1.96 | A | A | A | Rectangular | Rectangular |
| Example 3-40 | A | 1400 | 1.96 | A | A | A | Rectangular | Rectangular |
| Example 3-41 | A | 1600 | 1.94 | A | A | A | Rectangular | Rectangular |
| Example 3-42 | A | 1400 | 1.96 | A | A | A | Rectangular | Rectangular |
| Example 3-43 | A | 1400 | 1.96 | A | A | A | Rectangular | Rectangular |
| Example 3-44 | A | 1300 | 1.96 | A | A | A | Rectangular | Rectangular |
| Example 3-45 | A | 1300 | 1.96 | A | A | A | Rectangular | Rectangular |
| Example 3-46 | A | 1300 | 1.96 | A | A | A | Rectangular | Rectangular |
| Example 3-47 | A | 1300 | 1.96 | A | A | A | Rectangular | Rectangular |
| Example 3-48 | A | 1300 | 1.96 | A | A | A | Rectangular | Rectangular |
| Example 3-49 | A | 1300 | 1.96 | A | A | A | Rectangular | Rectangular |
| Example 3-50 | A | 1200 | 1.96 | A | A | A | Rectangular | Rectangular |
| Example 3-51 | A | 1200 | 1.96 | A | A | A | Rectangular | Rectangular |
| Comparative Example 3-7 | A | 3000 | 1.92 | A | B | C | Reverse Tapered | Tapered |
| Comparative Example 3-8 | A | 2800 | 1.92 | A | C | B | Reverse Tapered | Tapered |
| Comparative Example 3-9 | A | 2800 | 1.92 | A | A | B | Reverse Tapered | Tapered |

In Table 6, the numbers 1 to 9 in the "Specified" column in the "Compound" column represent Specified Compound 1 to Specified Compound 9 and the numbers 1 to 3 in the "Comparative" column represent Comparative Compound 1 to Comparative Compound 3.

Sensitizing agents A1 to A3 and Co-Sensitizing agents F1 to F3 and LD-5 shown in Table 6 are the compounds described above.

The results of Tables 4 to 6 indicate that the colored polymerizable composition of each Example containing the specified oxime compound (each of Compounds 1 to 9) has excellent storage stability (stability over time). Moreover, it is also shown that these colored polymerizable compositions have high exposure sensitivity and high developability when being used for the formation of colored patterns of a color filter, that the resulting colored patterns exhibit no coloration by heat aging, and that the colored patterns are excellent in adhesion to a substrate, cross-sectional pattern shape and post-heating cross-sectional pattern shape.

Moreover, Table 6 clearly shows that the compositions have excellent exposure sensitivity even in the event that the pigment content is large.

*Examples 4-1 to 4-38 and Comparative Examples 4-1 to 4-12*

*Preparation of Black Polymerizable Composition*

*Preparation of Carbon Black Dispersion Liquid A*

A dispersion was obtained by subjecting the following composition 1 to high-viscosity dispersion process with a double roll. The dispersion had a viscosity of 70,000 mPa·S.

The composition 2 shown below was then added to the dispersion, and the mixture was stirred with a homogenizer at 3,000 rpm for 3 hours. The resulting mixture solution was subjected to a micro-dispersion process for 4 hours in a dispersing machine (trade name: DISPERMAT, manufactured by Getzmann) with 0.3 mm zirconia beads, so that a carbon black dispersion liquid A (hereinafter referred to as CB dispersion liquid A) was prepared. In this process, the mixture solution had a viscosity of 37 mPa·s.

*Composition 1*

- Carbon black (Pigment Black 7) with an average primary particle diameter of 15 nm: 23 parts by mass
- 45% solution of a benzyl methacrylate/methacrylic acid copolymer in propylene glycol monomethyl ether acetate: 22 parts by mass
  (BzMA/MAA = 70/30, Mw: 30,000)
- SOLSPERSE 5000 (manufactured by The Lubrizol Corporation): 1.2 parts by mass

*Composition 2*

- 45% solution of a benzyl methacrylate/methacrylic acid copolymer in propylene glycol monomethyl ether acetate: 22 parts by mass
  (BzMA/MAA = 70/30, Mw: 30,000)
- Propylene glycol monomethyl ether acetate: 200 parts by mass

*Preparation of Titanium Black Dispersion Liquid A*

The composition 3 shown below was subjected to a high-viscosity dispersion process with two rolls to form a dispersion liquid with a viscosity of 40,000 mPa·s.

Optionally, kneading with a kneader may be performed for 30 minutes before the high-viscosity dispersion process.

*Composition 3*

- Titanium Black 13M-C having an average primary particle diameter of 75 nm: 39 parts by mass
  (manufactured by Mitsubishi Materials) (Pigment Black 35)
- Solution of a benzyl (meth)acrylate/(meth)acrylic acid copolymer in propylene glycol monomethyl ether acetate: 8 parts by mass
  (BzWA/MAA = 70/30, Mw: 30,000, solid content: 40% by weight)
- SOLSPERSE 5000 (manufactured by The Lubrizol Corporation): 1 part by weight

The composition 4 shown below was then added to the resulting dispersion, and the mixture was stirred with a homogenizer at 3,000 rpm for 3 hours. The resulting mixture solution was subjected to a micro-dispersion process for 4 hours in a dispersing machine (trade name: DISPERMAT, manufactured by Getzmann) with 0.3 mm zirconia beads so that a black titanium oxide dispersion liquid A (hereinafter referred to as TB dispersion liquid A) was prepared.

In this process, the mixture solution had a viscosity of 7.0 mPa·s.

*Composition 4*

- Solution of a benzyl (meth)acrylate/(meth)acrylic acid copolymer in propylene glycol monomethyl ether acetate: 8 parts by mass
  (BzMA/MAA= 70/30, Mw: 30,000, solid content: 40% by weight)
- Propylene glycol monomethyl ether acetate: 200 parts by mass

*Preparation of Black Polymerizable Compositions E-1 to E-18, and E'-1 to E'-6*

The components of the following composition E-a were mixed with a stirring machine to prepare black polymerizable compositions E-1 to E-18 and E'-1 to E'-6.

*Composition E-a*

- Methacrylate/acrylic acid copolymer (alkali-soluble resin): 1.6 parts by mass
- Dipentaerythritol hexaacrylate: 2.3 parts by mass
- Ethoxylated pentaerythritol tetraacrylate: 0.8 parts by mass
- The CB dispersion liquid A or the TB dispersion liquid A: 24 parts by mass
- Propylene glycol monomethyl ether acetate: 10 parts by mass
- Ethyl 3-ethoxypropionate: 8 parts by mass
- Compound given in the following Table 7: Specified oxime compound or Comparative Compound Amount given in Table 7
- Co-sensitizing agent: F3 not added or 0.1 parts by mass

*Preparation of Black Polymerizable Compositions E-19 to E-38, and E'-7 to E'-12*

The components of the following composition E-b were mixed with a stirring machine to prepare black polymerizable compositions E-19 to E-38 and E'-7 to E'-12.

*Composition E-b*

- Dipentaerythritol hexaacrylate: 2.3 parts by mass
- The CB dispersion liquid A or the TB dispersion liquid A: 24 parts by mass
- Propylene glycol monomethyl ether acetate: 10 parts by mass
- Ethyl 3-ethoxypropionate: 8 parts by mass
- Compound given in the following Table 7: Specified oxime compound or Comparative Compound
- Co-sensitizing agent: F3 not added or 0.1 parts by mass

*Evaluation*

The following evaluations were performed using the black polymerizable compositions E-1 to E-38 and E'-1 to E'-12 obtained as described above. The results are summarized in Tables 7 and 8.

*Evaluation of Exposure Sensitivity*

The exposure sensitivity of the black polymerizable compositions E-1 to E-38 and E'-1 to E'-12 obtained as described above were measure by the following method and evaluated.

Each of the black photopolymerizable compositions E-1 to E-38 and E'-1 to E'-12 was used and uniformly applied to a silicon wafer by spin coating at a rotational speed that was controlled such that the thickness of the coating would be 1.0 μm after the coating was subjected to heat treatment on a hot plate at a surface temperature of 120°C for 120 seconds conducted after the coating.

The resulting 1.0 μm-thick coating film was exposed to light from an i-line stepper (trade name: FPA-3000iS+,

manufactured by Canon Inc.) at different exposure amounts in the range of 10 $mJ/cm^2$ to 5,100 $mJ/cm^2$ through a mask having a 10 nm L & S (line and space) pattern.

After the exposure, the coating film was subjected to a paddle development process at 23°C for 60 seconds with an aqueous solution of 0.3% tetramethylammonium hydroxide (TMAH). The film was then rinsed with pure water for 20 seconds by spin shower and further washed with pure water. Thereafter, the deposited droplets of water were removed with high speed air, and the substrate was naturally dried, so that a black patterned image was obtained.

Each of the resulting colored patterned images was evaluated with an optical microscope according to the criteria below.

The minimum exposure amount at which the post-development thickness of the region that was irradiated with light in the exposure step became 95% or more of the thickness (100%) of the film before the exposure was measured, and it was evaluated as an exposure sensitivity.

A smaller exposure sensitivity indicates a higher sensitivity.

*Evaluation of Storage Stability (Stability over Time)*

With respect to the storage stability (stability over time) of the black polymerizable compositions E-1 to E-38 and E'-1 to E'-12 obtained as described above, evaluation was performed by the following method.

That is, after the black photopolymerizable compositions E-1 to E-38 and E'-1 to E'-12 were stored at room temperature for one month, the degree of precipitation of matters was visually evaluated according to the following criteria.

*Criteria*

A: No precipitation was observed.
B: Precipitation was slightly observed.
C: Precipitation was observed.

*Evaluation of Developability*

With respect to the developability of the black polymerizable compositions E-1 to E-38 and E'-1 to E'-12 obtained as described above, evaluation was performed by the following method.

That is, the presence or absence of residues in the region (unexposed portion) which was not irradiated with light in the exposure step of the Sensitivity Evaluation above was observed, so as to evaluate the developability. The evaluation was preformed according to the following criteria.

*Criteria*

A: No residue was observed in the unexposed portion.
B: Residues were slightly observed in the unexposed portion, but the residue level was practically acceptable.
C: Residues were significantly observed in the unexposed portion.

Table 7

| | Black Polymerizable Composition | Composition | Dispersion Liquid | Compound | | | Co-Sensitizing Agent | | Exposure Sensitivity | Storage Stability | Developability |
|---|---|---|---|---|---|---|---|---|---|---|---|
| | | | | Specified | Comparative | Content (parts by mass) | Kind | Content (parts by mass) | ($mJ/cm^2$) | | |
| Example 4-1 | E-1 | E-a | CB Dispersion Liquid A | 1 | - | 0.8 | - | - | 200 | A | A |
| Example 4-2 | E-2 | E-a | CB Dispersion Liquid A | 2 | - | 0.8 | - | - | 100 | A | A |
| Example 4-3 | E-3 | E-a | CB Dispersion Liquid A | 3 | - | 0.8 | - | - | 100 | A | A |
| Example 4-4 | E-4 | E-a | CB Dispersion liquid A | 4 | - | 0.8 | - | - | 100 | A | A |
| Example 4-5 | E-5 | E-a | CB Dispersion Liquid A | 5 | - | 0.8 | - | - | 100 | A | A |
| Example 4-6 | E-6 | E-a | CB Dispersion Liquid A | 6 | - | 0.8 | - | - | 100 | A | A |
| Examples 4-7 | E-7 | E-a | CB Dispersion Liquid A | 7 | - | 0.8 | - | - | 100 | A | A |
| Example 4-8 | E-8 | E-a | CB Dispersion Liquid A | 8 | - | 0.7 | - | - | 300 | A | A |
| Example 4-9 | E-9 | E-a | CB Dispersion Liquid A | 9 | - | 0.7 | - | - | 200 | A | A |
| Example 4-10 | E-10 | E-a | CB Dispersion Liquid A | 1 | - | 0.1 | F3 | 0.1- | 80 | A | A |
| Example 4-11 | E-11 | E-a | TB Dispersion Liquid A | 1 | - | 0.8 | - | - | 200 | A | A |
| Example 4-12 | E-12 | E-a | TB Dispersion Liquid A | 2 | - | 0.8 | - | - | 100 | A | A |
| Example 4-13 | E-13 | E-a | TB Dispersion Liquid A | 3 | - | 0.8 | - | - | 200 | A | A |

(continued)

| | Black Polymerizable Composition | Composition | Dispersion Liquid | Compound | | | Co-Sensitizing Agent | | Exposure Sensitivity | Storage Stability | Developability |
|---|---|---|---|---|---|---|---|---|---|---|---|
| | | | | Specified | Comparative | Content (parts by mass) | Kind | Content (parts by mass) | (mJ/cm$^2$) | | |
| Example 4-14 | E-14 | E-a | TB Dispersion Liquid A | 4 | - | 0.8 | - | - | 200 | A | A |
| Example 4-15 | E-15 | E-a | TB Dispersion Liquid A | 7 | - | 0.8 | - | - | 400 | A | A |
| Example 4-16 | E-16 | E-a | TB Dispersion Liquid A | 8 | - | 0.8 | - | - | 200 | A | A |
| Example 4-17 | E-17 | E-a | TB Dispersion Liquid A | 9 | - | 0.8 | - | - | 200 | A | A |
| Example 4-18 | E-18 | E-a | TB Dispersion Liquid A | 1 | - | 0.7 | F3 | 0.1 | 150 | A | A |
| Comparative Example 4-1 | E’-1 | E-a | CB Dispersion Liquid A | - | 1 | 0.8 | - | - | 600 | A | A |
| Comparative Example 4-2 | E’-2 | E-a | CB Dispersion Liquid A | - | 2 | 0.8 | - | - | 500 | A | A |
| Comparative Example 4-3 | E’-3 | E-a | CB Dispersion Liquid A | - | 3 | 0.8 | - | - | 500 | A | A |
| Comparative Example 4-4 | E’-4 | E-a | TB Dispersion Liquid A | - | 1 | 0.8 | - | - | 800 | A | A |
| Comparative Example 4-5 | E’-5 | E-a | TB Dispersion Liquid A | - | 2 | 0.8 | - | - | 700 | A | A |
| Comparative Example 4-6 | E’-6 | E-a | TB Dispersion Liquid A | - | 3 | 0.8 | - | - | 700 | A | A |

Table 8

| | Black Polymerizable Composition | Composition | Dispersion Liquid | Compound | | | Co-Sensitizing Agent | | Sensitivity (mJ/cm$^2$) | Storage Stability | Developability |
|---|---|---|---|---|---|---|---|---|---|---|---|
| | | | | Specified | Comparative | Content (parts by mass) | Kind | Content (parts by mass) | | | |
| Example 4-19 | E-19 | E-b | CB Dispersion Liquid A | 1 | - | 0.8 | - | - | 500 | A | A |
| Example 4-20 | E-20 | E-b | CB Dispersion Liquid A | 2 | - | 0.8 | - | - | 400 | A | A |
| Example 4-21 | E-21 | E-b | CB Dispersion Liquid A | 3 | - | 0.8 | - | - | 400 | A | A |
| Example 4-22 | E-22 | E-b | CB Dispersion Liquid A | 4 | - | 0.8 | - | - | 400 | A | A |
| Example 4-23 | E-23 | E-b | CB Dispersion Liquid A | 5 | - | 0.8 | - | - | 400 | A | A |
| Example 4-24 | E-24 | E-b | CB Dispersion Liquid A | 6 | - | 0.8 | - | - | 400 | A | A |
| Example 4-25 | E-25 | E-b | CB Dispersion Liquid A | 7 | - | 0.8 | - | - | 700 | A | A |
| Example 4-26 | E-26 | E-b | CB Dispersion Liquid A | 8 | - | 0.8 | - | - | 400 | A | A |
| Example 4-27 | E-27 | E-b | CB Dispersion Liquid A | 9 | - | 0.8 | - | - | 400 | A | A |
| Example 4-28 | E-28 | E-b | CB Dispersion Liquid A | 2 | - | 0.7 | F3 | 0.1- | 300 | A | A |
| Example 4-29 | E-29 | E-b | TB Dispersion Liquid A | 1 | - | 0.8 | - | - | 300 | A | A |
| Example 4-30 | E-30 | E-b | TB Dispersion Liquid A | 2 | - | 0.8 | - | - | 200 | A | A |
| Example 4-31 | E-31 | E-b | TB Dispersion Liquid A | 3 | - | 0.8 | - | - | 200 | A | A |

(continued)

| | Black Polymerizable Composition | Composition | Dispersion Liquid | Compound | | | Co-Sensitizing Agent | | Sensitivity (mJ/cm$^2$) | Storage Stability | Developability |
|---|---|---|---|---|---|---|---|---|---|---|---|
| | | | | Specified | Comparative | Content (parts by mass) | Kind | Content (parts by mass) | | | |
| Example 4-32 | E-32 | E-b | TB Dispersion Liquid A | 4 | - | 0.8 | - | - | 200 | A | A |
| Example 4-33 | E-33 | E-b | TB Dispersion Liquid A | 5 | | 0.8 | | | 200 | A | |
| Example 4-34 | E-34 | E-b | TB Dispersion Liquid A | 6 | | 0.8 | | | 200 | A | |
| Example 4-35 | E-35 | E-b | TB Dispersion Liquid A | 7 | - | 0.8 | - | - | 400 | A | A |
| Example 4-36 | E-36 | E-b | TB Dispersion Liquid A | 8 | - | 0.8 | - | - | 200 | A | A |
| Example 4-37 | E-37 | E-b | TB Dispersion Liquid A | 9 | - | 0.8 | - | - | 200 | A | A |
| Example 4-38 | E-38 | E-b | TB Dispersion Liquid A | 2 | - | 0.7 | F3 | 0.1 | 150 | A | A |
| Comparative Example 4-7 | E'-7 | E-b | CB Dispersion Liquid A | - | 1 | 0.8 | - | - | 900 | A | B |
| Comparative Example 4-8 | E'-8 | E-b | CB Dispersion Liquid A | - | 2 | 0.8 | - | - | 800 | A | B |
| Comparative Example 4-9 | E'-9 | E-b | CB Dispersion Liquid A | - | 3 | 0.8 | - | - | 800 | A | A |
| Comparative Example 4-10 | E'-10 | E-b | TB Dispersion Liquid A | - | 1 | 0.8 | - | - | 600 | A | B |
| Comparative Example 4-11 | E'-11 | E-b | TB Dispersion Liquid A | - | 2 | 0.8 | - | - | 600 | A | B |
| Comparative Example 4-12 | E'-12 | E-b | TB Dispersion Liquid A | - | 3 | 0.8 | - | - | 600 | A | A |

In Tables 7 and 8, the numbers 1 to 9 in the "Specified" column in the "Compound" column represent Specified Compound 1 to Specified Compound 9 and the numbers 1 to 3 in the "Comparative" column represent Comparative Compound 1 to Comparative Compound 3.

Tables 7 and 8 clearly show that the black polymerizable composition of each Example containing the specified oxime compound has excellent storage stability (stability over time). Moreover, these black polymerizable compositions are higher in exposure sensitivity and superior in developability of unexposed portions than Comparative Examples. Therefore, it is shown that the black polymerizable compositions can form satisfactory black patterns (colored patterns) even at a small exposure amount.

*Example 5*

*Preparation of Full-Color Color Filter*

Using the black image pattern prepared in Example 4-1 as a black matrix, a green (G) colored pattern of 1.6 μm x 1.6 μm was formed on the black matrix in the same manner as in Example 3-1 by using the colored polymerizable composition A-1. Furthermore, with respect to the colored photopolymerizable composition A-1, a blue (B) colored polymerizable composition and a red (R) colored polymerizable composition were prepared in the same manner except for changing only the pigment (the 30/70 (weight ratio) mixture of C. I. Pigment Green 36 and C. I. Pigment Yellow 219) to a blue pigment (a 30/70 (weight ratio) mixture of C. I. Pigment Blue 15:6 and C. I. Pigment Violet 23) and a red pigment (C. I. pigment Red 254), respectively.

First of all, a color filter for solid-state imaging devices was prepared on the substrate by successively forming a blue (B) pattern and a red (R) pattern both in a size of 1.6 μm × 1.6 μm in the same manner as that performed using the green (G) colored polymerizable composition A-1.

With respect to the resulting full-color color filter, a black image pattern, an R colored pattern, a G colored pattern, and a B colored pattern were evaluated for their cross-sectional shapes and adhesion to the substrate. It was found that all the patterns were rectangular, that none of the patterns was suffered from pattern loss, and therefore that they were excellent in adhesion to the support.

*Example 6*

*Preparation of Solid state Imaging Device*

When the full-color color filter obtained in Example 5 was mounted into a solid-state imaging device, the solid-state imaging device was confirmed to have high resolution and excellent color separation.

*Examples 7-1 to 7-15 and Comparative Examples 7-1 to 7-4*

*Preparation of Support*

The surface of a 0.30 mm-thick aluminum sheet (1S material) was grained using a nylon brush No. 8 and a 800-mesh pumice stone-water suspension, and then washed thoroughly with water. This sheet was etched by 60-second immersion in a 10% aqueous solution of sodium hydroxide kept at 70°C, washed with running water, neutralized and cleaned with 20% nitric acid, and further washed with water. Furthermore, the aluminum sheet was immersed in a 1% aqueous solution of nitric acid, and subjected to electrolytic surface roughening treatment using sine-wave alternating waveform current under the condition that VA was 12.7 V and the anodic electricity was 300 coulomb/$dm^2$. The surface roughness of the sheet was found to be 0 to 45 μm (expressed in Ra). Subsequently, the aluminum sheet was immersed in a 30% aqueous solution of $H_2SO_4$ at 55°C for 2 minutes for desmutting. The aluminum sheet was then anodized in a 20% aqueous solution of $H_2SO_4$ at 33°C for 50 seconds at a current density of 5 A/$dm^2$ with a cathode placed on the grained surface, so that an anodized layer having a thickness of 2.7 g/$m^2$ was formed.

Thus, a support A-1 for planographic printing plate precursors was obtained.

*Formation of Photosensitive Layer*

A photosensitive layer coating liquid with the composition described below was applied to the resulting support so as to provide a dry coating amount of 1.4 g/$m^2$ and was dried at 95°C, so that a photosensitive layer was formed.

*Composition of Coating Liquid for Photosensitive Layer*

- Addition-polymerizable compound (M, N or O given in Table 9): 0.80 parts by mass
- Binder polymer (B1, B2 or B3 given in Table 9): 0.90 parts by mass
- Sensitizing Agent (A1, A2 or A3 given in Table 9): not added or 0.10 parts by mass
- Compound given in the following Table 9: Specified oxime compound, Comparative Compound, or LD-5: 0.05 parts by mass
- Co-sensitizing agent (F2 or F3 given in Table 9): not added or 0.25 parts by mass
- Fluorosurfactant: 0.02 parts by mass
  (MEGAFAC F-177, manufactured by Dainippon Ink & Chemicals, Inc.)
- Thermalpolymerization initiator: 0.03 parts by mass
  (N-nitrosohydroxylamine aluminum salt)
- Dispersion of ε-type copper phthalocyanine: 0.2 parts by mass
- Methyl ethyl ketone: 16.0 g
- Propylene glycol monomethyl ether: 16.0 parts by mass

*Formation of Protective Layer*

A 3% by weight aqueous solution of polyvinyl alcohol (saponification degree: 98 mol %; polymerization degree: 550) was coated on each of the resulting photosensitive layers so that a dry coating amount would become 2 g/$m^2$, followed by drying at 100°C for 2 minutes to form a protective layer.

By the method described above, the planographic printing plate precursors of Examples and the planographic printing plate precursors of Comparative Examples were obtained.

*Plate Making*

Planographic printing plate precursors were subjected to the following exposure and development.

*Exposure to Light*

Each of planographic printing plate precursors was scan-exposed to a solid image and a 1 to 99% halftone dot image (1% intervals) under the conditions of 50 μ J/$cm^2$ in exposure amount, 4,000 dpi and 175 lines/inch using a violet LD of 405 nm in wavelength (VIOLET BOXER, manufactured by FFEI).

*Development*

An automatic processor (trade name: LP-850P2, manufactured by FUJIFILM Corporation) into which Developer 1 shown below and a finishing gum liquid (trade name: FP-2W, manufactured by FUJIFILM Corporation) was loaded was used to perform a standard process. Preheating was performed under the conditions where the plate surface reached 100°C. The developer temperature was 30°C, and the time of immersion in the developer was about 15 seconds.

Liquid developer 1 had the following formulation and had a pH of 11.5 at 25°C and an electric conductivity of 5 mS/cm.

*Formulation of Liquid Developer 1*

- Potassium hydroxide: 0.15 g
- Polyoxyethylene phenyl ether (n=13): 5.0 g
- CHELEST 400 (chelating agent): 0.1 g
- Water: 94.75 g

*Evaluation*

The sensitivity and the storage stability of the planographic printing plate precursors were evaluated by the methods described below. The results are summarized in Table 9.

*1. Evaluation of Sensitivity*

Each of the planographic printing plate precursors was exposed under the conditions provided above and, immediately thereafter, was developed under the conditions provided above to form an image. The area (%) of 50% halftone dot was measured by using a halftone dot area analyzer (Gretag-Macbeth). The larger the number, the higher the sensitivity.

*2. Image Area Printing durability Test*

Printing was conducted using an "R201" manufactured by Roland Co. as a printing machine, a GEOS-G (N) manufactured by Dainippon Ink & Chemicals, Inc. as ink, and a planographic printing plate precursor. While continuing the printing, the solid image area of the printed material was observed and the printing durability was examined by the number of prints when the image started to become thin. The larger the numeral, the better the printing durability.

*3. Evaluation of Amount of Change by Forced Aging (Storage Stability)*

The measurement of the halftone dot area was conducted in the same manner as in the evaluation of sensitivity except for using the planographic printing plate precursors after being left for 4 days at 60°C in a state of being tightly closed with aluminum kraft paper together with an interleaf. Next, the difference between the halftone dot area measured after being left for 4 days at 60°C and the halftone dot area measured before failing to be left for 4 days at 60°C was calculated to determine halftone dot variation (Δ %) during a forced lapse of time. A smaller absolute numerical value shows that the sample is less affected by the forced aging, that is, that the sample has a higher storage stability.

Table 9

| | Support | Photosensitive Layer | | | | | | Sensitivity (%) | Amount of Change by Forced Aging (%) | Image Area Printing durability Test |
|---|---|---|---|---|---|---|---|---|---|---|
| | | Specified Compound or Comparative Compound | Addition-Polymerizable Compound | Binder Polymer | Sensitizing Agent | Co-Sensitizing Agent | Coating Amount ($g/m^2$) | (50% Halftone Dot Area) | | |
| Example 7-1 | A-1 | Specified Compound 1 | M | B1 | - | - | 1.4 | 56 | 2 | 70000 |
| Example 7-2 | A-1 | Specified Compound 2 | M | B1 | - | - | 1.4 | 56 | 2 | 70000 |
| Example 7-3 | A-1 | Specified Compound 3 | M | B1 | - | - | 1.4 | 56 | 2 | 60000 |
| Example 7-4 | A-1 | Specified Compound 4 | M | B1 | - | - | 1.4 | 55 | 2 | 60000 |
| Example 7-5 | A-1 | Specified Compound 5 | M | B1 | - | - | 1.4 | 56 | 2 | 70000 |
| Example 7-6 | A-1 | Specified Compound 6 | M | B1 | - | - | 1.4 | 56 | 2 | 70000 |
| Example 7-7 | A-1 | Specified Compound 7 | M | B1 | - | - | 1.4 | 56 | 2 | 70000 |
| Example 7-8 | A-1 | Specified Compound 8 | M | B1 | - | - | 1.4 | 54 | 2 | 55000 |
| Example 7-9 | A-1 | Specified Compound 9 | M | B1 | - | - | 1.4 | 55 | 2 | 60000 |
| Example 7-10 | A-1 | Specified Compound 2 | M | B1 | A1 | F2 | 1.4 | 55 | 2 | 70000 |
| Example 7-11 | A-1 | Specified Compound 2 | N | B2 | A1 | F2 | 1.4 | 55 | 2 | 70000 |
| Example 7-12 | A-1 | Specified Compound 2 | O | B3 | A1 | F2 | 1.4 | 55 | 2 | 100000 |

(continued)

| | Support | Photosensitive Layer | | | | | | Sensitivity (%) | Amount of Change by Forced Aging (%) | Image Area Printing durability Test |
|---|---|---|---|---|---|---|---|---|---|---|
| | | Specified Compound or Comparative Compound | Addition-Polymerizable Compound | Binder Polymer | Sensitizing Agent | Co-Sensitizing Agent | Coating Amount ($g/m^2$) | (50% Halftone Dot Area) | | |
| Example 7-13 | A-1 | Specified Compound 2 | O | B3 | A2 | F2 | 1.4 | 57 | 2 | 100000 |
| Example 7-14 | A-1 | Specified Compound 2 | O | B3 | A3 | F3 | 1.4 | 58 | 2 | 100000 |
| Example 7-15 | A-1 | Specified Compound 2 | O | B3 | A3 | F3 | 1.4 | 60 | 2 | 100000 |
| Comparative Example 7-1 | A-1 | Comparative Compound 1 | M | B1 | - | - | 1.4 | 50 | 2 | 40000 |
| Comparative Example 7-2 | A-1 | Comparative Compound 2 | M | B1 | - | - | 1.4 | 51 | 2 | 40000 |
| Comparative Example 7-3 | A-1 | Comparative Compound 3 | M | B1 | - | - | 1.4 | 51 | 2 | 40000 |
| Comparative Example 7-4 | A-1 | LD-5 | M | B1 | A1 | F2 | 1.4 | 53 | 2 | 50000 |

In Table 9, particulars of M, N, and O in the "Addition-Polymerizable Compound" column and particulars of B1, B2 and B3 in the "Binder Polymer" column are as follows. The B3 is a mixture (mixing molar ratio = 50/50) of an MDI/HMDI copolymer [molar ratio = 80/20] and a copolymer [molar ratio = 52/22/26] of DMPA, PPG (m = 3) and TEG having the structures given below.

M 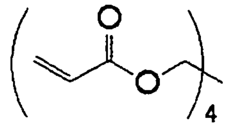

N 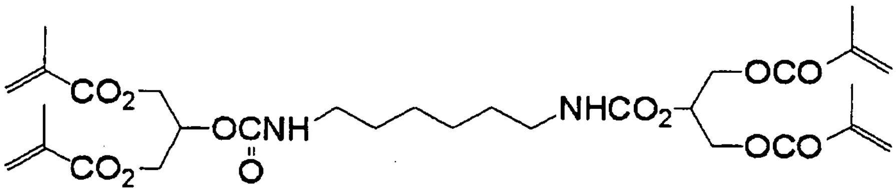

O 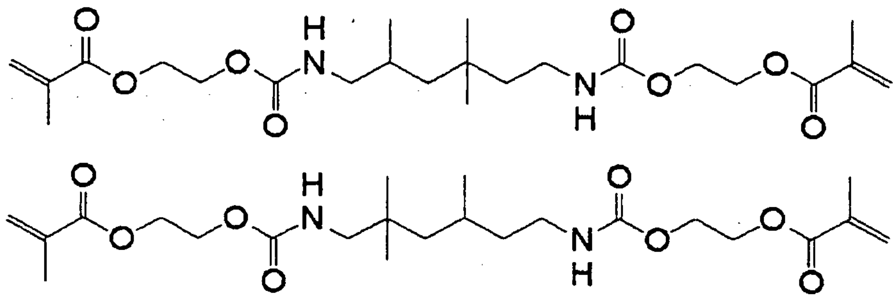

O: Mixture of these isomers.

B1 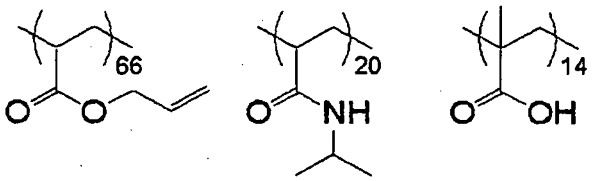

B2 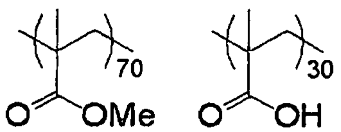

B3 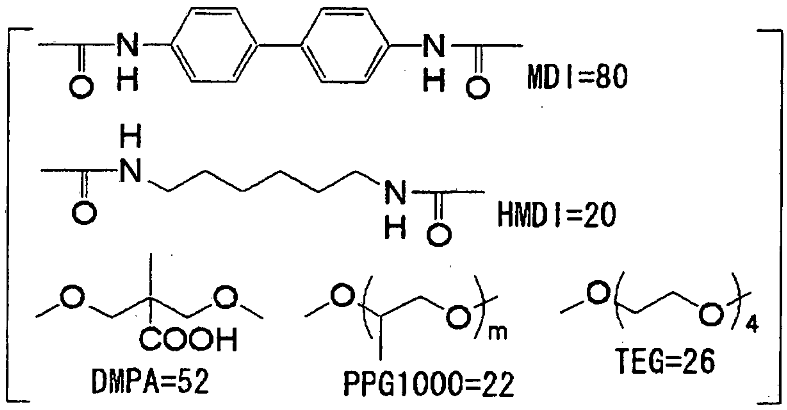

Table 9 clearly shows that the planographic printing plate precursors of Examples 7-1 to 7-15 which contain the specified oxime compound in the photosensitive layer are high in sensitivity, are excellent in storage stability and printing durability.

On the other hand, the planographic printing plate precursors of Comparative Examples 7-1 to 7-4 were inferior in both sensitivity and printing durability to the planographic printing plate precursors of the Examples.

## Claims

**1.** A polymerizable composition comprising:

(A) an oxime polymerization initiator which is represented by the following Formula (1A) or (1B), and which has, in ethyl acetate, a molar absorption coefficient of 20,000 or more at a λmax within a wavelength range of 300 nm or more and less than 400 nm and a molar absorption coefficient of 400 or less at (λmax + 20 nm); and
(B) a polymerizable compound:

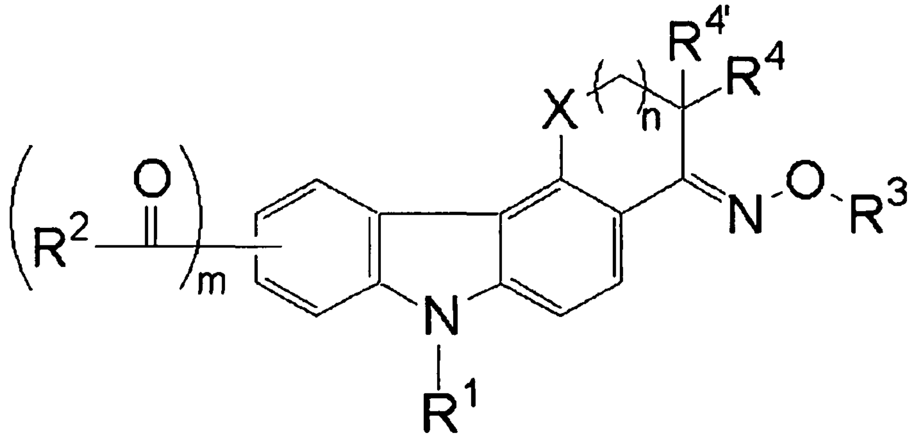

(1A)

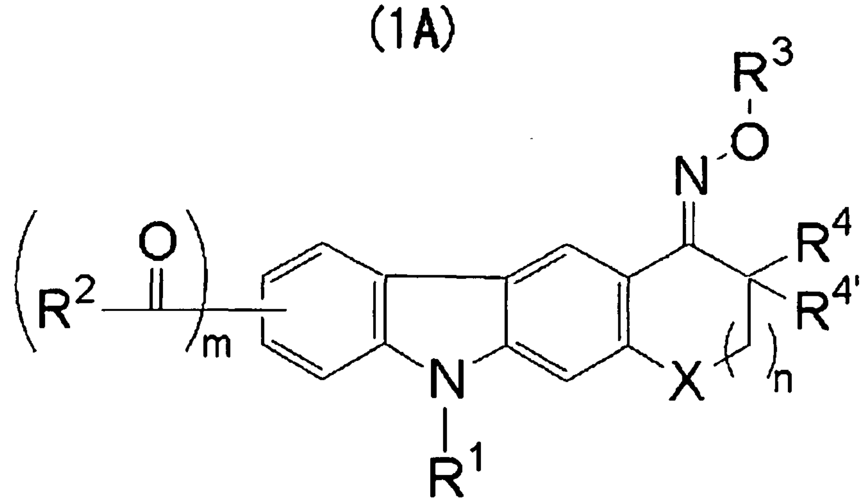

(1B)

wherein $R^1$ and $R^2$ each independently represents a substituted or unsubstituted alkyl group, a substituted or unsubstituted aryl group or a substituted or unsubstituted heterocyclic group; and $R^3$ represents a substituted or unsubstituted acyl group or a substituted or unsubstituted sulfonyl group; $R^4$ and $R^{4'}$ each independently represents a hydrogen atom, a substituted or unsubstituted alkyl group or a substituted or unsubstituted allyl group; n represents an integer of from 0 to 2, m represents 0 or 1; and X represents a carbon atom, an oxygen atom, a sulfur atom or a nitrogen atom.

**2.** A polymerizable composition according to Claim 1, wherein $R^2$ in Formula (1A) or (1B) is an aryl group represented by the following Formula (2):

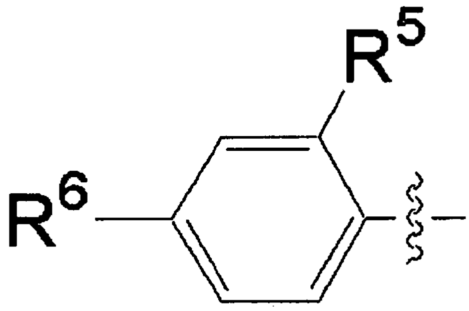

(2)

wherein $R^5$ and $R^6$ each independently represents a hydrogen atom, a halogen atom, an alkyl group, an aryl group, a hydroxy group, a thiol group, an amino group, a morpholino group, an alkyloxycarbonyl group, an acyloxy group, an alkoxy group, an alkylthio group or an alkylseleno group, and these groups may each further have a substituent.

**3.** A polymerizable composition according to Claim 1 or Claim 2, further comprising (C) a colorant.

**4.** A polymerizable composition according to Claim 3, wherein (C) the colorant is a pigment, and the polymerizable composition further comprises (D) a pigment dispersant.

**5.** A polymerizable composition according to Claim 3 or Claim 4, wherein (C) the colorant is a black colorant.

**6.** The use of a polymerizable composition as defined in any of Claims 3 to 5, for forming a colored pattern of a color filter.

**7.** A color filter, comprising:

a support; and
a colored pattern formed on the support by using the polymerizable composition according to Claim 6.

**8.** A method for producing a color filter comprising:

applying the polymerizable composition according to Claim 6 onto a support to form a polymerizable composition layer;
subjecting the polymerizable composition layer to pattern exposure; and
developing the exposed polymerizable composition layer to form a colored pattern.

**9.** A solid-state imaging device comprising the color filter according to Claim 7.

**10.** A planographic printing plate precursor, comprising:

a support; and
a photosensitive layer including the polymerizable composition according to any one of Claims 1 to 3.

**11.** A compound represented by the following Formula (1A) or (1B):

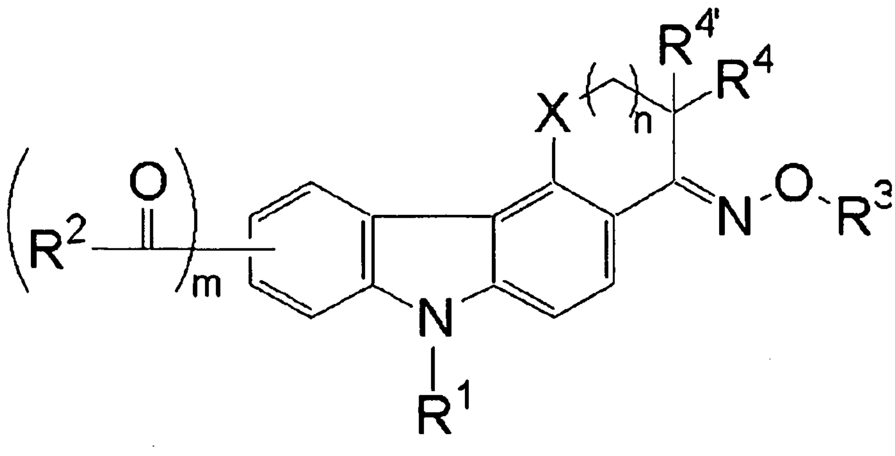

(1A)

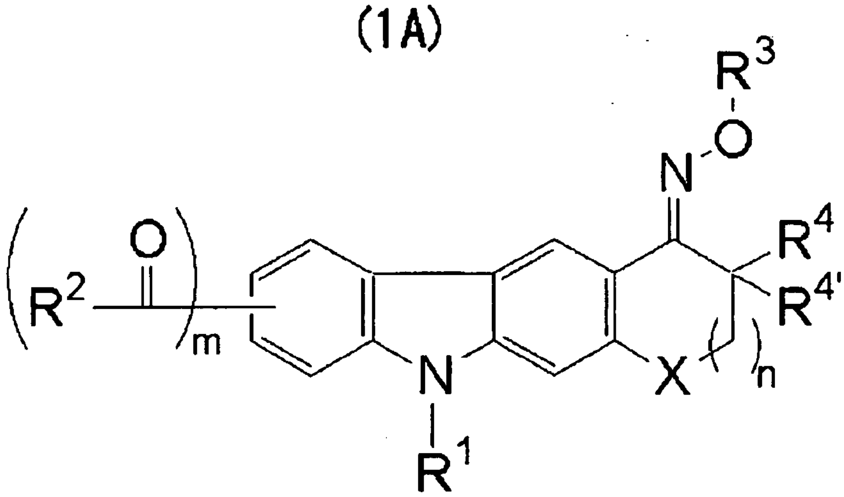

(1B)

wherein $R^1$ and $R^2$ each independently represents a substituted or unsubstituted alkyl group, a substituted or unsubstituted aryl group or a substituted or unsubstituted heterocyclic group, and $R^3$ represents a substituted or unsubstituted acyl group or a substituted or unsubstituted sulfonyl group; $R^4$ and $R^{4'}$ each independently represents a hydrogen atom, a substituted or unsubstituted alkyl group or a substituted or unsubstituted allyl group; n represents an integer of from 0 to 2, m represents 0 or 1; and X represents a carbon atom, an oxygen atom, a sulfur atom or a nitrogen atom.

## Patentansprüche

**1.** Polymerisierbare Zusammensetzung, umfassend:

(A) einen Oxim-Polymerisationsinitiator, der durch die folgende Formel (1A) oder (1B) dargestellt ist und der in Ethylacetat einen molaren Absorptionskoeffizienten von 20.000 oder mehr bei einem λmax innerhalb eines Wellenlängenbereichs von 300 nm oder länger und kürzer als 400 nm aufweist und der einen molaren Absorptionskoeffizienten von 400 oder weniger bei (λmax + 20 nm) aufweist; und
(B) eine polymerisierbare Verbindung:

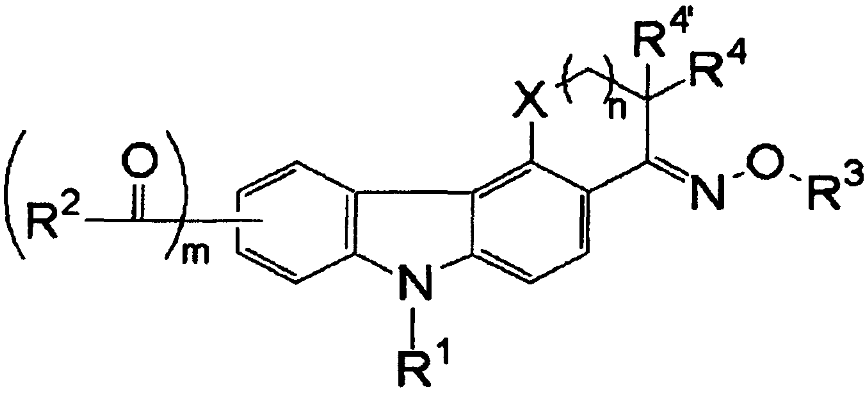

(1A)

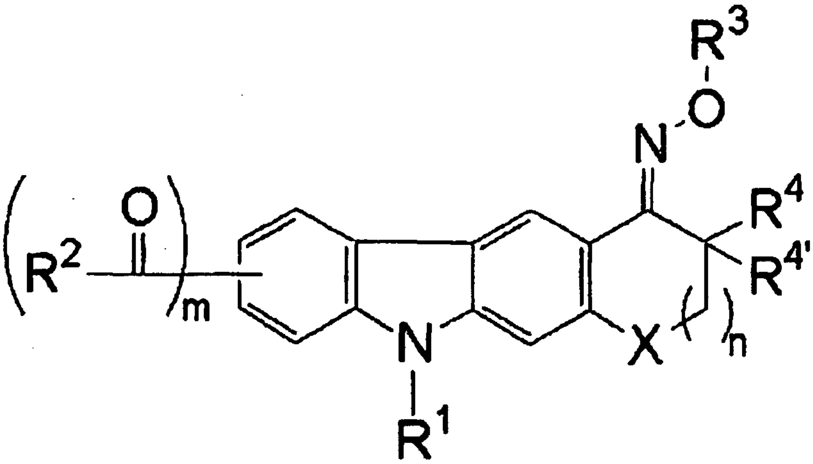

(1B)

worin $R^1$ und $R^2$ jeweils unabhängig eine substituierte oder unsubstituierte Alkylgruppe, eine substituierte oder unsubstituierte Arylgruppe oder eine substituierte oder unsubstituierte heterocyclische Gruppe darstellen;
und $R^3$ eine substituierte oder unsubstituierte Acylgruppe oder eine substituierte oder unsubstituierte Sulfonylgruppe darstellt; $R^4$ und $R^{4'}$ jeweils unabhängig ein Wasserstoffatom, eine substituierte oder unsubstituierte Alkylgruppe oder eine substituierte oder unsubstituierte Allylgruppe darstellen; n eine ganze Zahl von 0 bis 2 darstellt, m 0 oder 1 darstellt; und X ein Kohlenstoffatom, ein Sauerstoffatom, ein Schwefelatom oder ein Stickstoffatom darstellt.

2. Polymerisierbare Zusammensetzung gemäß Anspruch 1, worin $R^2$ in Formel (1A) oder (1B) eine durch die folgende Formel (2) dargestellte Arylgruppe ist:

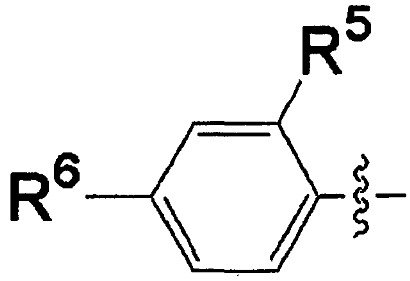

(2)

worin $R^5$ und $R^6$ jeweils unabhängig ein Wasserstoffatom, ein Halogenatom, eine Alkylgruppe, eine Arylgruppe, eine Hydroxygruppe, eine Thiolgruppe, eine Aminogruppe, eine Morpholinogruppe, eine Alkyloxycarbonylgruppe, eine Acyloxygruppe, eine Alkoxygruppe, eine Alkylthiogruppe oder eine Alkylselenogruppe darstellen, und diese Gruppen ferner einen Substituenten aufweisen können.

**3.** Polymerisierbare Zusammensetzung gemäß Anspruch 1 oder Anspruch 2, ferner umfassend (C) ein Färbemittel.

**4.** Polymerisierbare Zusammensetzung gemäß Anspruch 3, worin (C) das Färbemittel ein Pigment ist und die polymerisierbare Zusammensetzung ferner (D) ein PigmentDispergiermittel umfasst.

**5.** Polymerisierbare Zusammensetzung gemäß Anspruch 3 oder Anspruch 4, worin (C) das Färbemittel ein schwarzes Färbemittel ist.

**6.** Verwendung einer polymerisierbaren Zusammensetzung gemäß irgendeinem der Ansprüche 3 bis 5 zum Bilden einer gefärbten Struktur eines Farbfilters.

**7.** Farbfilter, umfassend:

einen Träger; und
eine gefärbte Struktur, die auf dem Träger unter Verwendung der polymerisierbaren Zusammensetzung gemäß Anspruch 6 gebildet ist.

**8.** Verfahren zur Herstellung eines Farbfilters, umfassend:

Auftragen der polymerisierbaren Zusammensetzung gemäß Anspruch 6 auf einen Träger, um eine Schicht der polymerisierbaren Zusammensetzung zu bilden;
Unterziehen der Schicht der polymerisierbaren Zusammensetzung unter eine Strukturbelichtung; und
Entwickeln der belichteten polymerisierbaren Zusammensetzungsschicht, um eine gefärbte Struktur zu bilden.

**9.** Festphasen-Bildgebungsvorrichtung, umfassend den Farbfilter gemäß Anspruch 7.

**10.** Flachdruckplattenvorläufer, umfassend:

einen Träger; und
eine fotoempfindliche Schicht, die die polymerisierbare Zusammensetzung gemäß irgendeinem der Ansprüche 1 bis 3 enthält.

**11.** Verbindung, dargestellt durch die folgende Formel (1A) oder (1B):

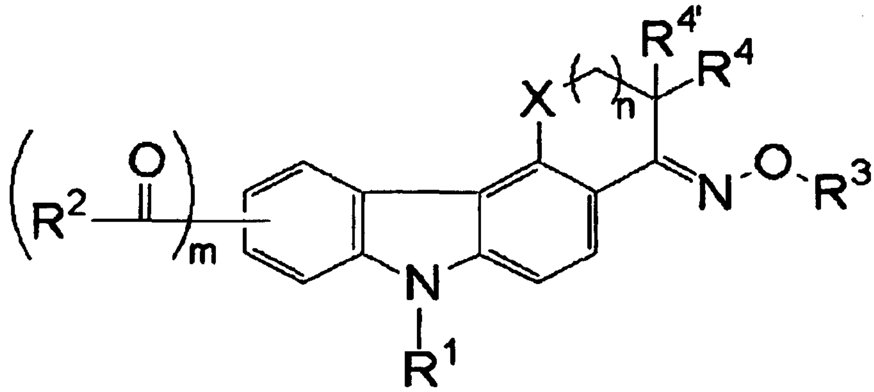

**(1A)**

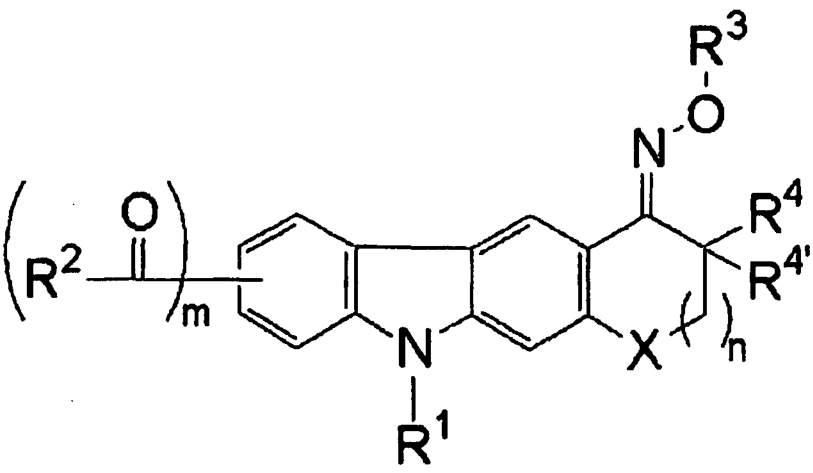

(1B)

worin $R^1$ und $R^2$ jeweils unabhängig eine substituierte oder unsubstituierte Alkylgruppe, eine substituierte oder unsubstituierte Arylgruppe oder eine substituierte oder unsubstituierte heterocyclische Gruppe darstellen; und $R^3$ eine substituierte oder unsubstituierte Acylgruppe oder eine substituierte oder unsubstituierte Sulfonylgruppe darstellt; $R^4$ und $R^{4'}$ jeweils unabhängig ein Wasserstoffatom, eine substituierte oder unsubstituierte Alkylgruppe oder eine substituierte oder unsubstituierte Allylgruppe darstellen; n eine ganze Zahl von 0 bis 2 darstellt, m 0 oder 1 darstellt; und X ein Kohlenstoffatom, ein Sauerstoffatom, ein Schwefelatom oder ein Stickstoffatom darstellt.

**Revendications**

**1.** Composition polymérisable comprenant :

(A) un initiateur de polymérisation oxime qui est représenté par la formule (1A) ou (1B) suivante, et qui a, dans l'acétate d'éthyle, un coefficient d'absorption molaire de 20 000 ou plus à une λmax dans une plage de longueurs d'onde de 300 nm ou plus et inférieure à 400 nm et un coefficient d'absorption molaire de 400 ou moins à (λmax + 20 nm) ; et
(B) un composé polymérisable :

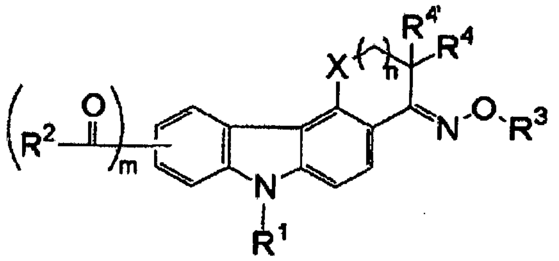

(1A)

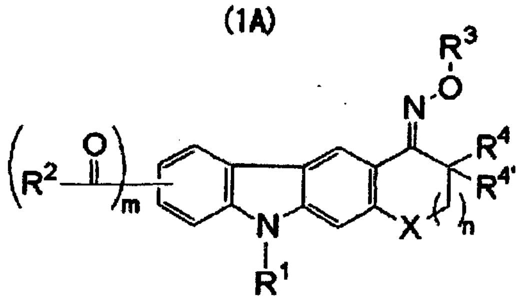

(1B)

où $R^1$ et $R^2$ représentent chacun indépendamment un groupe alkyle substitué ou non substitué, un groupe aryle substitué ou non substitué ou un groupe hétérocyclique substitué ou non substitué ; et $R^3$ représente un groupe acyle substitué ou non substitué ou un groupe sulfonyle substitué ou non substitué ; $R^4$ et $R^{4'}$ représentent chacun indépendamment un atome d'hydrogène, un groupe alkyle substitué ou non substitué ou un groupe allyle substitué ou non substitué ; n représente un entier de 0 à 2, m représente 0 ou 1 ; et X représente un atome de carbone, un atome d'oxygène, un atome de soufre ou un atome d'azote.

**2.** Composition polymérisable selon la revendication 1, où $R^2$ dans la formule (1A) ou (1B) est un groupe aryle représenté par la formule (2) suivante :

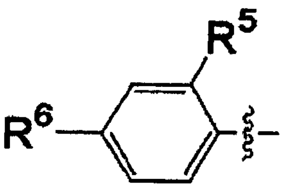

(2)

où $R^5$ et $R^6$ représentent chacun indépendamment un atome d'hydrogène, un atome d'halogène, un groupe alkyle, un groupe aryle, un groupe hydroxy, un groupe thiol, un groupe amino, un groupe morpholino, un groupe alkyloxycarbonyle, un groupe acyloxy, un groupe alcoxy, un groupe alkylthio ou un groupe alkylséléno, et ces groupes peuvent avoir chacun en outre un substituant.

**3.** Composition polymérisable selon la revendication 1 ou la revendication 2 comprenant en outre (C) une matière colorante.

**4.** Composition polymérisable selon la revendication 3 où (C) la matière colorante est un pigment, et la composition polymérisable comprend en outre (D) un dispersant de pigment.

**5.** Composition polymérisable selon la revendication 3 ou la revendication 4 où (C) la matière colorante est une matière colorante noire.

**6.** Utilisation d'une composition polymérisable telle que définie dans l'une quelconque des revendications 3 à 5 pour former un motif coloré d'un filtre couleur.

**7.** Filtre couleur comprenant :

un support ; et
un motif coloré formé sur le support au moyen de la composition polymérisable selon la revendication 6.

**8.** Procédé pour produire un filtre couleur comprenant :

l'application de la composition polymérisable selon la revendication 6 sur un support pour former une couche de composition polymérisable ;
l'exposition de la couche de composition polymérisable à une exposition à un motif ; et
le développement de la couche de composition polymérisable exposée pour former un motif coloré.

**9.** Dispositif d'imagerie à l'état solide comprenant le filtre couleur selon la revendication 7.

**10.** Précurseur de plaque d'impression planographique comprenant :

un support ; et
une couche photosensible incluant la composition polymérisable selon l'une quelconque des revendications 1 à 3.

**11.** Composé représenté par la formule (1A) ou (1B) suivante :

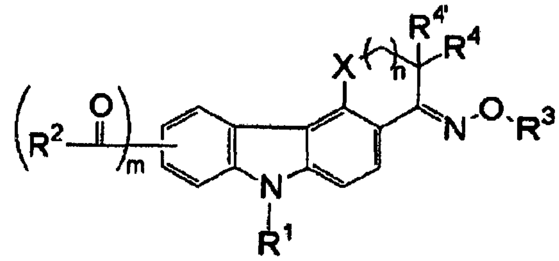

(1A)

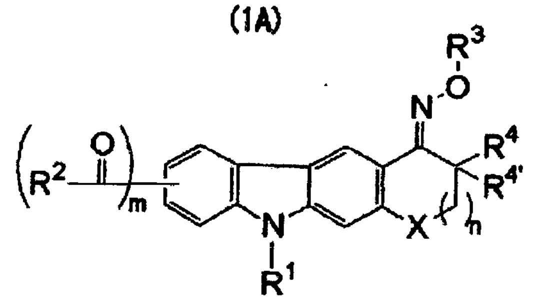

(1B)

où $R^1$ et $R^2$ représentent chacun indépendamment un groupe alkyle substitué ou non substitué, un groupe aryle substitué ou non substitué ou un groupe hétérocyclique substitué ou non substitué ; et $R^3$ représente un groupe acyle substitué ou non substitué ou un groupe sulfonyle substitué ou non substitué ; $R^4$ et $R^{4'}$ représentent chacun indépendamment un atome d'hydrogène, un groupe alkyle substitué ou non substitué ou un groupe allyle substitué ou non substitué ; n représente un entier de 0 à 2, m représente 0 ou 1 ; et X représente un atome de carbone, un atome d'oxygène, un atome de soufre ou un atome d'azote.

FIG. 1

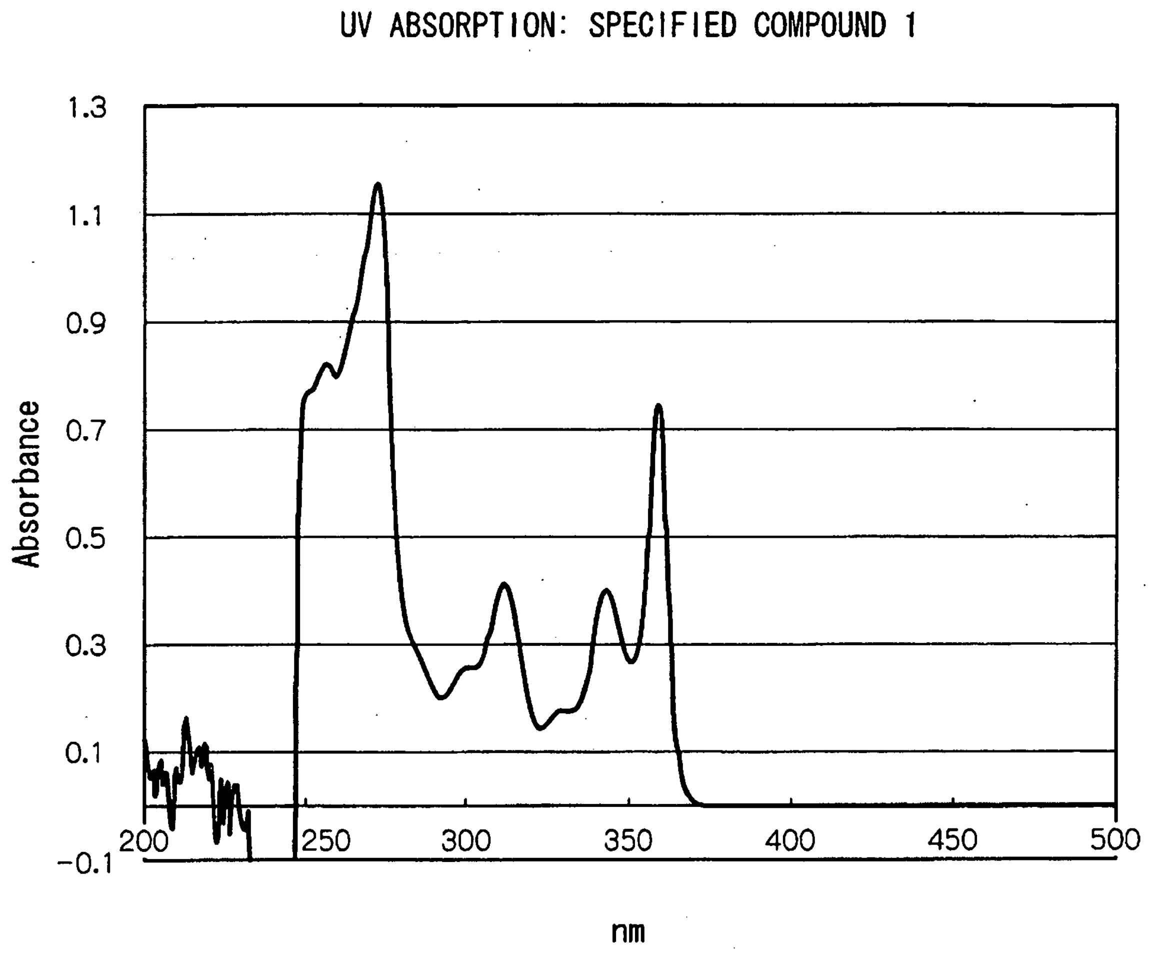
UV ABSORPTION: SPECIFIED COMPOUND 1
Absorbance
1.3
1.1
0.9
0.7
0.5
0.3
0.1
-0.1
200
250
300
350
400
450
500
nm

FIG. 2

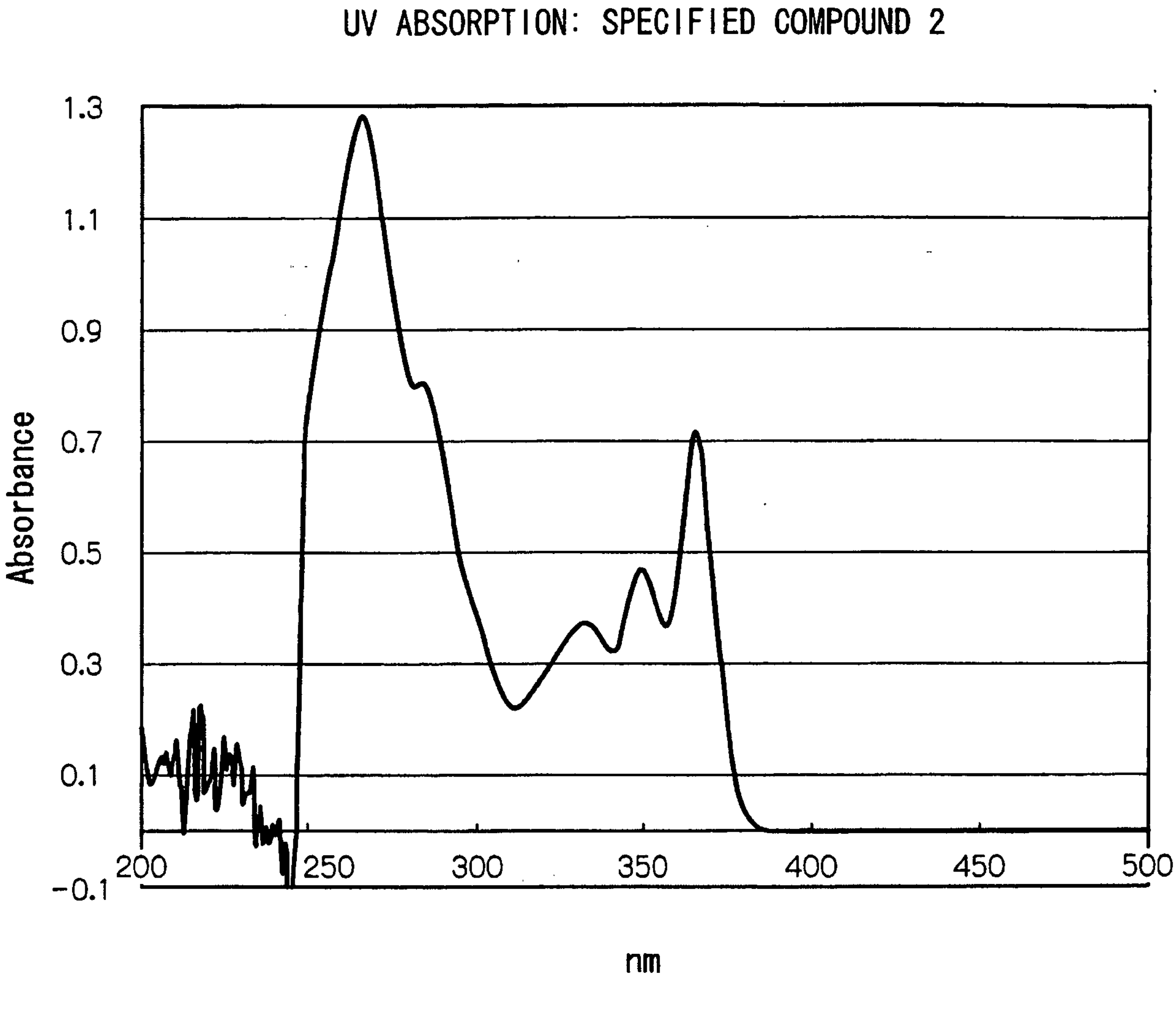
UV ABSORPTION: SPECIFIED COMPOUND 2
Absorbance
1.3
1.1
0.9
0.7
0.5
0.3
0.1
-0.1
200
250
300
350
400
450
500
nm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4255513 A
- US 4590145 A
- JP 2000080068 A
- JP 2001233842 A
- JP 2006342166 A
- JP 2007231000 A
- JP 2005202252 A
- JP 2007322744 A
- JP 2007023100 A
- EP 2105443 A
- US 4575330 A
- DE 19700064
- EP 678534 A
- JP 51047334 B
- JP 57196231 A
- JP 59005240 A
- JP 59005241 A
- JP 2226149 A
- JP 1165613 A
- JP 54021726 B
- JP 48041708 B
- JP 51037193 A
- JP 2032293 B
- JP 2016765 B
- JP 58049860 B
- JP 56017654 B
- JP 62039417 B
- JP 62039418 B
- JP 63277653 A
- JP 63260909 A
- JP 1105238 A
- JP 48064183 A
- JP 49043191 B
- JP 52030490 B
- JP 46043946 B
- JP 1040337 B
- JP 1040336 B
- JP 2025493 A
- JP 61022048 A
- JP 2007302836 A
- JP 49005432 A
- JP 57205322 A
- JP 60065069 A
- JP 61201610 A
- JP 6490403 A
- JP 6491102 A
- JP 1094301 A
- JP 6011614 A
- JP 2592207 B
- US 4808501 A
- US 5667920 A
- US 5059500 A
- JP 5333207 A
- JP 6035183 A
- JP 6051115 A
- JP 6194828 A
- JP 8211599 A
- JP 4249549 A
- JP 10123316 A
- JP 11302283 A
- JP 7286107 A
- JP 2001004823 A
- JP 8015522 A
- JP 8029771 A
- JP 8146215 A
- JP 11343437 A
- JP 8062416 A
- JP 2002014220 A
- JP 2002014221 A
- JP 2002014222 A
- JP 2002014223 A
- JP 8302224 A
- JP 8073758 A
- JP 8179120 A
- JP 8151531 A
- JP 4420189 B
- JP 51082102 A
- JP 52134692 A
- JP 59138205 A
- JP 60084305 A
- JP 62018537 A
- JP 6433104 A
- JP 53000702 A
- JP 55500806 B
- JP 5142772 A
- JP 56075643 A
- JP 48042965 B
- JP 55034414 B
- JP 6308727 A
- JP 59044615 A
- JP 54034327 B
- JP 58012577 B
- JP 54025957 B
- JP 54092723 A
- JP 59053836 A
- JP 59071048 A
- JP 2002309057 A
- JP 2002311569 A

- JP 2000187322 A
- JP 2002062698 A
- JP 2001242612 A
- JP 7012004 B
- JP 7120041 B
- JP 7120042 B
- JP 8012424 B
- JP 63287944 A
- JP 63287947 A
- JP 1271741 A
- JP 2002107918 A
- EP 993966 A
- EP 1204000 A
- JP 2001318463 A
- JP 2006078749 A
- JP 11171907 A
- JP 7120040 B
- JP 6250387 A
- JP 48018327 B
- JP 54063902 A
- JP 47005125 B
- US 3658662 A
- JP 46027481 B
- JP 52058602 A
- JP 52030503 A
- JP 56028893 A
- JP 7154983 A
- US 3055295 A
- JP 56013168 A
- JP 9080744 A
- JP 8507727 A
- US 3458311 A
- JP 55049729 B
- US 3458313 A
- JP 55049729 A
- JP 57007427 B
- US 3375171 A
- US 3615480 A
- JP 50026601 A
- JP 58054341 A
- JP 56039464 B
- JP 56042860 B
- JP 2002202616 A


**Non-patent literature cited in the description**


- *Journal of the Adhesion Society of Japan,* 1984, vol. 20 (7), 300-308
- **M. R. SANDER et al.** *Journal of Polymer Society,* 1972, vol. 10, 3173
- *J. Appl. Chem.,* 1961, vol. 34, 2203-2207
- **C. HANSCH ; A. LEO.** Substituent Constants for Correlation Analysis in Chemistry and Biology. J. Wiley & Sons, 1979
- **A. K. GHOSE et al.** *J. Comput. Chem.,* 1988, vol. 9, 80